(19) Europäisches Patentamt
European Patent Office
Office européen des brevets

(11) Veröffentlichungsnummer: **0 569 912 A1**

(12) **EUROPÄISCHE PATENTANMELDUNG**

(21) Anmeldenummer: **93107583.2**

(22) Anmeldetag: **10.05.93**

(51) Int. Cl.5: **C07D 239/28**, C07D 239/30, C07D 239/34, C07D 239/38, C07D 239/42, C07D 239/48, C07D 239/46, C07D 401/04, C07D 407/04, C07D 409/04, C07D 413/04, A01N 43/54

(30) Priorität: **15.05.92 DE 4216131**

(43) Veröffentlichungstag der Anmeldung: **18.11.93 Patentblatt 93/46**

(84) Benannte Vertragsstaaten: **AT CH DE ES FR GB GR IT LI PT**

(71) Anmelder: **HOECHST AKTIENGESELLSCHAFT**

**D-65926 Frankfurt(DE)**

(72) Erfinder: **Minn, Klemens, Dr.**
**Rossertstrasse 61**
**W-6234 Hattersheim/Main(DE)**
Erfinder: **Waltersdorfer, Anna, Dr.**
**Rauenthaler Weg 28**
**W-6000 Frankfurt/Main(DE)**
Erfinder: **Braun, Peter, Dr.**
**Pfarrer-Dorn-Strasse 13**
**W-6500 Mainz(DE)**
Erfinder: **Sachse, Burkhard, Dr.**
**An der Ziegelei 30**
**W-6233 Kelkheim/Ts.(DE)**

(54) **4-Alkyl-substituierte Pyrimidin-5-carboxanilide, Verfahren zu deren Herstellung, diese enthaltende Mittel und deren Verwendung als Fungizide.**

(57) Die vorliegende Erfindung betrifft Pyrimidin-5-carboxanilide der Formel I, deren Tautomeren und deren Säureadditionssalze,

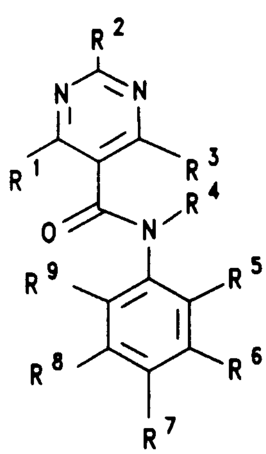

(I)

in welcher $R^1$ gegebenenfalls substituiertes Alkyl oder Cycloalkyl bedeutet, $R^2$ und $R^3$ gleich oder verschieden sind und Wasserstoff bedeuten oder die anderen, in der Beschreibung definierten Bedeutungen haben, $R^4$ Wasserstoff, Acyl, gegebenenfalls substituiertes Alkyl oder dergleichen bedeutet, $R^5$-$R^9$ gleich oder verschieden

EP 0 569 912 A1

sind und Wasserstoff bedeuten oder die anderen, in der Beschreibung definierten Bedeutungen haben, Verfahren zu deren Herstellung, diese enthaltende Mittel und deren Verwendung als Fungizide.

Die vorliegende Erfindung betrifft Pyrimidin-5-carboxanilide, die in der 4-Position des Pyrimidins einen gegebenenfalls substituierten Alkylrest tragen.

Pyrimidin-5-carboxanilide und deren Verwendung als Herbizide bzw. Wachstumsregulatoren sind beispielsweise bekannt aus JP-A-01180808 (Chem.Abstr. 112:2621h) oder JP-A-61243067 (Chem.Abstr. 106:102320d).

Überraschenderweise wurden nun 4-alkyl-substituierte Pyrimidin-5-carboxanilide gefunden, die vorteilhafte Wirkungen bei der Bekämpfung eines breiten Spektrums phytopathogener Pilze, insbesondere bei niedrigen Dosierungen aufweisen.

Die vorliegende Erfindung betrifft daher Mittel, die mindestens eine Verbindung der Formel I, deren Tautomeren oder deren Säureadditionssalz enthalten,

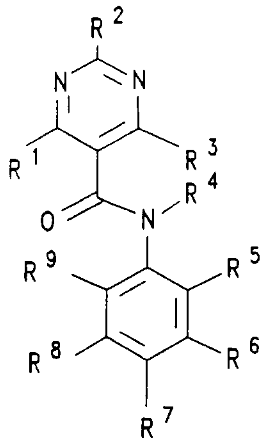

( I )

worin

$R^1$ = $(C_1\text{-}C_4)$-Alkyl, Hydroxy-$(C_1\text{-}C_4)$-alkyl, Halo-$(C_1\text{-}C_4)$-alkyl, Perhalo-$(C_1\text{-}C_4)$-alkyl, $(C_1\text{-}C_4)$-Alkoxy-$(C_1\text{-}C_4)$-alkyl, Halo-$(C_1\text{-}C_4)$-alkoxy-$(C_1\text{-}C_4)$-alkyl, $(C_1\text{-}C_4)$-Alkoxy-$(C_1\text{-}C_4)$-haloalkyl, Perhalo$(C_1\text{-}C_4)$-alkoxy-$(C_1\text{-}C_4)$-alkyl, Halo-$(C_1\text{-}C_4)$-alkylthio-$(C_1\text{-}C_4)$-alkyl, Perhalo-$(C_1\text{-}C_4)$-alkylthio-$(C_1\text{-}C_4)$-alkyl, $(C_1\text{-}C_4)$-Alkylthio-$(C_1\text{-}C_4)$-alkyl, $(C_1\text{-}C_4)$-Alkylamino-$(C_1\text{-}C_4)$-alkyl, $(C_1\text{-}C_4)$-Dialkylamino-$(C_1\text{-}C_4)$-alkyl, $(C_3\text{-}C_9)$-Cycloalkylamino-$(C_1\text{-}C_4)$-alkyl, $(C_3\text{-}C_9)$-Cycloalkyl, $(C_3\text{-}C_9)$-Cycloalkyl-$(C_1\text{-}C_4)$-alkyl oder Cyano-$(C_1\text{-}C_4)$-alkyl bedeutet und unter Halo die Halogenatome Cl, Br und insbesondere F zu verstehen sind;

$R^2$ = Wasserstoff, Hydroxy, Amino, Mercapto, Halogen, $(C_1\text{-}C_8)$-Alkyl, Hydroxy-$(C_1\text{-}C_4)$-alkyl, Dihydroxy-$(C_1\text{-}C_4)$-alkyl, Cyano-$(C_1\text{-}C_4)$-alkyl, Halo-$(C_1\text{-}C_4)$-alkyl, Perhalo-$(C_1\text{-}C_4)$-alkyl, $(C_1\text{-}C_6)$-Alkoxy, $(C_2\text{-}C_6)$-Alkenyloxy, $(C_2\text{-}C_6)$-Alkinyloxy, $(C_1\text{-}C_4)$-Alkoxy-$(C_1\text{-}C_4)$-alkyl, Halo$(C_1\text{-}C_4)$-alkoxy-$(C_1\text{-}C_4)$-alkyl, Perhalo-$(C_1\text{-}C_4)$-alkoxy-$(C_1\text{-}C_4)$-alkyl, $(C_1\text{-}C_4)$-Alkoxy-$(C_1\text{-}C_4)$-haloalkyl, $(C_1\text{-}C_4)$-Alkylthio, Halo-$(C_1\text{-}C_4)$-alkylthio, Perhalo-$(C_1\text{-}C_4)$-alkylthio, Halo-$(C_1\text{-}C_4)$-alkylthio-$(C_1\text{-}C_4)$-alkyl, Perhalo-$(C_1\text{-}C_4)$-alkylthio-$(C_1\text{-}C_4)$-alkyl, $(C_1\text{-}C_4)$-Alkylthio-$(C_1\text{-}C_4)$-alkyl, $(C_1\text{-}C_4)$-Alkylsulfonyl, $(C_1\text{-}C_4)$-Alkylsulfonyl-$(C_1\text{-}C_4)$-alkyl, $(C_1\text{-}C_4)$-Alkoxycarbonyl-$(C_1\text{-}C_4)$-alkyl, $(C_1\text{-}C_4)$-Alkylamino-$(C_1\text{-}C_4)$-alkyl, $(C_1\text{-}C_4)$-Dialkylamino-$(C_1\text{-}C_4)$-alkyl, $(C_3\text{-}C_9)$-Cycloalkylamino-$(C_1\text{-}C_4)$-alkyl, $(C_3\text{-}C_9)$-Cycloalkyl, $(C_3\text{-}C_9)$-Cycloalkyl-$(C_1\text{-}C_4)$-alkyl oder $(C_3\text{-}C_9)$-Heterocycloalkyl-$(C_1\text{-}C_4)$-alkyl bedeutet, wobei die cyclischen Reste bis zu dreifach durch $(C_1\text{-}C_4)$-Alkyl substituiert sein können und unter Halo die Halogenatome F, Br und insbesondere Cl zu verstehen sind;

$R^3$ = Wasserstoff, Hydroxy, Amino, Mercapto, Halogen, $(C_1\text{-}C_8)$-Alkyl, Hydroxy-$(C_1\text{-}C_4)$-alkyl, Halo-$(C_1\text{-}C_4)$-alkyl, Perhalo-$(C_1\text{-}C_4)$-alkyl, $(C_1\text{-}C_6)$-Alkoxy, $(C_2\text{-}C_6)$-Alkenyloxy, $(C_2\text{-}C_6)$-Alkinyloxy, $(C_1\text{-}C_4)$-Alkoxy-$(C_1\text{-}C_4)$-alkyl, Halo$(C_1\text{-}C_4)$-alkoxy-$(C_1\text{-}C_4)$-alkyl, $(C_1\text{-}C_4)$-Alkoxy-$(C_1\text{-}C_4)$-haloalkyl, Perhalo$(C_1\text{-}C_4)$-alkoxy-$(C_1\text{-}C_4)$-alkyl, $(C_1\text{-}C_4)$-Alkylthio, Halo-$(C_1\text{-}C_4)$-alkylthio, Perhalo-$(C_1\text{-}C_4)$-alkylthio, Halo-$(C_1\text{-}C_4)$-alkylthio-$(C_1\text{-}C_4)$-alkyl, Perhalo-$(C_1\text{-}C_4)$-alkylthio-$(C_1\text{-}C_4)$-alkyl, $(C_1\text{-}C_4)$-Alkylthio-$(C_1\text{-}C_4)$-alkyl, $(C_1\text{-}C_4)$-Alkylsulfonyl, $(C_1\text{-}C_4)$-Alkylsulfonyl-$(C_1\text{-}C_4)$-alkyl, $(C_1\text{-}C_4)$-Alkoxycarbonyl-$(C_1\text{-}C_4)$-alkyl, $(C_1\text{-}C_4)$-Alkylamino-$(C_1\text{-}C_4)$-alkyl,

$(C_1$-$C_4)$-Dialkylamino-$(C_1$-$C_4)$-alkyl, $(C_3$-$C_9)$-Cycloalkylamino-$(C_1$-$C_4)$-alkyl, $(C_3$-$C_9)$-Cycloalkyl, $(C_3$-$C_9)$-Cycloalkyl-$(C_1$-$C_4)$-alkyl, $(C_3$-$C_9)$-Heterocycloalkyl-$(C_1$-$C_4)$-alkyl, gegebenenfalls substituiertes Phenyl, gegebenenfalls substituiertes $(C_1$-$C_{10})$-Heteroaryl, gegebenenfalls substituiertes Phenoxy-$(C_1$-$C_4)$-alkyl, gegebenenfalls substituiertes Phenyl-$(C_1$-$C_4)$-alkyl, gegebenenfalls substituiertes Phenylmercapto-$(C_1$-$C_4)$-alkyl, gegebenenfalls substituiertes Phenylamino-$(C_1$-$C_4)$-alkyl oder gegebenenfalls substituiertes Phenoxyphenyl-$(C_1$-$C_4)$-alkyl bedeutet, wobei unter gegebenenfalls substituiertem Phenyl bzw. Heteroaryl vorzugsweise zu verstehen ist, daß der Phenyl- bzw. Heteroarylteil bis zu dreifach durch gleiche oder verschiedene Reste aus der Reihe Halogen, Nitro, Cyano, $(C_1$-$C_6)$-Alkoxycarbonyl, $(C_1$-$C_6)$-Alkanyloxy, $(C_1$-$C_6)$-Alkyl, $(C_1$-$C_4)$-Alkoxy, $(C_1$-$C_4)$-Alkylthio, $(C_1$-$C_4)$-Haloalkyl und $(C_1$-$C_4)$-Haloalkoxy substituiert sein kann, und unter Halo die Halogenatome F,Br und insbesondere Cl zu verstehen sind;

$R^4$ = Wasserstoff, Formyl, $(C_1$-$C_4)$-Alkyl, $(C_1$-$C_4)$-Alkylcarbonyl, Hydroxy-$(C_1$-$C_4)$-alkyl, Halo-$(C_1$-$C_4)$-alkyl, Perhalo-$(C_1$-$C_4)$-alkyl, $(C_1$-$C_4)$-Alkoxy-$(C_1$-$C_4)$-alkyl, Halo$(C_1$-$C_4)$-alkoxy-$(C_1$-$C_4)$-alkyl, Perhalo$(C_1$-$C_4)$-alkoxy-$(C_1$-$C_4)$-alkyl, Perhalo-$(C_1$-$C_4)$-alkylthio, $(C_1$-$C_4)$-Alkylthio-$(C_1$-$C_4)$-alkyl, $(C_2$-$C_6)$-Alkenyl, $(C_2$-$C_6)$-Alkinyl, $(C_1$-$C_4)$-Alkylamino-$(C_1$-$C_4)$-alkyl, $(C_1$-$C_4)$-Dialkylamino-$(C_1$-$C_4)$-alkyl, $(C_3$-$C_9)$-Cycloalkylamino-$(C_1$-$C_4)$-alkyl, $(C_3$-$C_9)$-Cycloalkyl, $(C_3$-$C_9)$-Cycloalkyl-$(C_1$-$C_4)$-alkyl, $(C_3$-$C_9)$-Heterocycloalkyl-$(C_1$-$C_4)$-alkyl, $(C_1$-$C_4)$-Alkoxycarbonyl-$(C_1$-$C_4)$-alkyl oder gegebenenfalls substituiertes Phenyl-$(C_1$-$C_4)$-alkyl bedeutet, wobei unter gegebenenfalls substituiertem Phenyl vorzugsweise gegebenenfalls bis zu dreifach durch gleiche oder verschiedene Reste aus der Reihe Halogen, Nitro, Cyano, $(C_1$-$C_6)$-Alkoxycarbonyl, $(C_1$-$C_6)$-Alkanyloxy, $(C_1$-$C_6)$-Alkyl, $(C_1$-$C_4)$-Alkoxy, $(C_1$-$C_4)$-Alkylthio, $(C_1$-$C_4)$-Haloalkyl und $(C_1$-$C_6)$-Haloalkoxy substituiertes Phenly zu verstehen ist, andere cyclische Reste bis zu dreifach durch $(C_1$-$C_6)$-Alkyl substituiert sein können und unter Halo die Halogenatome F, Cl, Br zu verstehen sind;

$R^5$, $R^6$, $R^7$, $R^8$ und $R^9$ gleich oder verschieden sind und unabhängig voneinander Wasserstoff, Halogen, Hydroxy, Amino, Nitro, Cyano, Thiocyano, $(C_1$-$C_4)$-Alkyl, Cyano-$(C_1$-$C_4)$-alkyl, $(C_1$-$C_4)$-Alkoxy, $(C_1$-$C_4)$-Alkylamino, $(C_1$-$C_4)$-Dialkylamino, $(C_1$-$C_4)$-Alkylcarbonylamino, Halo-$(C_1$-$C_4)$-alkyl, Perhalo-$(C_1$-$C_4)$-alkyl, Hydroxy-$(C_1$-$C_4)$-alkyl, Dihydroxy-$(C_1$-$C_4)$-alkyl, $(C_1$-$C_4)$-Alkoxy-$(C_1$-$C_4)$-alkyl, Halo$(C_1$-$C_4)$-alkoxy-$(C_1$-$C_4)$-alkyl, $(C_1$-$C_4)$-Alkoxy-$(C_1$-$C_4)$-haloalkyl, Perhalo$(C_1$-$C_4)$-alkoxy-$(C_1$-$C_4)$-alkyl, $(C_1$-$C_4)$-Alkylthio, Halo-$(C_1$-$C_4)$-alkylthio, Perhalo-$(C_1$-$C_4)$-alkylthio, Halo-$(C_1$-$C_4)$-alkylthio-$(C_1$-$C_4)$-alkyl, Perhalo-$(C_1$-$C_4)$-alkylthio-$(C_1$-$C_4)$-alkyl, $(C_1$-$C_4)$-Alkylthio-$(C_1$-$C_4)$-alkyl, $(C_2$-$C_6)$-Alkenyl, $(C_2$-$C_6)$-Alkinyl, $(C_1$-$C_4)$-Alkylamino-$(C_1$-$C_4)$-alkyl, $(C_1$-$C_4)$-Dialkylamino-$(C_1$-$C_4)$-alkyl, $(C_3$-$C_9)$-Cycloalkylamino-$(C_1$-$C_4)$-alkyl, $(C_3$-$C_9)$-Cycloalkyl, $(C_3$-$C_9)$-Cycloalkyl-$(C_1$-$C_4)$-alkyl, $(C_3$-$C_9)$-Heterocycloalkyl-$(C_1$-$C_4)$-alkyl, wobei die cyclischen Reste bis zu dreifach durch $(C_1$-$C_4)$-Alkyl substituiert sein können, gegebenenfalls substituiertes Phenyl, gegebenfalls substituiertes $(C_1$-$C_{10})$-Heteroaryl, gegebenenfalls substituiertes Phenoxy, gegebenenfalls substituiertes Anilino, gegebenenfalls substituiertes Phenylmercapto, gegebenenfalls substituiertes Benzoyl-$(C_1$-$C_4)$-alkyl, $(C_1$-$C_4)$-Alkoxycarbonyl-$(C_1$-$C_4)$-alkyl, gegebenenfalls substituiertes Phenyloxycarbonyl-$(C_1$-$C_4)$-alkyl, $(C_1$-$C_4)$-Alkylaminocarbonyl-$(C_1$-$C_4)$-alkyl, $(C_1$-$C_4)$-Dialkylaminocarbonyl-$(C_1$-$C_4)$-alkyl, $(C_1$-$C_4)$-Alkylcarbonyl, $(C_1$-$C_4)$-Haloalkylcarbonyl, $(C_1$-$C_4)$-Perhaloalkylcarbonyl, $(C_1$-$C_4)$-Alkyloxycarbonyl, $(C_1$-$C_4)$-Haloalkyloxycarbonyl, $(C_1$-$C_4)$-Perhaloalkyloxycarbonyl, $(C_1$-$C_4)$-Alkylthiocarbonyl, $(C_1$-$C_4)$-Haloalkylthiocarbonyl, $(C_1$-$C_4)$-Perhaloalkylthiocarbonyl, Aminocarbonyl, $(C_1$-$C_4)$-Alkylaminocarbonyl, $(C_1$-$C_4)$-Haloalkylaminocarbonyl, $(C_1$-$C_4)$-Perhaloalkylaminocarbonyl, gegebenenfalls substituiertes Phenoxy-$(C_1$-$C_4)$-alkyl, gegebenenfalls substituiertes Phenyl-$(C_1$-$C_4)$-alkyl, gegebenenfalls substituiertes Phenylmercapto-$(C_1$-$C_4)$-alkyl, gegebenenfalls substituiertes Phenylamino-$(C_1$-$C_4)$-alkyl, gegebenenfalls substituiertes Phenyl-$(C_1$-$C_4)$-alkylamino, gegebenenfalls substituiertes N-Phenyl-N-$(C_1$-$C_4)$-alkylamino oder gegebenenfalls substituiertes Phenoxyphenyl-$(C_1$-$C_4)$-alkyl bedeuten, wobei unter

gegebenenfalls substituiertem Phenyl bzw. Heteroaryl vorzugsweise gegebenfalls bis zu dreifach durch gleiche oder verschiedene Rest aus der Reihe Halogen, Nitro, Cyano, $(C_1-C_6)$-Alkoxycarbonyl, $(C_1-C_6)$-Alkanyloxy, $(C_1-C_6)$-Alkyl, $(C_1-C_4)$-Alkoxy, $(C_1-C_4)$-Alkylthio, $(C_1-C_4)$-Haloalkyl und $(C_1-C_4)$-Haloalkoxy substituiertes Phenyl bzw. Heteroaryl und unter Halo die Halogenatome F, Cl, Br oder J zu verstehen sind; oder

zwei benachbarte Reste aus der Reihe,

$R^5$, $R^6$, $R^7$,$R^8$ und $R^9$ zusammen mit den dazu tragenden C-Atomen für einen teilweise ungesättigten oder aromatischen Isocyclus oder Heterocyclus mit den Heteroatomen O, N, S, Si und/oder P und 4 bis 10 Ringgliedern stehen und die übrigen Reste wie vorstehend definiert sind,

Bevorzugt sind Mittel, worin in Formel I

$R^1$ = $(C_1-C_4)$-Alkyl, Hydroxy-$(C_1-C_4)$-alkyl, Halo-$(C_1-C_4)$-alkyl, Perhalo-$(C_1-C_4)$-alkyl, $(C_1-C_4)$-Alkoxy-$(C_1-C_4)$-alkyl, Perhalo$(C_1-C_4)$-alkoxy-$(C_1-C_4)$-alkyl, Halo-$(C_1-C_4)$-alkylthio-$(C_1-C_4)$-alkyl, $(C_1-C_4)$-Alkylamino-$(C_1-C_4)$-alkyl, $(C_1-C_4)$-Dialkylamino-$(C_1-C_4)$-alkyl, $(C_3-C_9)$-Cycloalkylamino-$(C_1-C_4)$-alkyl, $(C_3-C_9)$-Cycloalkyl oder $(C_3-C_9)$-Cycloalkyl-$(C_1-C_4)$-alkyl bedeutet;

$R^2$ = Wasserstoff, Hydroxy, Amino, Mercapto, Halogen, $(C_1-C_8)$-Alkyl, Hydroxy-$(C_1-C_4)$-alkyl, Halo-$(C_1-C_4)$-alkyl, $(C_1-C_6)$-Alkoxy, $(C_2-C_6)$-Alkenyloxy, $(C_2-C_6)$-Alkinyloxy, $(C_1-C_4)$-Alkoxy-$(C_1-C_4)$-alkyl, Halo-$(C_1-C_4)$-alkoxy-$(C_1-C_4)$-alkyl, $(C_1-C_4)$-Alkylthio, Halo-$(C_1-C_4)$-alkylthio, Halo-$(C_1-C_4)$-alkylthio-$(C_1-C_4)$-alkyl, $(C_1-C_4)$-Alkylsulfonyl, $(C_1-C_4)$-Alkylsulfonyl-$(C_1-C_4)$-alkyl, $(C_1-C_4)$-Alkoxycarbonyl-$(C_1-C_4)$-alkyl, $(C_1-C_4)$-Alkylamino-$(C_1-C_4)$-alkyl, $(C_1-C_4)$-Dialkylamino-$(C_1-C_4)$-alkyl, $(C_3-C_9)$-Cycloalkylamino-$(C_1-C_4)$-alkyl oder $(C_3-C_9)$-Cycloalkyl bedeutet, wobei die cyclischen Reste bis zu dreifach durch $(C_1-C_4)$-Alkyl substituiert sein können;

$R^3$ = Wasserstoff, Hydroxy, Amino, Mercapto, $(C_1-C_8)$-Alkyl, Hydroxy-$(C_1-C_4)$-alkyl, Halo-$(C_1-C_4)$-alkyl, $(C_1-C_6)$-Alkoxy, $(C_2-C_6)$-Alkenyloxy, $(C_2-C_6)$-Alkinyloxy, ( $C_1-C_4$)-Alkoxy-$(C_1-C_4)$-alkyl, Halo-$(C_1-C_4)$-alkoxy-$(C_1-C_4)$-alkyl, $(C_1-C_4)$-Alkylthio, Halo-$(C_1-C_4)$-alkylthio, Halo-$(C_1-C_4)$-alkylthio-$(C_1-C_4)$-alkyl, $(C_1-C_4)$-Alkylsulfonyl, $(C_1-C_4)$-Alkylsulfonyl-$(C_1-C_4)$-alkyl, $(C_1-C_4)$-Alkoxycarbonyl-$(C_1-C_4)$-alkyl, $(C_1-C_4)$-Alkylamino-$(C_1-C_4)$-alkyl, $(C_1-C_4)$-Dialkylamino-$(C_1-C_4)$-alkyl, $(C_3-C_9)$-Cycloalkylamino-$(C_1-C_4)$-alkyl, $(C_3-C_9)$-Cycloalkyl, $(C_3-C_9)$-Cycloalkyl-$(C_1-C_4)$-alkyl, gegebenenfalls substituiertes Phenyl, gegebenenfalls substituiertes $(C_1-C_{10})$-Heteroaryl, gegebenenfalls substituiertes Phenoxy-$(C_1-C_4)$-alkyl oder gegebenenfalls substituiertes Phenyl-$(C_1-C_4)$-alkyl bedeutet;

$R^4$ = Wasserstoff, Formyl, $(C_1-C_4)$-Alkyl, $(C_1-C_4)$-Alkylcarbonyl, Hydroxy-$(C_1-C_4)$-alkyl, Halo-$(C_1-C_4)$-alkyl, $(C_1-C_4)$-Alkoxy-$(C_1-C_4)$-alkyl, Halo$(C_1-C_4)$-alkoxy-$(C_1-C_4)$-alkyl, $(C_2-C_6)$-Alkenyl, $(C_2-C_6)$-Alkinyl, $(C_1-C_4)$-Alkylamino-$(C_1-C_4)$-alkyl, $(C_1-C_4)$-Dialkylamino-$(C_1-C_4)$-alkyl, $(C_3-C_9)$-Cycloalkylamino-$(C_1-C_4)$-alkyl, wobei die cyclischen Reste bis zu dreifach durch $(C_1-C_4)$-Alkyl substituiert sein können, $(C_1-C_4)$-Alkoxycarbonyl-$(C_1-C_4)$-alkyl oder gegebenenfalls substituiertes Phenyl-$(C_1-C_4)$-alkyl bedeutet;

$R^5$, $R^6$, $R^7$, $R^8$ und $R^9$ gleich oder verschieden sind und unabhängig voneinander Wasserstoff, Halogen, Hydroxy, Amino, Nitro, Cyano, $(C_1-C_4)$-Alkyl, $(C_1-C_4)$-Alkoxy, $(C_1-C_4)$-Alkylamino, $(C_1-C_4)$-Dialkylamino, $(C_1-C_4)$-Alkylcarbonylamino, Halo-$(C_1-C_4)$-alkyl, Perhalo-$(C_1-C_4)$-alkyl, Hydroxy-$(C_1-C_4)$-alkyl, Dihydroxy-$(C_1-C_4)$-alkyl, $(C_1-C_4)$-Alkoxy-$(C_1-C_4)$-alkyl, Halo$(C_1-C_4)$-alkoxy-$(C_1-C_4)$-alkyl, $(C_1-C_4)$-Alkylthio, Halo-$(C_1-C_4)$-alkylthio, Halo-$(C_1-C_4)$-alkylthio-$(C_1-C_4)$-alkyl, $(C_1-C_4)$-Alkylthio-$(C_1-C_4)$-alkyl, $(C_2-C_6)$-Alkenyl, $(C_2-C_6)$-Alkinyl, $(C_1-C_4)$-Alkylamino-$(C_1-C_4)$-alkyl, $(C_1-C_4)$-Dialkylamino-$(C_1-C_4)$-alkyl, $(C_3-C_9)$-Cycloalkylamino-$(C_1-C_4)$-alkyl, $(C_3-C_9)$-Cycloalkyl, $(C_3-C_9)$-Cycloalkyl-$(C_1-C_4)$-alkyl, $(C_3-C_9)$-Heterocycloalkyl-$(C_1-C_4)$-alkyl, wobei die cyclischen Reste bis zu dreifach durch $(C_1-C_4)$-Alkyl substituiert sein können, gegebenenfalls substituiertes Phenyl, gegebenenfalls substituiertes $(C_1-C_{10})$-Heteroaryl, gegebenenfalls substituiertes Phenoxy, gegebenenfalls substituiertes Benzoyl-$(C_1-C_4)$-alkyl, $(C_1-C_4)$-Alkoxycarbonyl-$(C_1-C_4)$-alkyl, gegebenenfalls substituiertes Phenyloxycarbonyl-$(C_1-C_4)$-alkyl, $(C_1-C_4)$-Alkylaminocarbonyl-$(C_1-C_4)$-alkyl, $(C_1-C_4)$-Dialkylaminocarbonyl-$(C_1-C_4)$-al-

kyl, ($C_1$-$C_4$)-Alkylcarbonyl, ($C_1$-$C_4$)-Haloalkylcarbonyl, ($C_1$-$C_4$)-Alkyloxycarbonyl, ($C_1$-$C_4$)-Haloalkyloxycarbonyl, ($C_1$-$C_4$)-Alkylthiocarbonyl, ($C_1$-$C_4$)-Haloalkylthiocarbonyl, ($C_1$-$C_4$)-Perhaloalkylthiocarbonyl, Aminocarbonyl, ($C_1$-$C_4$)-Alkylaminocarbonyl, ($C_1$-$C_4$)-Haloalkylaminocarbonyl, ($C_1$-$C_4$)-Perhaloalkylaminocarbonyl, gegebenenfalls substituiertes Phenoxy-($C_1$-$C_4$)-alkyl, gegebenenfalls substituiertes Phenyl-($C_1$-$C_4$)-alkyl, gegebenenfalls substituiertes Phenylmercapto-($C_1$-$C_4$)-alkyl oder gegebenenfalls substituiertes Phenylamino-($C_1$-$C_4$)-alkyl bedeuten; oder

zwei der Reste

$R^5$, $R^6$, $R^7$,$R^8$ und $R^9$ zusammen mit den diese tragenden C-Atomen für einen teilweise ungesättigten oder aromatischen Isocyclus oder Heterocyclus mit den Heteroatomen O, N und/oder S und 4 bis 10 Ringgliedern stehen und die anderen Reste wie vorstehend definiert sind.

Insbesondere hervorzuheben sind solche Mittel, worin in Formel I,

$R^1$ = ($C_1$-$C_4$)-Alkyl, Halo-($C_1$-$C_4$)-alkyl oder Perhalo-($C_1$-$C_4$)-alkyl bedeutet, und unter Halo die Halogenatome Cl und insbesondere F zu verstehen sind;

$R^2$ = Wasserstoff, Hydroxy, Amino, Mercapto, Halogen, ($C_1$-$C_8$)-Alkyl, ($C_1$-$C_6$)-Alkoxy, ($C_2$-$C_6$)-Alkenyloxy, ($C_2$-$C_6$)-Alkinyloxy, ($C_1$-$C_4$)-Alkylthio, ($C_1$-$C_4$)-Alkylsulfonyl, ($C_1$-$C_4$)-Alkylamino-($C_1$-$C_4$)-alkyl oder ($C_1$-$C_4$)-Dialkylamino-($C_1$-$C_4$)-alkyl bedeutet;

$R^3$ = Wasserstoff, Hydroxy, Amino, Mercapto, ($C_1$-$C_8$)-Alkyl, ($C_1$-$C_4$)-Alkylthio, gegebenenfalls substituiertes Phenyl oder gegebenenfalls substituiertes ($C_1$-$C_8$)-Heteroaryl bedeutet, wobei unter gegebenenfalls substituiertem Phenyl bzw. Heteroaryl vorzugsweise gegebenenfalls bis zu dreifach durch Halogen, Nitro, Cyano, ($C_1$-$C_6$)-Alkoxycarbonyl, ($C_1$-$C_6$)-Alkyl, ($C_1$-$C_4$)-Alkoxy, ($C_1$-$C_4$)-Alkylthio und/oder ($C_1$-$C_4$)-Haloalkyl substituiertes Phenyl bzw. Heteroaryl zu verstehen ist;

$R^4$ = Wasserstoff, Formyl, ($C_1$-$C_4$)-Alkyl,

$R^5$, $R^6$, $R^7$, $R^8$ und $R^9$ gleich oder verschieden sind und unabhängig voneinander gleich Wasserstoff, Halogen, Hydroxy, Amino, Nitro, Cyano, ($C_1$-$C_4$)-Alkyl, ($C_1$-$C_4$)-Alkoxy, ($C_1$-$C_4$)-Alkylamino, ($C_1$-$C_4$)-Dialkylamino, gegebenenfalls substituiertes Phenyl oder gegebenenfalls substituiertes Phenoxy bedeuten und unter Halo die Halogenatome F, Cl, Br zu verstehen sind,

oder zwei der Reste

$R^5$, $R^6$, $R^7$,$R^8$ und $R^9$ zusammen mit den diese tragenden C-Atome für einen teilweise ungesättigten oder aromatischen Isocyclus oder Heterocyclus mit den Heteroatomen O und/oder N und 5 bis 10 Ringgliedern stehen und die anderen Reste wie vorstehend definiert sind.

Die Erfindung betrifft weiterhin Verbindungen der Formel I, deren Tautomere, sowie deren Säureadditionssalze, worin $R^1$-$R^9$ wie oben definiert sind, ausgenommen jene Verbindungen der Formel I, worin $R^1$ = $CF_3$, $R^2$ = Cl, $R^3$ = H, $R^4$ = H und $R^5$ bis $R^9$ = 3,5-Dichlor, 2,6-Difluor, 3-Trifluormethyl oder 4-Trifluormethoxy bedeuten oder worin $R^1$ = $CF_3$, $R^2$ = $NH_2$, $R^3$ = H, $R^4$, = H und $R^5$ bis $R^9$ = 4-Fluor, 4-Trifluormethoxy, 2-Methyl-4-fluor oder 2-Methylthio bedeuten.

In den vorstehenden Definitionen und im folgenden können (sofern im einzelnen nicht anders festgelegt) Alkyl, Alkenyl und Alkinyl geradkettig oder verzweigt sein. Entsprechendes gilt für die davon abgeleiteten Reste wie z. B. Alkoxy, Alkenyloxy, Alkinyloxy, Alkylthio, Alkanoyl.

Unter Haloalkyl und davon abgeleiteten Resten wie z. B. Haloalkoxy versteht man Reste, worin ein oder mehrere Wasserstoffatome des Alkylteils durch Halogen ersetzt sind. Bei Perhaloalkyl und davon abgeleiteten Resten sind alle Wasserstoffatome des Alkylteils durch Halogen ersetzt.

Halogen ist Fluor, Chlor, Brom oder Iod.

Cycloalkyl ist ein mono-, bi- oder tricyclischer Rest wie Cyclobutyl, Cyclopentyl, Cyclohexyl oder Norbornyl. Unter einem Heterocycloalkylrest ist ein Cycloalkylrest zu verstehen, worin mehrere, vorzugsweise bis zu zwei $CH_2$ durch O, S und/oder NH ersetzt sind und/oder worin das C-Atom in der Radikalposition durch N ersetzt ist; Beispiele sind Piperidino, Piperizino, Morpholino.

Unter Heteroaryl versteht man einen von Aryl, vorzugsweise Phenyl oder Naphthyl, abgeleiteten Rest, worin mindestens eine CH-Einheit durch N, P und/oder SiH ersetzt ist und/oder mindestens zwei benachbarte CH-Einheiten durch NH, PH, S und/oer O ersetzt sind. Beispiele solcher Reste sind Thionyl, Benzothionyl, Furyl, Benzofuryl, Pyrrolyl, Imidazolyl, Pyrazolyl, Pyridyl, Pyrazinyl, Pyrimidinyl, Pyridazinyl, Indolizinyl,

Isoindolyl, Indolyl, Indazolyl, Purinyl, Chinolyl, Isochinolyl, Phthalazinyl, Naphthyridinyl, Chinoxalinyl, Chinazolinyl, Chinnolinyl, Pferidinyl, Oxazolyl, Isoxazolyl, Thiazolyl, Isothiazolyl, Tetrazolyl.

Unter aromatischen Isocyclus versteht man insbesondere Benzol oder Naphthalin, unter einem aromatischen Heterocyclus insbesondere die den obengenannten Heteroarylresten zugrundliegenden Verbindungen. Von diesem Iso- bzw. Heterocyclen sind die teilweise ungesättigten Iso- bzw. Heterocyclen abgeleitet, die daraus formal durch Addition von 2, 4, 6 oder 8 Wasserstoffatomen entstehen.

Zur Herstellung der Säureadditionssalze der Verbindungen der Formel I kommen folgende Säuren in Frage: Halogenwasserstoffsäuren, wie Chlorwasserstoffsäure oder Bromwasserstoffsäure, weiterhin Phosphorsäure, Salpetersäure, Schwefelsäure, mono- oder bifunktionelle Carbonsäuren und Hydroxycarbonsäuren wie Essigsäure, Maleinsäure, Bernsteinsäure, Fumarsäure, Weinsäure, Citronensäure, Salicylsäure, Sorbinsäure oder Milchsäure, sowie Sulfonsäuren wie p-Toluolsulfonsäure oder 1,5-Naphthalindisulfonsäure. Die Säureadditionssalze der Verbindungen der Formel I können in einfacher Weise nach den üblichen Salzbildungsmethoden, z. B. durch Lösen einer Verbindung der Formel I in einem geeigneten organischen Lösungsmittel und Hinzufügen der Säure erhalten werden und in bekannter Weise, z. B. durch Abfiltrieren, isoliert und gegebenenfalls durch Waschen mit einem inerten organischen Lösemittel gereinigt werden.

Die neuen Pyrimidine der Formel I können nach bekannten Verfahren oder in Analogie zu bekannten Verfahren oder nach neuen Verfahren hergestellt werden.

Bespielhaft seien einige Verfahren genannt, nach denen die erfindungsgemäßen Verbindungen hergestellt werden können:

1. Ausgehend von Acetessigsäureaniliden der Formel II kann man durch Kondensation mit beispielsweise Orthoameisenester die entsprechenden 2-Acetyl-3-ethoxy-acrylsäureanilide der Formel III herstellen, die dann mit (Thio)-Harnstoff oder (Thio)-Harnstoff-Derivaten, aber auch Guanidinen oder deren Derivaten zu den Pyrimidinen der Formel I cyclysiert werden können. Dieses Verfahren ist angelehnt an entsprechende literaturbekannte Verfahren, die von den analogen Ethoxymethylenmalondinitrilen ausgehen (vgl. Comprehensive Heterocyclic Chemistry, A. R. Katritzky, C. W. Rees, Pergamon Press, Oxford, New York, 1984, Vol. 3, S. 126 ff). Bei diesem Verfahren können auch Acrylsäureanilide mit anderen Abgangsgruppen, z. B. Dimethylamino (vgl. G. Roma et al.; Farmaco. Ed. Sci. 1983, 38(8), 546 - 558) eingesetzt werden. in den Verbindungen der Formeln II und III haben $R^1$ bis $R^9$ die obengenannten Bedeutungen; X ist eine Abgangsgruppe.

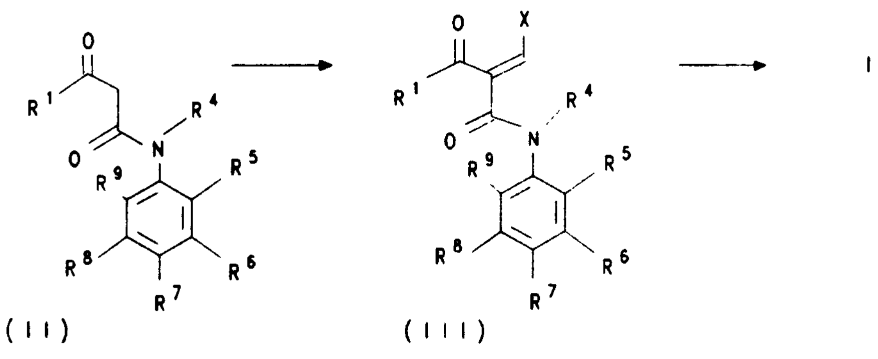

(II) (III) I

2. Ebenso kann man ausgehend von Acetessigsäureaniliden der Formel II durch Kondensation mit Aldehyden und (Thio)-Harnstoff, (Thio)-Harnstoff-Derivaten oder Guanidinen der Formel IV oder dessen Derivaten im Sinne einer Biginelli-Reaktion und nachfolgender Oxydation die entsprechenden Pyrimidin-Anilide erhalten (T. Kato, et al.; Yakugaku Zasshi 1981, 101(2), 182-5, Chem.Abstr. 95:62117; Z. Kahil, et al.; J. Indian Chem. Soc. 1981, 58(5), 494-7, Chem.Abstr. 95:97711; G. Dubars, et al.; Khim. Geterotsikl. Soedin. 1976, 12, 220; Chem. Heterocycl. Compd.(Engl. Transl.) 1976, 12, 191, Chem.Abstr. 85:32946; A. Machon, A. Dlugosz; Pol. J. Pharmacol. Pharm. 1973, 25(6), 579, Chem.Abstr. 80:95864). In den folgenden Formeln haben $R^1$ bis $R^9$ die obengenannten Bedeutungen:

II +

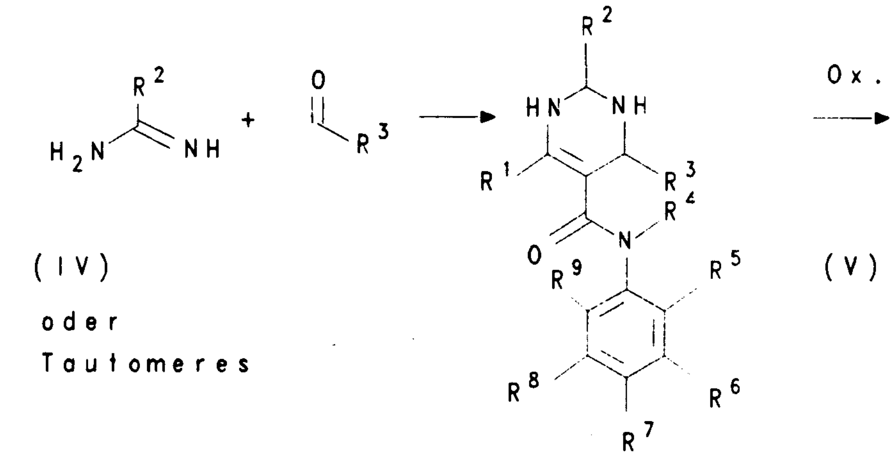


(IV) oder Tautomeres

(V)

I

3. Verbindungen der Formel I können auch ausgehend von entsprechenden Pyrimidin-5-carbonsäureestern der Formel VI hergestellt werden. Die Ester können entweder mit Anilinen der Formel VIII zur Reaktion gebracht werden oder verseift werden und dann, z. B. via Säurechlorid, in das entsprechende Anilid überführt werden.

(VIII)

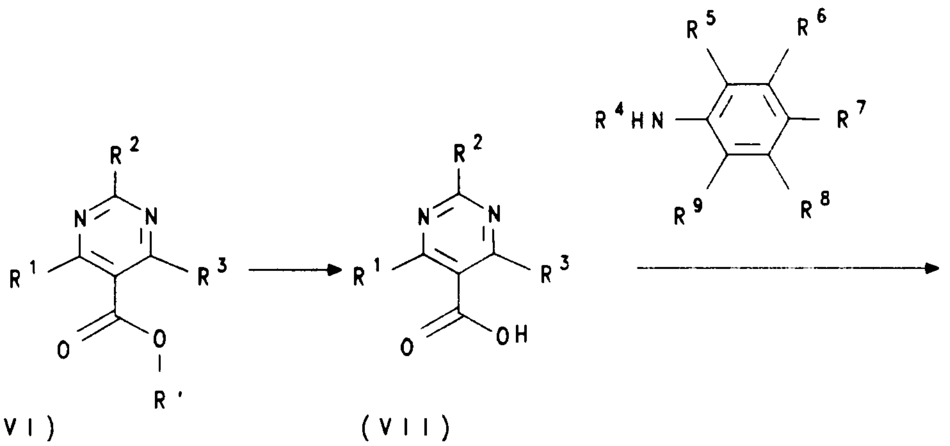

I

(VI) (VII)

R' bedeutet in dieser und in den nachfolgenden Formeln eine Gruppe, die mit einer Carbonsäure ein Ester bildet wie Z. B. ($C_1$-$C_4$)-Alkyl, Benzyl oder ähnliche, die übrigen Reste sind wie oben definiert.

In diese Reaktionssequenz kann auch ein Gemisch aus Verbindungen entsprechend der Formel VI und VI' eingesetzt werden. Im Falle $R^1$ = Perhaloalkyl (insbesondere $R^1$ = $CF_3$) erhält man bei der Verseifung ein Gemisch wechselnder Zusammensetzung aus einer Verbindung entsprechend Formel VII und dessen hydratisierter Form (Formel VII'). Dieses Gemisch kann ohne Trennung in nachfolgende Schritte der Reaktionssequenz eingesetzt werden.

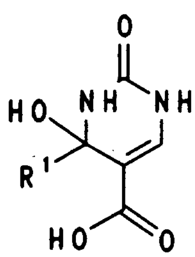

VII'

Stellt $R^2$ eine Abgangsgruppe wie z.B. $-S-R^1$, $-SO_2-R^1$ oder $-O-R^1$ dar, so kann diese Gruppe bei der alkalischen Verseifung gegen $R^2$ = OH ausgetauscht werden (analoge Vorschrift: J. Arkuwe, K. Undheim; Acta Chem. Scand.; 1986, B40(9), 764). Auch in dieser Reaktion erhält man im Falle $R^1$ = Perhaloalkyl (insbesondere $R^1$ = $CF_3$) ein Gemisch wechselnder Zusammensetzung aus einer Verbindung entsprechend Formel VII und deren hydratisierter Form (Formel VII').

Überführt man Säuren entsprechend der Formel VII mit $R^2$ = OH in die entsprechenden Säurechloride, so kann man die Hydroxy-Stufe in dem gleichen Reaktionsschritt mit Phosphoroxyhalogeniden in das entsprechende Halogen-Derivat ($R^2$ = z.B. Cl oder Br) umwandeln.

Setzt man bei der Umwandlung der Säuren in die entsprechenden Säurechloride Verbindungen Gemische des Typs VII/VII' ein, so wird der hydratisierte Anteil, wenn man z.B. Mischungen aus Phosphoroxyhalogeniden und Thionylhalogeniden als halogenierende Reagenzien einsetzt, gleichzeitig dehydratisiert.

Die Ester der Formel VI können nach verschiedenen Methoden hergestellt werden; einige Verfahren seien beispielhaft genannt.

a. Durch basenkatalysierte Kondensation von Acrylestern der Formel X mit (Thio)-Harnstoff oder (Thio)-Harnstoff-Derivaten, aber auch Guanidinen oder dessen Derivate (Formel IV) (R. Urban, O. Schnider; Helv. Chim. Acta 1958, 41, 1806). Insbesondere bei der Verwendung von Harnstoff in dieser Reaktionssequenz kann die Kondensation auch in saurem Medium (z.B. HCl/Wasser oder Essigsäure) durchgeführt werden (analoge Vorschriften: R.S. Vartanyan; et. al.; Khimiya Geterosikl. Soedin.; 1982; 11; 1558-1559).

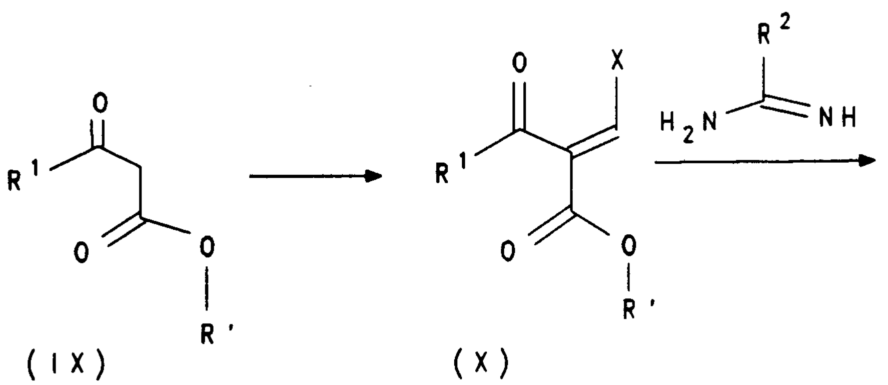

(IX) (X) VI

Im Falle $R^1$ = Perhaloalkyl (insbesondere $R^1$ = $CF_3$) erhält man bei der sauren Kondensation mit Harnstoff ein Gemisch wechselnder Zusammensetzung aus der einer Verbindung entsprechend Formel VI und deren hydratisierter Form (Formel VI'). Dieses Gemisch kann ohne Trennung in nachfolgende Schritte der Reaktionssequenz eingesetzt werden.

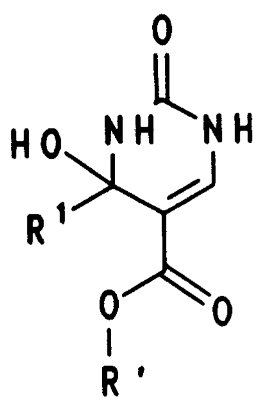

VI'

b. Durch Umsetzung von Acetessigsäurestern der Formel XI mit Verbindungen der Formel IV im Sinne einer Biginelli-Reaktion (Rutter et al., J. Frankin Inst. 1954, 258, 413) und nachfolgender Oxydation (M. S. Akhtar, et al., Indian J. Chem., Sect. B 1987, 26B, 556; Chem.Abstr. 108:150408).

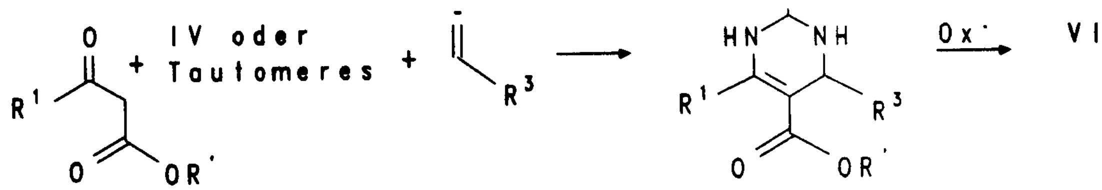


(XI) (XII)

c. Pyrimidin-5-carbonsäureester der Formel VI kann man auch durch Kondensation von Malonsäurederivaten der Formel XV mit Nitrilen der Formel XIII und XIV herstellen (Y. L. Sing, L. F. Lee; J. Heterocyclic Chem. 1989, 26, 7, Chem.Abstr. 111:39305).

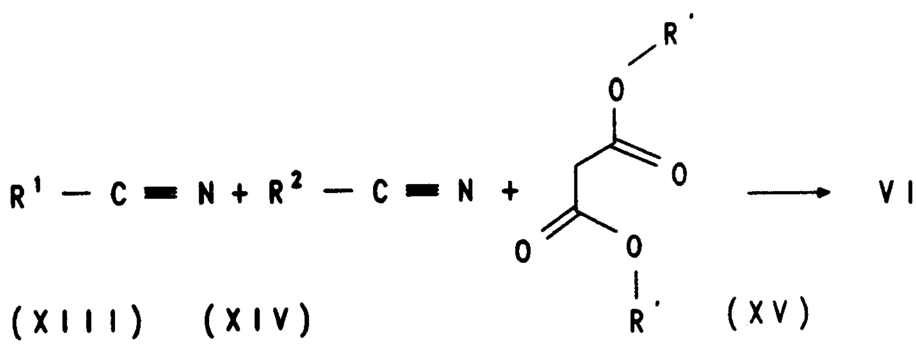

(XIII) (XIV) (XV)

d. Als weitere Methode zur Darstellung Von Pyrimidin-5-carbonsäuren und entsprechender Ester der Formel VI sei der Halogen-Lithium-Austausch mit anschließender Carboxylierung genannt (M. D. Metha, D. Miller; J. Chem. Soc. 1965, 6695; Chem.Abstr. 64:5089d).

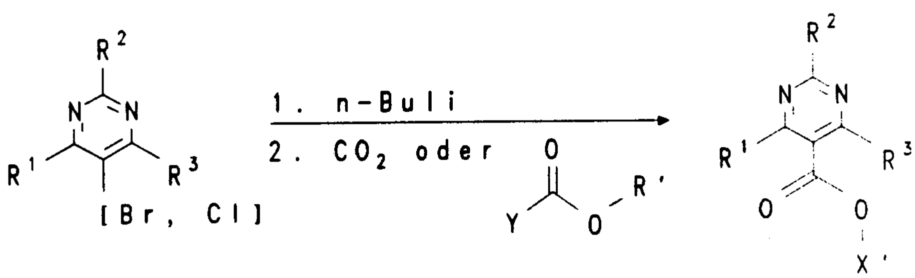


(XVI) (XVII)

X': H, R' Y: Halogen oder andere Abgangsgruppe

4. Verbindungen der Formel I können auch ausgehend von Pyrimidinyl-5-perhaloethanonen (Formel XVIII) durch Reaktion mit Anilinen der Formel VIII hergestellt werden.

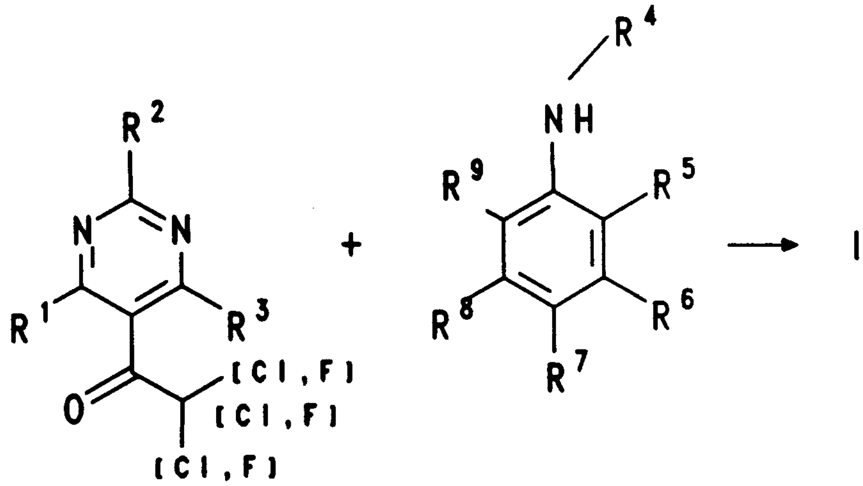

XVIII VIII

Die Edukte entsprechend der Formel XVIII können nach verschiedenen Verfahren hergestellt werden.

a. Durch Haloform-Reaktion entsprechender Ethanone.

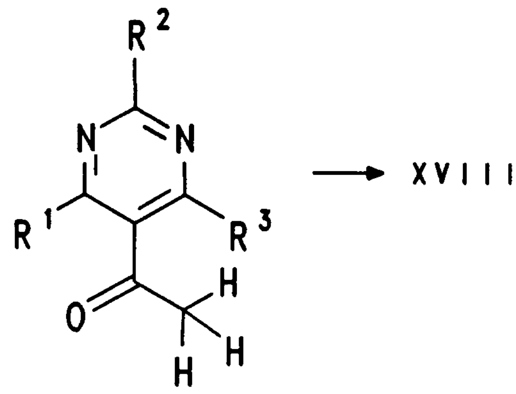

XIX

b. Durch basische oder saure Kondensation entsprechender Diketone analog der Formel XX mit (Thio)-Harnstoff oder (Thio)-Harnstoff-Derivaten, aber auch mit Guanidinen oder dessen Derivaten (Formel IV) der unter 3a. beschriebenen Kondensationsverfahren. Im Falle $R^1$ = Perhaloalkyl (insbesondere $R^1$ = $CF_3$) erhält man bei der sauren Kondensation mit Harnstoff ein Gemisch wechselnder Zusammensetzung aus einer Verbindung entsprechend Formel XVIII und deren hydratisierter Form (Formel XVIII'). Dieses Gemisch kann ohne Trennung in nachfolgende Schritte der Reaktionssequenz eingesetzt werden.

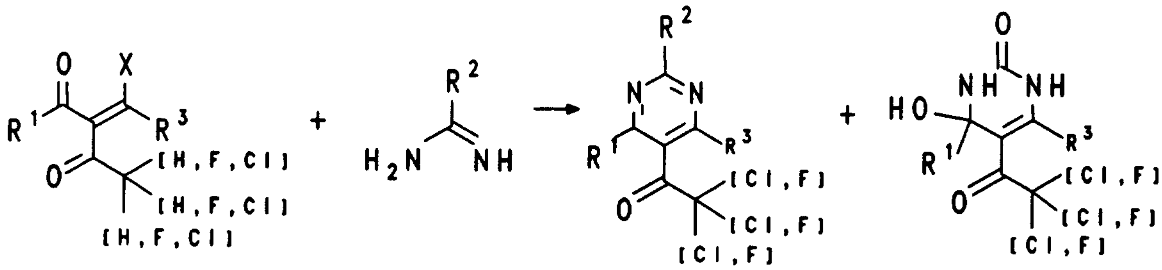

XX IV XVIII XVIII'

Bei entsprechenden Verbindungen der Formel XVIII mit $R^2$ = OH bzw. Gemischen aus XVIII und XVIII' lassen sich die Hydroxy-Funktionen in Halogene durch Behandlung mit Phosphoroxychloriden umwandeln ($R^2$ = z.B. Cl oder Br) wobei auch in diesem Falle der hydratisierte Anteil entwässert wird.

Die Zwischenprodukte der allgemeinen Formel XX lassen sich nach verschiedenen Verfahren herstellen, beispielsweise

b.a.) durch Reaktion der Diketone XXI mit Verbindungen der allgemeinen Formel XXII, (entsprechende Verfahren sind veröffentlicht von:

1. Tsuge Otohiko, Wada Eiji, Kanemasa Shuji, Sakoh Hirohiko; Bull. Chem. Soc. Jap.; 1984, 57-(11), 3221-3233;
2. G. Menozzi, et al.; J. Het. Chem., 1987, 24, 1669;
3. D. Villemin, A.B. Alloum, Synthesis, 1991 (4), 301).

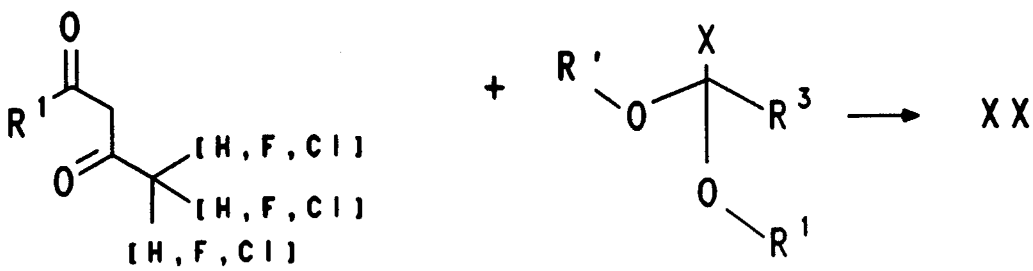

XX

XXI XXII

b.b.) durch Acylierung der entsprechenden Olefine der allgemeinen Formel XXIV in Abwandlung literaturbekannter Verfahren (M. Hajo.; Synthesis, 1990, 347).

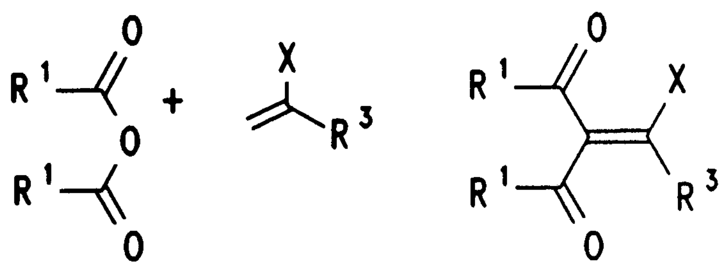

XXIII XXIV XX

b.c.) asymetrisch substituierte Verbindungen durch Abwandlung dieses Verfahrens in ein zweistufiges, die hierzu benötigten Edukte lassen sich nach literaturbekannten Verfahren (M. Hajo; Chem. Lett.; 1976, 499 oder A.M. Platoshkin; Izv. Akad. Nauk SSR, Ser. Khim.; 1969, 1, 112-116, CA 70:114541c) herstellen.

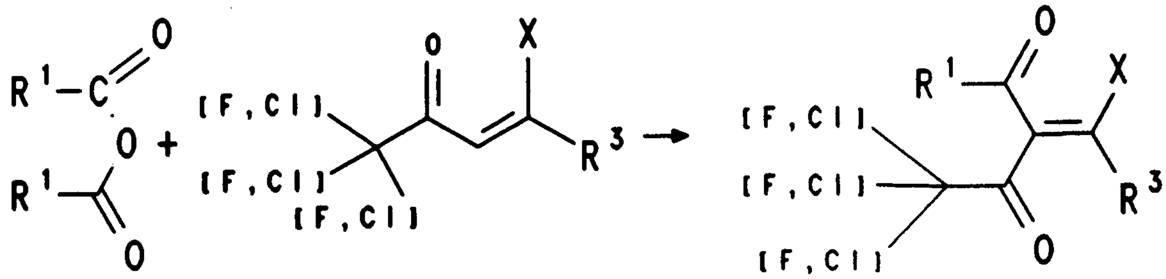

XXIII XXV XX

5. Auf diese Weise erhaltene Verbindungen der Formel I können nachträglich unter Bildung weiterer Verbindungen der Formel I modifiziert werden. Nachfolgend sind einige geeignete Methoden aufgelistet:

a. Oxydation von Teilstrukturen im Molekül, wie zum Beispiel von Methylmercapto-Einheiten mit Oxydationsmitteln wie $H_2O_2$ oder ®Oxone (Kaliumperoxomonosulfat, $2KHSO_5 x KHSO_4 x K_2SO_4$) (vgl. Comprehensive Heterocyclic Chemistry, A. R. Katritzky, C. W. Rees, Pergamon Press, Oxford, New York, 1984, Vol. 3, S. 96; D. J. Brown, B. T. England; J. Chem. Soc. (C); 1971, 256).

b. Die Synthese von halogensubstituierten, (insbesondere Chlor) Pyrimidin-5-carbonsäureestern kann beispielsweise durch die Umwandlung entsprechender Hydroxy-Vorstufen mit Phosphoroxyhalogeniden erfolgen (vgl. Comprehensive Heterocyclic Chemistry, A. R. Katritzky, C. W. Rees, Pergamon Press, Oxford, New York, 1984, Vol. 3, S. 139).

c. Austausch von Abgangsgruppen im Molekül wie zum Beispiel Chlor, die Methylmercapto-Gruppe (W. Schaper; Synthesis 1985, 861) oder die Sulfonylmethylgruppe (vgl. Comprehensive Heterocyclic Chemistry, A. R. Katritzky, C. W. Rees, Pergamon Press, Oxford, New York, 1984, Vol. 3, S. 97) mit Nucleophilen.

Die erfindungsgemäßen Verbindungen der Formel I zeichnen sich durch hervorragende fungizide Wirkung aus. Sie können zum Schutze verschiedener Kulturpflanzen gegen phytopathogene Mikroorganismen, insbesondere Fungi, eingesetzt werden, wobei sie sich durch eine besonders hohe Kulturpflanzenverträglichkeit auszeichnen. Sie besitzen vorteilhafte präventive und systemische Eigenschaften, bereits in das pflanzliche Gewebe eingedrungene pilzliche Krankheitenerreger lassen sich auch erfolgreich kurativ bekämpfen. Durch Besprühen, Bestäuben oder andere Applikationen mit Wirkstoffen der Formel I können Pflanzen und bestehende oder zuwachsende Pflanzenteile vor auftretenden Schädlingen geschützt werden. Sie eignen sich auch als Beizmittel zur Behandlung von Saatgut und Stecklingen zum Schutze vor Pilzinfektionen sowie im Erdboden auftretende pathogene Pilze. Das Wirkungsspektrum der beanspruchten Verbindungen erfaßt eine Vielzahl verschiedener wirtschaftlich bedeutender, phytopathogener Pilze, wie beispielhaft Alternaria mali; Botrytis cinerea, Benzimidazol- und/oder Dicarboximid- sensible und/oder resistente Stämme, Sclerotinia sclerotinorum sowie weitere Grauschimmelarten; Cercospora beticola; Ceratobasidium cerealis; Erysiphe graminis, Erysiphe graminis hordei, Erysiphe chichoracearum sowie andere Echte Mehltauarten; Fusarium culmorum und andere Fusariumarten; Moniliniaarten; Leptosphaeria nodorum sowie andere Septoriaarten und Blattflecken verursachende Arten; Pellicularia sasakii, Piricularia oryzae und andere Reispilzsarten; Phytophthora infestans, Phytophtora capsici und verschiedene andere Kraut-und Knollenfäulepilze; Plasmopara viticola, Pseudopernospora cubensis und weitere Pernospora oder falsche Mehltauarten; Pseudocercosporella herpotrichoides und verschiedene andere Augenflecken bzw. Halmbruch verursachende Pilzarten; Puccinia recondita und verschiedene andere Rostpilze; Pyrenophora teres und andere Drechslerarten; Ustilagoarten; Venturia inaequalis und Schorfarten.

Das Wirkungsspektrum der beanspruchten Verbindungen betrifft schwerpunktmäßig den wirtschaftlich bedeutenden, phytopathogenen Pilz Plasmopara viticola verbunden mit Nebenwirkung gegenüber Phytophthora infestans und Botrytis cinerea.

Die erfindungsgemäßen Verbindungen eignen sich daneben auch für den Einsatz in technischen Bereichen, beispielsweise als Holzschutzmittel, als Konservierungsmittel in Anstrichfarben, in Kühlschmiermittel für die Metallbearbeitung oder als Konservierungsmittel in Bohr- und Schneidölen.

Neben Verbindungen der Formel I enthalten die erfindungsgemäßen Mittel geeignete Formulierungshilfsmittel. Der Gehalt an Wirkstoff der Formel I liegt im allgemeinen zwischen 1 und 95 Gew.-%.

Sie können auf verschiedene Art formuliert werden, je nachdem wie es durch die biologischen und/oder chemisch-physikalischen Parameter vorgegeben ist. Als Formulierungsmöglichkeiten kommen daher in Frage: Spritzpulver (WP), emulgierbare Konzentrate (EC), wäßrige Dispersionen auf Öl- oder Wasserbasis (SC), Suspoemulsionen (SG), Stäubemittel (DP), Beizmittel, Granulate in Form von wasserdispergierbare

Granulate (WG), ULV-Formulierungen, Mikrokapseln, Wachse oder Köder.

Diese einzelnen Formulierungstypen sind im Prinzip bekannt und werden beispielsweise beschrieben in: Winnacker-Küchler, "Chemische Technologie", Band 7, C-Hauser Verlag München, 4. Aufl., 1986; van Falkenberg, "Pesticides Formulations", Marcel Dekker N.Y., 2nd Ed., 1972 - 73; K. Martens, "Spray Drying Handbook", 3rd Ed., 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in:
Watkins, "Handbook of Insecticide Dust Diluents and Carrier", 2nd Ed., Darland Books, Caldwell N. J.; H. v. Olphen, "Introduction to Clay Colloid Chemistry", 2nd Ed., J. Wiley & Sons, N.Y.; Marschen, "Solvents Guide", 2nd Ed., Interscience, N.Y., 1950; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y., 1964; Schönfeldt, "Grenzflächenaktive Äthylenoxidaddukte", Wiss. Verlagsges., Stuttgart, 1976; Winnacker-Küchler, "Chemische Technologie", Band 7, C. Hauser Verlag München, 4. Aufl., 1986.

Auf der Basis dieser Formulierungen lassen sich auch Kombinationen mit anderen pestizid wirksamen Stoffen, Düngemitteln und/oder Wachstumsregulatoren herstellen, z. B. in Form einer Fertigformulierung oder als Tankmix.

Spritzpulver sind in Wasser gleichmäßig dispergierbare Präparate, die neben dem Wirkstoff außer einem Verdünnungs-oder Inertstoff noch Netzmittel, z. B. polyoxethylierte Alkylphenole, polyoxethylierte Fettalkohole, Alkyl- oder Alkylphenolsulfonate und Dispergiermittel, z. B. ligninsulfonsaures Natrium, 2,2'-dinaphthylmethan-6,6'-disulfonsaures Natrium, dibutylnaphthalin-sulfonsaures Natrium oder auch oleylmethyltaurinsaures Natrium enthalten. Emulgierbare Konzentrate werden durch Auflösen des Wirkstoffes in einem organischen Lösungsmittel, z. B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden:

Alkylarylsulfonsaure Calcium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-/ Ethylenoxid-Sorbitanfettsäureester, Polyoxyethylensorbitan-Fettsäureester oder Polyoxethylensorbitester.

Stäubemittel erhält man durch Vermahlen des Wirkstoffes mit fein verteilten festen Stoffen, z. B. Talkum, natürlichen Tonen wie Kaolin, Bentonit, Poryphillit oder Diatomeenerde. Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration z. B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-%. Bei Granulaten hängt der Wirkstoffgehalt zum Teil davon ab, ob die wirksame Verbindung flüssig oder fest vorliegt und welche Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z. B. bei Spritzpulvern, emulgierbaren Konzentraten, Dispersionen und teilweise auch bei Mikrogranulaten mittels Wasser.

Staubförmige und granulierte Zubereitungen sowie versprühbare Lösungen werden vor der Anwendung üblicherweise nicht mehr mit weiteren inerten Stoffen verdünnt.

Mit den äußeren Bedingen wie Temperatur, Feuchtigkeit u. a. variiert die erforderliche Aufwandmenge. Sie kann innerhalb weiter Grenzen schwanken, z. B. zwischen 0,005 und 10,0 kg/ha oder mehr Aktivsubstanz, vorzugsweise liegt sie jedoch zwischen 0,01 und 5 kg/ha.

Die erfindungsgemäßen Wirkstoffe können in ihren handelsüblichen Formulierungen entweder allein oder in Kombination mit weiteren, literaturbekannten Fungiziden angewendet werden.

Als literaturbekannte Fungizide, die erfindungsgemäß mit den Verbindungen der Formel I kombiniert werden können, sind z. B. folgende Produkte zu nennen:

Anilazine, Benalaxyl, Benodanil, Benomyl, Binapacryl, Bitertanol, Buthiobat, Captafol, Captan, Carbendazim, Carboxin, CGD-94240F, Chlobenzthiazone, Chlobenzthiazone, Chlorthalonil, Cymoxanil, Cyproconazole, Cyprofuram, Dichlofluanid, Dichlomezin, Diclobutrazol, Diethofencarb, Difluconazole, Dimethirimol, Dimethomorph, Diniconazole, Dinocap, Dithianon, Dodemorph, Dodine, Edifenfos, Ethirimol, Etridiazol, Fenarimol,

Fenfuram, Fenpiclonil, Fenpropidin, Fenpfopomorph, Fentinacetate, Fentinhydroxide, Fluaziram, Fluobenzimine, Fluorimide, Flusilazole, Flutolanil, Flutriafol, Folpet, Foserylaluminium, Fuberidazole, Furalaxyl, Fürmecyclox, Güazatine, Hexaconazole, Imazalil, Iprobenfos, Iprodione, Isoprothiolane, Kupferverbindungen wie Cu-oxychlorid, Oxine-Cu, Cin-oxide, Mancozeb, Maneb, Mepanipyrim, Mepronil, Metalaxyl, Methasulfocarb, Methfuroxam, Myclobintanil, Nabam, Nitrothalidopropyl, Nuarimol, Ofurace, Oxadixyl, Oxycarboxin, Penconazol, Pencycuron, PP969, Probenazole, Probineb, Prochloraz, Procymidon, Propamocarb, Propiconazol, Prothiocarb, Pyracarbolid, Pyrifenox, Pyroqinilon, Rabenzazole, Schwefel, Tebuconazole, Thiabendazole, Thiofanatemethyl, Thiram, Tolclofos-methyl, Tolylfluanid, Triadimefon, Triadimenol, Tricyclazole, Tridemorph, Triflumizol, Triforine, Vinchlozolin, Zineb;

Natrium-dodecylsülfonat, Natrium-dodecylsulfat, Natrium-C13/C15-alkoholethersulfonat, Natrium-cetostearylphosphatester, Dioctylnatriumsulfosinccinat, Natrium-isopropylnaphthalinsülfonat, Natriummethylenbisnaphthalinsulfonat, Cetyl-trimethyl-ammoniumchlorid, Salze von langkettigen primärem, sekundären oder tertiären Aminen, Alkyl-propylenamine, Lauryl-pyrimidiniumbromid, ethoxilierte quarternierte Fettamine, Alkyldimethylbenzyl-ammoniumchlorid und 1-Hydroxyethyl-2-alkyl-imidazolin.

Die oben genannten Kombinationspartner sind bekannte Wirkstoffe, die zum großen Teil in Ch. R. Worthing, The Pesticide Manual, 9. Auflage (bzw. auch frühere Auflagen), British Crop Protection Council 1991 und The Agrochemicals Handbook, 3.Ed.; The Royal Society of Chemisty, 1991 beschrieben sind.

Darüber hinaus kann der erfindungsgemäße Wirkstoff in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden oder Herbiziden vorliegen. Zu den Insektiziden zählen beispielsweise Phosphorsäureester, Carbamate, Carbonsäureester, Formamidine, Zinnverbindungen, durch Mikroorganismen hergestellte Stoffe u.a.

Bevorzugte Mischungspartner sind:

1. Aus der Gruppe der Phosphorverbindungen

Acephate, Azamethiphos, Azinphos-ethyl, Azinphos-methyl, Bromophos, Bromophos-ethyl, Chlorfenvinphos, Chlormephos, Chlorpyrifos, Chlorpyrifos-methyl, Demeton, Demeton-S-methyl, Demeton-S-methyl sulphone, Dialifos, Diazinon, Dichlorvos, Dicrotophos, 0,0-1,2,2,2-Tetrachlorethylphosphorthioat (SD208 304), Dimethoate, Disulfoton, EPN, Ethion, Ethoprophos, Etrimfos, Famphur, Fenaminphos, Fenitriothion, Fensulfothion, Fenthion, Fonofos, Formothion, Heptenophos, Isozophos, Isothioate, Isoxathion, Malathion, Methacrifos, Methamidophos, Methidation, Salithion, Mevinphos, Monocrotophos, Naled, Omethoate, Oxydemetonmethyl, Parathion, Parathion-methyl, Phenthoate, Phorate, Phosalone, Phosfolan, Phosmet, Phosphamidon, Phoxim, Pirimiphos-ethyl, Pirimiphos-methyl, Profenofos, Propaphos, Proetamphos, Prothiofos, Pyraclofos, Pyridapenthion, Quinalphos, Sulprofos, Temephos, Terbufos, Tetrachlorvinphos, Thiometon, Triazophos, Trichlorphon, Vamidothion;

2. Aus der Gruppe der Carbamate

Aldicarb, 2-sec.-Butylphenylmethylcarbamat (BPMC), Carbaryl, Carbofuran, Carbosulfan, Cloethocarb, Benfuracarb, Ethiofencarb, Furathiocarb, Isoprocarb, Methomyl, 5-Methyl-m-cumenylbutyryl(methyl)-carbamat, Oxamyl, Primicarb, Propoxur, Thiodicarb, Thiofanox, Ethyl-4,6,9-triaza-4-benzyl-6,10-dimethyl-8-oxa-7 oxo-5,11-dithia-9-dodecenoat (OK 135), 1-Methylthio(ethylideneamino)-N-methyl-N-(morpholinothio)-carbamat (UC 51717);

3. Aus der Gruppe der Carbonsäureester

Allethrin, Alphamethrin, 5-Benzyl-3-furylmethyl-(E)-(1R)-cis-2,2-di-methyl-3-(2-oxothiolan-3-ylidenemethyl)cyclopropanecarboxylat, Bioallethrin, Bioallethrin((S)-cyclopentylisomer), Bioresmethrin, Biphenate, (RS)-1-Cyano-1-(6-phenoxy-2-pyridyl)-methyl-(1RS)-trans-3-(4-tert.butylphenyl)-2,2-dimethylcyclopropanecarboxylat (NCI 85193), Cycloprothrin, Cyfluthrin, Cyhalothrin, Cypermethrin, Cyphenothrin, Deltamethrin, Empenthrin, Esfenvalerate, Fenfluthrin, Fenpropathrin, Fenvalerate, Flucythrinate, Flumethrin, Fluvalinate (D-Isomer), Permethrin, Pheothrin ((R)-Isomer), d-Pralethrin, Pyrethrine (natürliche Produkte), Resmethrin, Tefluthrin, Tetramethrin, Tralomethrin.

4. Aus der Gruppe der Amidine

Amitraz, Chlordimeform

5. Aus der Gruppe der Zinnverbindungen

Cyhexatin, Fenbutatinoxide

6. Sonstige

Abamectin, Bacillus thuringiensis, Bensultap, Binapacyl, Bromopropylate, Buprofezin, Camphechlor, Cartap, Chlorbenzialate, Chlorfluazuron, 2-(4-Chlorphenyl)-4,5-diphenylthiophen (UBI-T 930), Chlofentezine, Cyclopropancarbonsäure(2-naphthylmethyl)-ester (Ro 12-0470), Cyromazin, N-(3,5-Dichlor-4-(1,1,2,3,3,3-hexafluor-1-propyloxy)phenyl)carbamoyl-2-chlorbenzcarboximidsäureethylester, DDT, Dicofol, N-(N-(3,5-Dichlor-4-(1,1,2,2-tetrafluorethoxy)phenylamino)carbonyl)-2,6-difluorbenzamid (XRD 473), Diflubenzuron, N-(2,3-Dihydro-3-methyl-1,3-thiazol-2-ylidene)2,4-xylidine, Dinobuton, Dinocap, Endosulfan, Ethofenprox, (4-Ethoxyphenyl)(dimethyl)(3-(3-phenoxyphenyl)propyl)silan, (4-Ethoxyphenyl)(3-(4-fluor-3-phenoxyphenyl)-propyl)dimethylsilan, Fenoxycarb, 2-Fluor-5-(4-(4-ethoxyphenyl)-4-methyl-1-pentyl)diphenylether (MTI 800), Granulose- und Kernpolyederviren, Fenthiocarb, Flubenzimine, Flucycloxuron, Flufenoxuron, Gamma-HCH, Hexyhiazox, Hydramethylnon (AC 217 300), Ivermectin, 2-Nitromethyl-4,5-dihydro-6H-thiazin (SD 52618), 2-Nitromethyl-3,4-dihydrothiazol (SD35651), 2-Nitromethylen-1,2-thiazinan-3-ylcarbamaldehyde (WL108 477), Propargite, Teflubenzuron, Tetradifon, Tetrasul, Thicyclam, Triflumaron.

Der Wirkstoffgehalt der aus den handelsüblichen Formulierungen bereiteten Anwendungsformen kann in weiten Bereichen variieren, die Wirkstoffkonzentration der Anwendungsformen kann von 0,0001 bis zu 100 Gew.-% Wirkstoff, vorzugsweise zwischen 0,001 und 1 Gew.-% liegen. Die Anwendung geschieht in einer den Anwendungsformen angepaßten üblichen Weisen.

Nachfolgende Beispiele dienen zur Erläuterung der Erfindung.

A. Formulierungsbeispiele

a) Ein Stäubemittel wird erhalten, indem man 10 Gew.-Teile Wirkstoff und 90 Gew.-Teile Talkum als Inertstoff mischt und in einer Schlagmühle zerkleinert.

b) Ein in Wasser leicht dispergierbares, benetzbares Pulver wird erhalten, indem man 25 Gew.-Teile Wirkstoff, 65 Gew.-Teile kaolinhaltigen Quarz als Inertstoff, 10 Gew.-Teile ligninsulfonsaures Kalium und 1 Gew.-Teil oleoylmethyltaurinsaures Natrium als Netz- und Dispergiermittel mischt und in einer Stiftmühle mahlt.

c) Ein in Wasser leicht dispergierbares Dispersionskonzentrat stellt man her, indem man 40 Gew.-Teile Wirkstoff mit 7 Gew.-Teilen eines Sulfobernsteinsäurehalbesters, 2 Gew.-Teilen eines Ligninsulfonsäure-Natriumsalzes und 51 Gew.-Teilen Wasser mischt und in einer Reibkugelmühle auf eine Feinheit von unter 5 Mikron vermahlt.

d) Ein emulgierbares Konzentrat läßt sich herstellen aus 15 Gew.-Teilen Wirkstoff, 75 Gew.-Teilen Cyclohexanon als Lösungsmittel und 10 Gew.-Teilen oxethyliertem Nonylphenol (10 EO) als Emulgator.

e) Ein Granulat läßt sich herstellen aus 2 bis 15 Gew.-Teilen Wirkstoff und einem inerten Granulatträgermaterial wie Attapulgit, Bimsgranulat und/oder Quarzsand. Zweckmäßigerweise verwendet man eine Suspension des Spritzpulvers aus Beispiel b) mit einem Feststoffanteil von 30 % und spritzt diese auf die Oberfläche eines Attapulgitgranulats, trocknet und vermischt innig. Dabei beträgt der Gewichtsanteil des Spritzpulvers ca. 5% und der des inerten Trägermaterials ca. 95 % des fertigen Granulats.

B. Chemische Beispiele

Beispiel 1 (Tabellenbeispiel 9)

N-(2,4-Difluorphenyl)-4-methyl-2-methylmercapto-5-pyrimidincarbonsäureamid

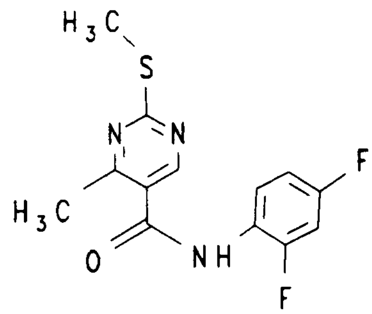

Eine Mischung aus 107 g Acetessigsäure-2,4-difluoranilid und 110 g Orthoameisensäuretriethylester in 140 ml Acetanhydrid und 300 ml Xylol wurde so erhitzt, daß zunächst das sich bildende Ethanol abdestillierte, abschließend wurde das Reaktionsgemisch noch eine Stunde bei 140° C Innentemperatur gehalten. Das Xylol wurde dann bei vermindertem Druck abdestilliert, das Gemisch dann auf Eis-Wasser gegeben und es konnten durch Absaugen 66 g Ethoxymethylenacetessigsäure-2,4-difluoranilid, Smp.:109-113° C, erhalten werden.

Zu 27 g Ethoxymethylenacetessigsäure-2,4-difluoranilid und 16,7 g S-Methylisothioharnstoffsulfat in 200 ml Ethanol wurden 8,2 g Natriumethanolat gegeben und 8 Stunden zum Rückfluß erhitzt. Das Ethanol wurde teilweise bei vermindertem Druck abdestilliert, das Gemisch wurde mit Eis-Wasser hydrolysiert. Das ausgefallene Produkt konnte abgesaugt werden, nach Kristallisation aus Essigester wurden 22,1 g N-(2,4-Difluorphenyl)-4-methyl-2-methylmercapto-5-pyrimidincarbonsäureamid, Smp.:158-160° C, erhalten.

Beispiel 2 (Tabellenbeispiel 10)

N-(2,4-Difluorphenyl)-4-methyl-2-methylsulfonyl-5-pyrimidincarbonsäureamid

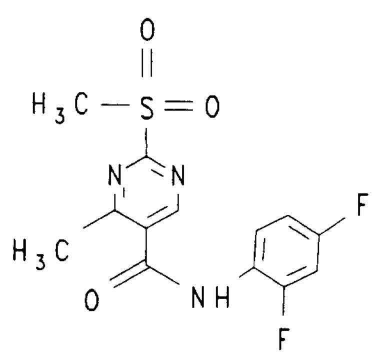

Zu 62 g ®Oxone (Kaliumperoxomonosulfat, $2KHSO_5 x KHSO_4 x K_2SO_4$) in einer Mischung aus 100 ml Methanol, 100 ml Essigsäure und 100 ml Wasser wurden 19 g N-(2,4-Difluorphenyl)-4-methyl-2-methylmercapto-5-pyrimidincarbonsäureamid gegeben. Nach dem Abklingen der exothermen Reaktion wurde mit Wasser verdünnt, dann mit Methylenchlorid extrahiert, die organische Phase gewaschen, mit $Na_2SO_4$ getrocknet und eingeengt. Das fest erhaltene Rohprodukt wurde in Essigester/Heptan (1:9) suspendiert, nach Absaugen wurden 15,3 g N-(2,4-Difluorphenyl)-4-methyl-2-methylsulfonyl-5-pyrimidincarbonsäureamid, Smp.:154-156° C, erhalten.

Beispiel 3 (Tabellenbeispiel 16)

N-(2,4-Difluorphenyl)-4-methyl-2-(3-propinyloxy)-5-pyrimidincarbonsaureamid

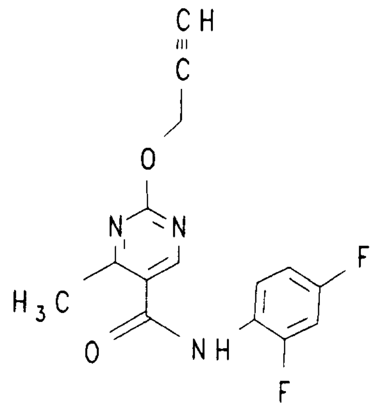

Bei 0° C wurden zu 1,1 g 80 % Natriumhydrid in Paraffinöl in 100 ml Tetrahydrofuran als Lösungsmittel 2,1 ml Propargylalkohol gegeben, nach 30 Minuten erfolgte der Zusatz von N-(2,4-Difluorphenyl)-4-methyl-2-methylsulfonyl-5-pyrimidincarbonsäureamid, das Reaktionsgemisch wurde dann 5 Stunden bei 25°C gerührt, anschließend wurde das Tetrahydrofuran bei etwas vermindertem Druck teilweise abdestilliert, das Reaktionsgemisch wurde auf Wasser gegeben, das so erhalten Rohprodukt abgesaugt nach Umkristallisation aus Essigester/Heptan wurden 4,6 g N-(2,4-Difluorphenyl)-4-methyl-2-(3-propinyloxy)-5-pyrimidincarbonsäureamid, Smp.: 135-138° C, erhalten.

Beispiel 4 (Tabellenbeispiel 731)

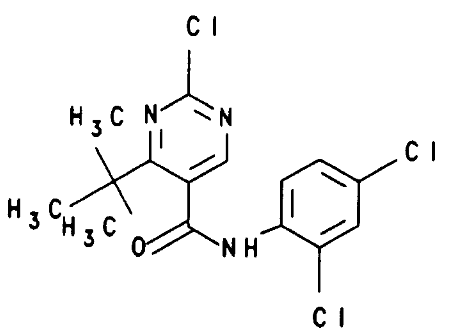

2-Chlor-N-(2,4-dichlorphenyl)-4-(1.1-dimethylethyl)-5-pyrimidincarbonsäureamid

Eine Mischung aus 158 g 4,4-Dimethyl-3-oxovaleriansäuremethylester und 125 g N,N-Dimethylformamid-dimethoxyacetal wurde 5 Stunden so zum Rückfluß erhitzt, daß das sich bildende Methanol abdestilliert wurde. Das erkaltete Reaktionsgemisch wurde in Heptan suspendiert und durch Absaugen konnten 121,3 g 4,4-Dimethyl-2 (N,N-dimethylaminomethylen)-3-oxovaleriansäuremethylester, Smp.: 68° C, erhalten werden.

21,4 g 4,4-Dimethyl-2-(N,N-dimethylaminomethylen)-3-oxovaleriansäuremethylester und 6,1 g Harnstoff wurden in 100 ml Essigsäure 16 Stunden zum Rückfluß erhitzt. Das erkaltete Reaktionsgemisch wurde mit 300 ml Eis-Wasser hydrolisiert, die wäßrige Phase wurde dann mit Essigester extrahiert, die organische Phase wurde mit Wasser gewaschen und einrotiert. Das Rohgemisch wurde in Heptan/Essigester 9:1 aufgenommen, durch Absaugen konnten 7,5 g 2-Hydroxy-4-(1,1-dimethylethyl)-5-pyrimidincarbonsäuremethylester, Smp. 175° C, erhalten werden.

32,9 g 2-Hydroxy-4-(1,1-dimethylethyl)-5-pyrimidincarbonsäuremethylester und 43,7 g Kaliumhydroxid wurden in 200 ml Methanol 5 Stunden zum Rückfluß erhitzt, das Methanol wurde abdestilliert, das Reaktionsgemisch in Eis-Wasser aufgenommen. Nach dem Ansäuern mit konz. Salzsäure konnten durch

Absaugen 25,4 g 2-Hydroxy-4-(1,1-dimethylethyl)-5-pyrimidincarbonsäure, Smp.: 188° C (Z), isoliert werden.

25,4 g 2-Hydroxy-4-(1,1-dimethylethyl)-5-pyrimidincarbonsäure wurden in einer Mischung aus 50 ml Thionylchlorid, 50 ml Phosphoroxychlorid und 1 ml N,N-Dimethylanilin so lange zum Rückfluß erhitzt, bis alles gelöst worden war (ca. 45 Minuten). Das überschüssige Chlorierungsagenz wurde unter vermindertem Druck weitgehend abdestilliert, nach Destillation im Hochvakuum wurden 22,6 g 2-Chlor-4-(1,1 dimethylethyl)-5-pyrimidincarbonsäurechlord, Sdp.: 70-85° C/0.01 Torr, erhalten.

Bei 5-15° C wurden zu 4,7 g 2-Chlor-4-(1,1-dimethylethyl)-5-pyrimidincarbonsäurechlorid in 100 ml Dioxan 3,3 g 2.4-Dichloranilin gegeben, anschließend tropfte man 3,3 g (4.2 ml) Triethylamin zu dem Reaktionsgemisch und rührte einige Stunden. Das Reaktionsgemisch wurde in Essigester aufgenommen und mit Wasser hydrolisiert. Nach Abtrennen der organischen Phase wurde diese mit Wasser gewaschen, getrocknet und einrotiert. Nach Reinigung mittels Säulenchromatographie mit Heptan/Essigester 7:3 wurden 4,5 g 2-Chlor-N-(2,4-dichlorphenyl)-4-(1,1-dimethylethyl)-5-pyrimidincarbonsäureamid, Smp.: 130-131° C, erhalten.

Beispiel 5 und 6 (Tabellenbeispiel 688 und 689)

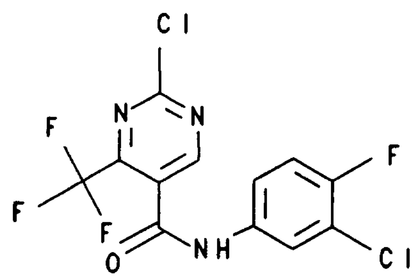

Tabellenbsp.: 688

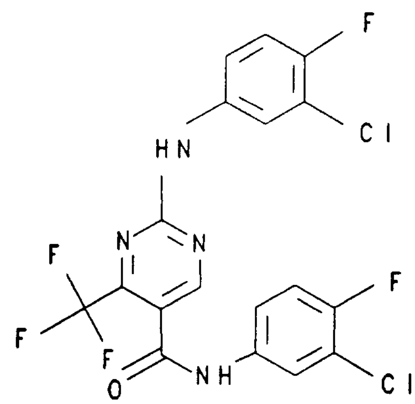

Tabellenbsp.: 689

Verfahren A:

Bei 20° C wurden zu einer Natriumethanolatlösung aus 300 ml Ethanol und 9.7 g Natrium 96.4 g 2-Ethoxymethylen-3-oxo-4.4.4-trifluor-3-buttersäureethylester und 61.2 g S-Methylisoharnstoffsulfat gegeben. Es wurde einige Stunden gerührt, dann das Ethanol weitgehend abrotiert und das Gemisch in Methylenchlorid aufgenommen. Die organische Phase wurde abgetrennt gewaschen, getrocknet. Nach einer Destillation unter vermindertem Druck konnten 84.4 g 2-Methylmercapto-4-trifluormethyl-5-pyrimidincarbonsäureethylester, Sdp.: 162° C bei 32 Torr, erhalten werden.

26.7 g 2-Methylmercapto-4-trifluormethyl-5-pyrimidincarbonsäureethylester und 28.5 g Kaliumhydroxyd wurden in 150 ml Ethanol 5 Stunden zum Rückfluß erhitzt, das Ethanol wurde abdestilliert, das Reaktionsgemisch in Eis-Wasser aufgenommen. Nach dem Ansäuern mit konz. Salzsäure konnten durch Absaugen 22 g eines Gemisches aus 2-Hydroxy-4-trifluormethyl-5-pyrimidincarbonsäure und 2.4-Dihydroxy-4-trifluormethyl-1H-5-pyrimidincarbonsäure, Smp.: 225-240° C (Zers.), isoliert werden.

Verfahren B:

72.0 g 2-Ethoxymethylen-3-oxo-4.4.4-trifluor-3-buttersäureethylester und 18.0 g Harnstoff wurden in 200 ml Essigsäure 6 Stunden zum Rückfluß erhitzt. Das erkaltete Reaktionsgemisch wurde mit 500 ml Eis-Wasser hydrolisiert. Durch Absaugen konnten 56.8 g eines Gemisches aus 2-Hydroxy-4-trifluormethyl-5-pyrimidincarbonsäureethylester und 2.4-Dihydroxy-4-trifluormethyl-1H-5-pyrimidincarbonsäureethylester, Smp.: 175-185° C (Zers.), isoliert werden.

21,0 g des Gemisches aus 2-Hydroxy-4-trifluormethyl-5-pyrimidincarbonsäureethylester und 2,4-Dihydroxy-4-trifluormethyl-1H-5-pyrimidincarbonsäureethylester und 25,3 g Kaliumhydroxid wurden in 150 ml Ethanol 5 Stunden zum Rückfluß erhitzt, das Ethanol wurde abdestilliert, das Reaktionsgemisch in Eis-Wasser aufgenommen. Nach dem Ansäuern mit konz. Salzsäure konnten durch Absaugen 16.5 g eines Gemisches aus 2-Hydroxy-4-trifluormethyl-5-pyrimidincarbonsäure und 2,4-Dihydroxy-4-trifluormethyl-1H-5-pyrimidincarbonsäure, Smp.: 225-240° C (Zers.), isoliert werden.

25,0 g des Gemisches aus einem Gemisch aus 2-Hydroxy-4-trifluormethyl-5-pyrimidincarbonsäure und 2,4-Dihydroxy-4-trifluormethyl-1H-5-pyrimidincarbonsäure wurden in einer Mischung aus 50 ml Thionylchlorid, 50 ml Phosphoroxychlorid und 1 ml N,N-Dimethylanilin so lange zum Rückfluß erhitzt bis alles gelöst worden war (ca. 45 Minuten). Das überschüssige Chlorierungsagenz wurde unter vermindertem Druck weitgehend abdestilliert, nach Destillation im Hochvakuum wurden 20.6 g 2-Chlor-4-trifluormethyl-5-pyrimidincarbonsäurechlorid, Sdp.: 90-92° C/20 Torr, erhalten.

Bei 5-15° C wurden zu 2,0 g 2-Chlor-4-trifluormethyl-5-pyrimidincarbonsäurechlorid in 25 ml Dioxan 1,4 g 3-Chlor-4-fluoranilin gegeben, anschließend tropfte man 1,1 g (1,5 ml) Triethylamin zu dem Reaktionsgemisch und rührte einige Stunden. Das Reaktionsgemisch wurde in Essigester aufgenommen und mit Wasser hydrolisiert. Nach Abtrennen der organischen Phase wurde diese mit Wasser gewaschen, getrocknet und einrotiert. Nach Reinigung mittels Säulenchromatographie mit Heptan/Essigester 7:3 wurden 1,3 g 2-Chlor-N-(3-chlor-4-fluorphenyl)-4-trifluormethyl-5-pyrimidincarbonsäureamid, Smp.: 153-154° C, als erste und 0,7 g 2-(3-Chlor-4-fluoranilino)-N-(3-chlor-4-fiuorphenyl)-4-trifluormethyl-5-pyrimidincarbonsäureamid, Smd.: 161-162° C als 2. Fraktion erhalten.

Beispiel 7 (Tabellenbeispiel 466)

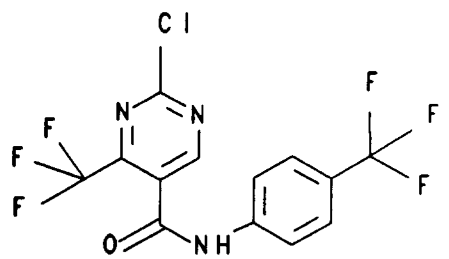

2-Chlor-N-(4-trifluorphenyl)-4-trifluormethyl-5-pyrimidincarbonsäureamid

Bei 0-5° C wurden zu einem Gemisch aus 43,3 g Ethylvinylether und 47,5 g Pyridin in 300 ml Methylenchlorid 127,3 g Trichloracetylchlorid hinzugetropft. Nach einigen Stunden wurde mit kalter 2 n HCl gewaschen, die organische Phase getrocknet und nach Destillation wurden 101.9 g 1-Ethoxy-4,4,4-trichlor-3-oxo-1-buten, Sdp.: 79-80° C/0,1 Torr, erhalten.

Bei 0-5° C wurden zu einem Gemisch aus 43.5 g 1-Ethoxy-4.4.4-trichchlor-3-oxo-1-buten, 19.8 g Pyridin in 100 ml Methylenchlorid 52.5 g Trifluoracetanhydrid hinzu getropft. Nach einigen Stunden wurde mit kalter 2 n HCl gewaschen, die organische Phase getrocknet und nach Destillation wurden 20.2 g 1-Ethoxy-4.4.4-trichlor-3-oxo-2-trifluoracetyl-1-buten, Sdp.: 145-153° C/18 Torr, erhalten.

14.0 g 1-Ethoxy-4.4.4-trichlor-3-oxo-2-trifluoracetyl-1-buten und 3.0 g Harnstoff wurden in 30 ml Essigsäure zum Rückfluß erhitzt. Das erkaltete Reaktionsgemisch wurde mit 100 ml Eis-Wasser hydrolysiert. Durch Absaugen konnten 10.0 g eines Gemisches aus 2-Hydroxy-4-trifluormethyl-5-pyrimidinyl-2.2.2-trichlorethanon und 2.4-Dihydroxy-4-trifluormethyl-1H-5-pyrimidinyl-2.2.2-trichlorethanon, Smp.: 175-185° C (Z), isoliert werden.

10.0 g des Gemisches aus 2-Hydroxy-4-triflrnormethyl-5-pyrimidinyl-2.2.2-trichlorethanon und 2.4-Dihydroxy-4-trifiuormethyl-1H-5-pyrimidinyl-2.2.2-trichlorethanon wurden in einer Mischung aus 25 ml Thionylchlorid, 25 ml Phosphoroxychlorid und 1 ml N,N-Dimethylanilin solange zum Rückfluß erhitzt bis alles gelöst worden war (ca. 30 Minuten). Das überschüssige Chlorierungsagenz wurde unter vermindertem Druck weitgehend abdestilliert, nach Destillation im Hochvakuum wurden 6.7 g 2-Chlor-4-flifluormethyl-5-pyrimidinyl-2.2.2-trichlorethanon, Sdp.: 70-75° C/0.1 Torr, erhalten.

Zu 6,7 g 2-Chlor-4-trifluormethyl-5-pyrimidinyl-2,2,2-trichlorethanon in einer Mischung aus 10 ml DMSO und 30 ml Dioxan wurden 3.2 g 4-Amino-benzotrifluorid gegeben. Nach Abklingen der exothermen Reaktion wurde einige Stunden nachgerührt und das Reaktionsgemisch anschließend auf Wasser gegeben. Die

wäßrige Phase wurde mit Essigester extrahiert, die organische Phase abgetrennt, gewaschen, getrocknet und einrotiert. Nach Reinigung durch Säulenchromatographie mit Heptan/Essigester 8:2 wurden 4,8 g 2-Chlor-N-(4-trifluorphenyl)-4-trifluormethyl-5-pyrimidincarbonsäureamid, Smp.: 120-122° C, erhalten.

Beispiel 8 (Tabellenbeispiel 729)

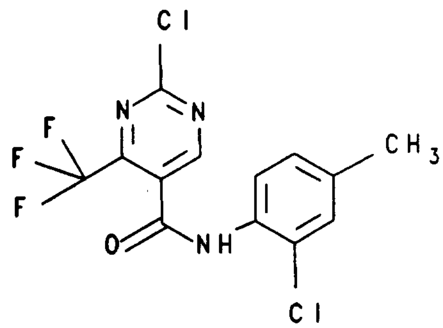

2-Chlor-N-(2-chlor-4-methylphenyl)-4-trifluormethyl-5-pyrimidincarbonsäureamid

23,5 g 1-(2-Methyl-propanyloxy)-4,4,4-trifluor-3-oxo-2-trifluoracetyl-1-buten und 4,8 g Harnstoff wurden in 30 ml Essigsäure 4 Stunden zum Rückfluß erhitzt. Das erkaltete Reaktionsgemisch wurde mit 100 ml Eis-Wasser hydrolisiert. Durch Absaugen konnten 13,3 g eines Gemisches aus 2-Hydroxy-4-trifluormethyl-5-pyrimidinyl-2,2,2-trifluormetanon und 2,4-Dihydroxy-4-trifluormethyl-1H-5-pyrimidinyl-2,2,2-trifluorethanon, Smp.: 203-206° C, isoliert werden.

10,0 g des Gemisches aus 2-Hydroxy-4-trifluormethyl-5-pyrimidinyl-2,2,2-trifluorethanon und 2,4-Dihydroxy-4-trifluormethyl-1H-5-pyrimidinyl-2,2,2-trifluorethanon wurden in 25 ml Phosphoroxychlorid und 1 ml N,N-Dimethylanilin so lange zum Rückfluß erhitzt bis alles gelöst worden war (ca. 30 Minuten). Das überschüssige Chlorierungsagenz wurde unter vermindertem Druck weitgehend abdestilliert. Das Rohgemisch wurde in Methylenchlorid aufgenommen und mit Wasser hydrolisiert, die organische Phase abgetrennt, getrocknet und nach dem Einrotieren wurden 4,2 g 2-Chlor-4-trifluormethyl-5-pyrimidinyl-2,2,2-trifluorethanon erhalten.

Zu 4,2 g 2-Chlor-4-trifluormethyl-5-pyrimidinyl-2,2,2-trifluorethanon in einer Mischung aus 10 ml DMSO und 30 ml Dioxan wurden 1,8 g 2-Chlor-4-methylanilin gegeben. Nach Abklingen der exothermen Reaktion wurde einige Stunden nachgerührt und das Reaktionsgemisch anschließend auf Wasser gegeben. Die wäßrige Phase wurde mit Essigwasser extrahiert, die organische Phase abgetrennt, gewaschen, getrocknet und einrotiert. Nach Reinigung durch Säulenchromatographie mit Heptan/Essigester 7:3 wurden 1,2 g 2-Chlor-N-(2-chlor-4-methylenphenyl)-4-trifluormethyl-5-pyrimidincarbonsäureamid, Smp.: 104-105° C, erhalten.

Beispiel 9 (Tabellenbeispiel 710)

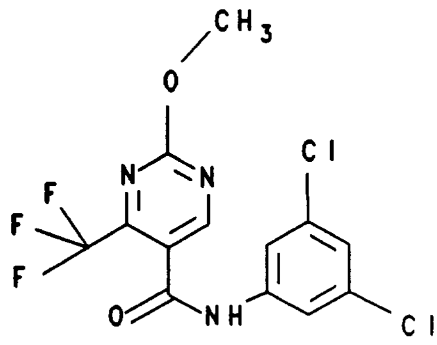

2-Methoxy-N-(3,5-dichlorphenyl)-4-trifluormethyl-5-pyrimidincarbonsäureamid

Zu 25 ml DMF wurden 0.6 g 80 % Natriumhydrid und anschließend 1,6 g (2 ml) Methanol sowie 3,7 g 2-Chlor-N-(3,5-dichlorphenyl)-4-trifluormethyl-5-pyrimidincarbonsäureamid gegeben. Das Reaktionsgemisch wurde dann einige Stunden gerührt, dann ca. 2 Stunden auf 120-130° C erhitzt. Nach der Hydrolyse mit Wasser wurde mit Essigester extrahiert, die abgetrennte organische Phase gewaschen und getrocknet. Das nach dem Einrotieren erhaltene Rohgemisch wurde säulenchromatographisch mit Heptan 7:3 gereinigt, es wurden 1,4 g 2-Methoxy-N-(3,5-dichlorphenyl)-4-trifluormethyl-5-pyrimidincarbonsäureamid, Smp.: 176-177° C, erhalten.

Beispiel 10 (Tabellenbeispiel 724)

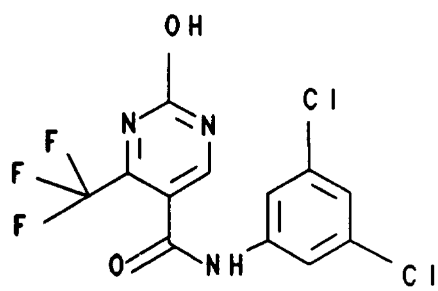

2-Hydroxy-N-(3,5-dichlorphenyl)-4-trifluormethyl-5-pyrimidincarbonsäureamid

Zu 1,2 g Kaliumhydroxid in einer Mischung aus 25 ml Dioxan und 25 ml Wasser wurden 5,2 g 2-Chlor-N-(3,5-dichlorphenyl)-4-trifluormethyl-5-pyrimidincarbonsäureamid gegeben. Das Reaktionsgemisch wurde einige Stunden gerührt, dann wurden ca. 100 ml Wasser zugegeben. Nach einer Extraktion mit Essigester, waschen, trocknen und einengen der organischen Phase wurden 4,7 g 2-Hydroxy-N-(3,5-dichlorphenyl)-4-trifluormethyl-5-pyrimidincarbonsäureamid, Smp.: 218-220° C, erhalten.

Die Ausbeuten der angegeben Beispiele sind nicht optimiert. Entsprechend den genannten Beispielen können die in der folgenden Tabelle genannten Vertreter hergestellt werden.

Tabelle

| Nr. | $R^1$ | $R^2$ | $R^3$ | $R^4$ | $R^5$ - $R^9$ | Smp.(°C): |
|---|---|---|---|---|---|---|
| 1. | $CH_3$ | H | H | H | H | |
| 2. | $CH_3$ | H | H | H | 4-Cl | |
| 3. | $CH_3$ | H | H | H | 4-F | |
| 4. | $CH_3$ | H | H | H | 4-$CH_3$ | |
| 5. | $CH_3$ | S-$CH_3$ | H | H | 3,5-di-Cl | 172-176 |
| 6. | $CH_3$ | $OCH_3$ | H | H | 3,5-di-Cl | |
| 7. | $CH_3$ | H | H | H | 2,4-di-F | |
| 8. | $CH_3$ | Cl | H | H | 2,4-di-F | 94-95 |
| 9. | $CH_3$ | S-$CH_3$ | H | H | 2,4-di-F | 158-160 |
| 10. | $CH_3$ | $SO_2CH_3$ | H | H | 2,4-di-F | 154-156 |
| 11. | $CH_3$ | H | H | H | 2,6-di-$C_2H_5$ | |
| 12. | $CH_3$ | $SO_2CH_3$ | H | H | 3,5-di-Cl | 185-188 |
| 13. | $CH_3$ | H | H | H | 4-$CF_3$ | |
| 14. | $CH_3$ | H | H | H | 3-$CF_3$ | |
| 15. | $CH_3$ | H | H | H | 2-$CF_3$ | |
| 16. | $CH_3$ | $OCH_2C{\equiv}CH$ | H | H | 2,4-di-F | 135-138 |

| Nr. | $R^1$ | $R^2$ | $R^3$ | $R^4$ | $R^5$ - $R^9$ | Smp.(°C): |
|---|---|---|---|---|---|---|
| 17. | $CH_3$ | H | H | H | 4-O-Ph | |
| 18. | $CH_3$ | H | H | H | 4-O-(2,4-di-Cl-Ph) | |
| 19. | $CH_3$ | H | H | H | 4-O-$CH_2$Ph | |
| 20. | $CH_3$ | H | H | H | 4-O-4-F-Ph | |
| 21. | $CH_3$ | H | H | H | 4-$COCH_3$ | |
| 22. | $CH_3$ | H | H | H | 4-CN | |
| 23. | $CH_3$ | OH | H | H | 3,5-di-Cl | |
| 24. | $CH_3$ | H | H | H | 3,5-di-Cl | 187-189 |
| 25. | $CH_3$ | H | H | H | 2-$COOC_2H_5$ | |
| 26. | $CH_3$ | H | H | H | 3,5-di-$CF_3$ | |
| 27. | $CH_3$ | H | H | H | 3,5-di-$CH_3$ | |
| 28. | $CH_3$ | $T^1$ | H | H | 3,5-di-Cl | |
| 29. | $CH_3$ | $N(CH_3)_2$ | H | H | 3,5-di-Cl | 196-198 |
| 30. | $CH_3$ | $CH_2Cl$ | H | H | 3,5-di-Cl | |
| 31. | $CH_3$ | H | H | H | (1,2)-$CH(CH_3)$-$C(CH_3)_2$- | |
| 32. | $CH_3$ | H | H | H | 4-$N(CH_3)_2$ | |
| 33. | $CHCl_2$ | H | H | H | 3-$NO_2$ | |
| 34. | $CHCl_2$ | Cl | H | H | H | |
| 35. | $CH_3$ | Cl | H | H | 4-Cl | |
| 36. | $CH_2Cl$ | Cl | H | H | 4-F | |
| 37. | $CH_2Br$ | Cl | H | H | 4-$CH_3$ | |
| 38. | $CH_3$ | Cl | H | H | 2,4-di-Cl | |
| 39. | $CH_3$ | Cl | H | H | 3,5-di-Cl | 129-132 |
| 40. | $CH_3$ | Cl | H | H | 3,5-di-F | |
| 41. | $CH_3$ | Cl | H | H | 2,6-di-F | |
| 42. | $CH_3$ | Cl | H | H | 4-O-$CH_3$ | |
| 43. | $CH_2OCH_3$ | Cl | H | H | 3,5-di-$OCH_3$ | |
| 44. | $CH_3$ | Cl | H | H | 4-O-$C_2H_5$ | |
| 45. | $CH_3$ | Cl | H | H | 2-$CH_3$ | |
| 46. | $CH_3$ | Cl | H | H | 2,6-di-$C_2H_5$ | |

| Nr. | $R^1$ | $R^2$ | $R^3$ | $R^4$ | $R^5$ - $R^9$ | Smp.(°C): |
|---|---|---|---|---|---|---|
| 47. | $CH_3$ | Cl | H | H | 3,5-di-$CH_3$ | |
| 48. | $CH_3$ | Cl | H | H | 4-$CF_3$ | |
| 49. | $CH_3$ | Cl | H | H | 3-$CF_3$ | |
| 50. | $CBr_3$ | Cl | H | H | 2-$CF_3$ | |
| 51. | $CH_3$ | Cl | H | H | 4-$OCF_3$ | |
| 52. | $CH_3$ | Cl | H | H | 4-t.-$C_4H_9$ | |
| 53. | $CH_3$ | Cl | H | H | 4-O-(2,4-di-Cl-Ph) | |
| 54. | $CH_3$ | Cl | H | H | 4-O-$CH_2$Ph | |
| 55. | $CH_3$ | Cl | H | H | 4-O-4-F-Ph | |
| 56. | $CH_3$ | Cl | H | H | 4-$COCH_3$ | |
| 57. | $CH_3$ | Cl | H | H | 4-$COOCH_3$ | |
| 58. | $CH_3$ | Cl | H | H | 4-$COOC_2H_5$ | |
| 59. | $CH_3$ | Cl | H | H | 4-$CONH_2$ | |
| 50. | $CH_3$ | Cl | H | H | 2-$COOC_2H_5$ | |
| 61. | $CH_3$ | Cl | H | H | 3,5-di-$CF_3$ | |
| 62. | $CH_3$ | Cl | H | H | 2,5-di-$CH_3$ | |
| 63. | $CH_3$ | Cl | H | H | 2,4-di-$CH_3$ | |
| 64. | $CH_3$ | Cl | H | H | 3-O-i-$C_3H_7$ | |
| 65. | $CH_3$ | Cl | H | H | (3,4)-O-$CH_2$O- | |
| 66. | $CH_3$ | Cl | H | H | (1,2)-$CH(CH_3)$-$C(CH_3)_2$- | |
| 67. | $CH_3$ | Cl | H | H | 4-$N(CH_3)_2$ | |
| 68. | $CH_3$ | Cl | H | H | 3-$NO_2$ | |
| 69. | $CBr_3$ | $NH_2$ | H | H | H | |
| 70. | $CH_3$ | $NH_2$ | H | H | 4-Cl | |
| 71. | $CH_3$ | $NH_2$ | H | H | 4-F | |
| 72. | $CH_2Cl$ | $NH_2$ | H | H | 4-$CH_3$ | |
| 73. | $CHCl_2$ | $NH_2$ | H | H | 2,4-di-Cl | |
| 74. | $CH_3$ | $NH_2$ | H | H | 3,5-di-Cl | 295-300 |
| 75. | $CH_2Cl$ | $NH_2$ | H | H | 2-$CH_3$-4-F | |
| 76. | $CH_3$ | $NH_2$ | H | H | 2,6-di-F | |

| Nr. | $R^1$ | $R^2$ | $R^3$ | $R^4$ | $R^5$ - $R^9$ | Smp.(°C): |
|---|---|---|---|---|---|---|
| 77. | $CH_2Br$ | $NH_2$ | H | H | 4-O-$CH_3$ | |
| 78. | $CH_3$ | $NH_2$ | H | H | 3,5-di-$OCH_3$ | |
| 79. | $CH_3$ | $NH_2$ | H | H | 4-O-$C_2H_5$ | |
| 80. | $CH_3$ | $NH_2$ | H | H | 2-$CH_3$ | |
| 81. | $CH_3$ | $NH_2$ | H | H | 2,6-di-$C_2H_5$ | |
| 82. | $CH_3$ | $NH_2$ | H | H | 3,5-di-$CH_3$ | |
| 83. | $CH_3$ | $NH_2$ | H | H | 4-$CF_3$ | |
| 84. | $CF_2Cl$ | $NH_2$ | H | H | 3-$CF_3$ | |
| 85. | $CH_3$ | $NH_2$ | H | H | 2-$CF_3$ | |
| 86. | $CH_3$ | $NH_2$ | H | H | 4-$OCF_3$ | 295-297 |
| 87. | $CH_3$ | $NH_2$ | H | H | 4-O-Ph | |
| 88. | $CH_3$ | $NH_2$ | H | H | 4-O-(2,4-di-Cl-Ph) | |
| 89. | $CH_3$ | $NH_2$ | H | H | 4-O-$CH_2$Ph | |
| 90. | $CH_3$ | $NH_2$ | H | H | 4-O-4-F-Ph | |
| 91. | $CH_3$ | $NH_2$ | H | H | 4-$COCH_3$ | |
| 92. | $CH_3$ | $NH_2$ | H | H | 4-$COOCH_3$ | |
| 93. | $CH_3$ | $NH_2$ | H | H | 4-$COOC_2H_5$ | |
| 94. | $CH_3$ | $NH_2$ | H | H | 4-COO-i-$C_3H_7$ | |
| 95. | $CH_3$ | $NH_2$ | H | H | 4-$CONH_2$ | |
| 96. | $CH_3$ | $NH_2$ | H | H | 2-$COOC_2H_5$ | |
| 97. | $CH_3$ | $NH_2$ | H | H | 3,5-di-$CF_3$ | |
| 98. | $CH_3$ | $NH_2$ | H | H | 2,3,5-tri-$CH_3$ | |
| 99. | $CH_3$ | $NH_2$ | H | H | 2,4-di-$CH_3$ | |
| 100. | $CH_3$ | $NH_2$ | H | H | 3-O-i-$C_3H_7$ | |
| 101. | $CH_3$ | $NH_2$ | H | H | (3,4)-O-$CH_2$O- | |
| 102. | $CH_3$ | $NH_2$ | H | H | (1,2)-$CH(CH_3)$-$C(CH_3)_2$- | |
| 103. | $CH_3$ | $NH_2$ | H | H | 4-$N(CH_3)_2$ | |
| 104. | $CH_3$ | $NH_2$ | H | H | 3-$NO_2$ | |
| 105. | $CH_3$ | $NH_2$ | H | H | 2-$SCH_3$ | |
| 106. | $CH_3$ | H | H | $CH_3$ | H | |

| Nr. | $R^1$ | $R^2$ | $R^3$ | $R^4$ | $R^5$ - $R^9$ | Smp.(°C): |
|---|---|---|---|---|---|---|
| 107. | $CH_3$ | H | $CH_3$ | H | 4-Cl | |
| 108. | $CH_3$ | H | $C_2H_5$ | H | 4-F | |
| 109. | $CH_3$ | H | $C_6H_5$ | H | 4-$CH_3$ | |
| 110. | $CH_3$ | H | H | CHO | 2,4-di-Cl | |
| 111. | $CH_3$ | H | H | H | 3,4-di-Cl | |
| 112. | $CH_3$ | $CH_3$ | H | H | 2,4-di-F | |
| 113. | $CH_3$ | $SCH_3$ | H | H | 4-O-$CH_3$ | |
| 114. | $CH_3$ | $SO_2CH_3$ | H | H | 4-O-$C_2H_5$ | |
| 115. | $CH_3$ | H | H | H | 2-$CH_3$ | |
| 116. | $CH_3$ | $OCH_3$ | H | H | 2,6-di-$C_2H_5$ | |
| 117. | $CH_3$ | H | n-$C_4H_9$ | H | 3,5-di-$CH_3$ | |
| 118. | $CH_3$ | H | H | $CH_2OCH_3$ | 4-$CF_3$ | |
| 119. | $CH_3$ | $CH_2Cl$ | H | H | 3-$CF_3$ | |
| 120. | $CH_3$ | $CH_2OCH_3$ | H | H | 2-$CF_3$ | |
| 121. | $CH_3$ | H | $CF_3$ | H | 4-$OCF_3$ | |
| 122. | $CH_3$ | $CH_2NHCH_3$ | H | H | 4-O-Ph | |
| 123. | $CH_3$ | H | H | $CH_3$ | 4-O-(2,4-di-Cl-Ph) | |
| 124. | $CH_3$ | H | $C_2H_5$ | H | 4-O-$CH_2$Ph | |
| 125. | $CH_3$ | $OCH_2CH=CH_2$, | H | H | 4-O-4-F-Ph | |
| 126. | $CH_3$ | $OCH_2C\equiv CH$, | H | H | 4-$COCH_3$ | |
| 127. | $CH_3$ | H | H | $CH_2C=CH$ | 4-$COOCH_3$ | |
| 128. | $CH_3$ | $OCH_2C\equiv CH$, | H | H | 4-$COOC_2H_5$ | |
| 129. | $CH_3$ | H | H | $CH_3$ | 4-$CONH_2$ | |
| 130. | $CH_3$ | O-n-$C_3H_7$ | H | H | 2-$COOC_2H_5$ | |
| 131. | $CH_2Cl$ | H | $CH_3$ | H | 3,5-di-$CF_3$ | |
| 132. | $CH_3$ | H | 2-Thienyl | H | 3,5-di-$CH_3$ | |
| 133. | $CH_3$ | H | $SCH_3$ | H | 2,4-di-$CH_3$ | |
| 134. | $CH_3$ | H | $OCH_3$ | H | 3-O-i-$C_3H_7$ | |
| 135. | $CH_3$ | H | $N(CH_3)_2$ | H | (3,4)-O-$CH_2$O- | |
| 136. | $CH_3$ | $SCH_3$ | H | H | (1,2)-$CH(CH_3)$-$C(CH_3)_2$- | |

| Nr. | $R^1$ | $R^2$ | $R^3$ | $R^4$ | $R^5$ - $R^9$ | Smp.(°C): |
|---|---|---|---|---|---|---|
| 137. | $CH_3$ | $CF_3$ | H | H | 4-$N(CH_3)_2$ | |
| 138. | $CH_3$ | $CCl_3$ | H | H | 3-$NO_2$ | |
| 139. | $CH_3$ | Cl | 2-Thienyl | H | H | |
| 140. | $CH_3$ | Cl | 2-Furyl | H | 4-Cl | |
| 141. | $CH_3$ | Cl | H | $C_6H_5$ | 4-F | |
| 142. | $CH_3$ | Cl | 4-F-$C_6H_4$ | H | 4-$CH_3$ | |
| 143. | $CH_3$ | Cl | H | $CH_3$ | 2,4-di-Cl | |
| 144. | $CH_3$ | Cl | 2-Thienyl | H | 3,5-di-Cl | |
| 145. | $CH_3$ | Cl | H | $COCH_3$ | 2,4-di-F | |
| 146. | $CH_3$ | Cl | H | $T^2$ | 2,6-di-F | |
| 147. | $CH_3$ | Cl | $CH_2C{\equiv}CH$, | H | 4-O-$CH_3$ | |
| 148. | $CH_3$ | Cl | $SCH_2C_6H_5$, | H | 3,5-di-$OCH_3$ | |
| 149. | $CH_3$ | Cl | $CH_2COOCH_3$, | H | 4-O-$C_2H_5$ | |
| 150. | $CH_3$ | Cl | $SO_2CH_3$ | H | 2-$CH_3$ | |
| 151. | $CH_3$ | Cl | $OCH_2C{\equiv}CH$, | H | 2,6-di-$C_2H_5$ | |
| 152. | $CH_3$ | Cl | H | $CH_3$ | 3,5-di-$CH_3$ | |
| 153. | $CH_3$ | Cl | H | CHO | 4-$CF_3$ | |
| 154. | $CH_3$ | Cl | $N(CH_3)_2$ | H | 3-$CF_3$ | |
| 155. | $CH_3$ | Cl | O-i-$C_3H_7$ | H | 2-$CF_3$ | |
| 156. | $CH_3$ | Cl | H | $CH_2OCH_3$ | 4-$OCF_3$ | |
| 157. | $CH_3$ | Cl | S-$COCH_3$ | H | 4-O-Ph | |
| 158. | $CH_3$ | Br | H | H | 4-O-(2,4-di-Cl-Ph) | |
| 159. | $CH_3$ | I | H | H | 4-O-$CH_2$Ph | |
| 160. | $CH_3$ | F | H | H | 4-O-4-F-Ph | |
| 161. | $CH_3$ | Cl | H | S-$CCl_3$ | 4-$COCH_3$ | |
| 162. | $CH_3$ | Cl | S-$CCl_3$ | H | 4-$COOCH_3$ | |
| 163. | $CH_3$ | Cl | $T^3$ | H | 4-$COOC_2H_5$ | |
| 164. | $CH_3$ | Cl | 4-t.-Butyl-phenyl | H | 4-$CONH_2$ | |
| 165. | $CH_3$ | Cl | H | $CH_2C{\equiv}CH$ | 2-$COOC_2H_5$ | |

| Nr. | $R^1$ | $R^2$ | $R^3$ | $R^4$ | $R^5$ - $R^9$ | Smp.(°C): |
|---|---|---|---|---|---|---|
| 166. | $CH_3$ | Cl | H | $CH_2CH=CH_2$ | 3,5-di-$CF_3$ | |
| 167. | $CH_3$ | Cl | H | $CH_2COOCH_3$ | 3,5-di-$CH_3$ | |
| 168. | $CH_3$ | Cl | $C_2H_5$ | H | 2,4-di-$CH_3$ | |
| 169. | $CH_3$ | Cl | n-$C_4H_9$ | H | 3-O-i-$C_3H_7$ | |
| 170. | $CH_3$ | Cl | $CCl_3$ | H | (3,4)-O-$CH_2$O- | |
| 171. | $CH_3$ | Cl | $CF_3$ | H | (1,2)-$CH(CH_3)$-$C(CH_3)_2$- | |
| 172. | $CH_3$ | Cl | $SCH_3$ | H | 4-$N(CH_3)_2$ | |
| 173. | $CH_3$ | Cl | H | $CH_3$ | 3-$NO_2$ | |
| 174. | $CH_3$ | $NH_2$ | $CH_3$ | H | H | |
| 175. | $CH_3$ | $NH_2$ | $C_2H_5$ | H | 4-Cl | |
| 176. | $CH_3$ | $NHCH_3$ | H | H | 4-F | |
| 177. | $CH_3$ | $N(CH_3)_2$ | H | H | 4-$CH_3$ | |
| 178. | $CH_3$ | $NHC_2H_5$ | H | H | 2,4-di-Cl | |
| 179. | $CH_3$ | $NHCH_2$-$CSCH_2$ | H | H | 3,5-di-Cl | |
| 180. | $CH_3$ | 4-Morpholino | H | H | 2-$CH_3$-4-F | |
| 181. | $CH_3$ | $T^4$ | H | H | 2,6-di-F | |
| 182. | $CH_3$ | $NHNH_2$ | H | H | 4-O-$CH_3$ | |
| 183. | $CH_3$ | $NHOCH_3$ | H | H | 3,5-di-$OCH_3$ | |
| 184. | $CH_3$ | NHCHO | H | H | 4-O-$C_2H_5$ | |
| 185. | $CH_3$ | $NHCOCH_3$ | H | H | 2-$CH_3$ | |
| 186. | $CH_3$ | $NHCH_2$-$COOCH_3$ | H | H | 2,6-di-$C_2H_5$ | |
| 187. | $CH_3$ | $T^5$ | H | H | 3,5-di-$CH_3$ | |
| 188. | $CH_3$ | $NH_2$ | H | $CH_3$ | 4-$CF_3$ | |
| 189. | $CH_3$ | $NH_2$ | H | $COCH_3$ | 3-$CF_3$ | |
| 190. | $CH_3$ | $NH_2$ | H | CHO | 2-$CF_3$ | |
| 191. | $CH_3$ | $NH_2$ | H | $CH_2OCH_3$ | 4-$OCF_3$ | |

| Nr. | $R^1$ | $R^2$ | $R^3$ | $R^4$ | $R^5$ - $R^9$ | Smp.(°C): |
|---|---|---|---|---|---|---|
| 192. | $CH_3$ | $NH_2$ | 4-t.-Butyl-phenyl | H | 4-O-Ph | |
| 193. | $CH_3$ | $NH_2$ | 2-Furyl | H | 4-O-(2,4-di-Cl-Ph) | |
| 194. | $CH_3$ | $NH_2$ | n-$C_4H_9$ | H | 4-O-$CH_2$Ph | |
| 195. | $CH_3$ | $NH_2$ | $C_6H_5$ | H | 4-O-4-F-Ph | |
| 196. | $CH_3$ | $NH_2$ | $CF_3$ | H | 4-$COCH_3$ | |
| 197. | $CH_3$ | $NH_2$ | $SCH_3$ | H | 4-$COOCH_3$ | |
| 198. | $CH_3$ | $NH_2$ | $SO_2CH_3$ | H | 4-$COOC_2H_5$ | |
| 199. | $CH_3$ | $NH_2$ | $OCH_3$ | H | 4-COO-i-$C_3H_7$ | |
| 200. | $CH_3$ | $NH_2$ | $OCH_2CH{=}CH_2$, | H | 4-$CONH_2$ | |
| 201. | $CH_3$ | $NH_2$ | $CCl_3$ | H | 2-$COOC_2H_5$ | |
| 202. | $CH_3$ | $NH_2$ | H | $CH_2N(CH_3)_2$ | 3,5-di-$CF_3$ | |
| 203. | $CH_3$ | $NH_2$ | H | H | 3-$CH_3$-5-Cl- | |
| 204. | $CH_3$ | $NHC_2H_5$ | H | H | 2,4-di-$CH_3$ | |
| 205. | $CH_3$ | $NH_2$ | $CH_3$ | $CH_3$ | 3-O-i-$C_3H_7$ | |
| 206. | $CH_3$ | $NH_2$ | H | $C_2H_5$ | (3,4)-O-$CH_2$O- | |
| 207. | $CH_3$ | $NH_2$ | $N(CH_3)_2$ | H | (1,2)-$CH(CH_3)$-$C(CH_3)_2$- | |
| 208. | $CH_3$ | $NH_2$ | $T^5$ | H | 4-$N(CH_3)_2$ | |
| 209. | $CH_3$ | $NH_2$ | $T^6$ | H | 3-$NO_2$ | |
| 210. | $CH_3$ | $NH_2$ | $T^4$ | H | 2-$SCH_3$ | |
| 211. | $CCl_3$ | H | H | H | H | |
| 212. | $CCl_3$ | H | H | H | 4-Cl | |
| 213. | $CCl_3$ | H | H | H | 4-F | |
| 214. | $CCl_3$ | H | H | H | 4-$CH_3$ | |
| 215. | $CCl_3$ | H | H | H | 2,4-di-Cl | |
| 216. | $CCl_3$ | H | H | H | 2,4-di-F | |
| 217. | $CCl_3$ | H | H | H | 4-O-$CH_3$ | |
| 218. | $CCl_3$ | H | H | H | 4-O-$C_2H_5$ | |
| 219. | $CCl_3$ | H | H | H | 2-$CH_3$ | |
| 220. | $CCl_3$ | H | H | H | 2,6-di-$C_2H_5$ | |

| Nr. | $R^1$ | $R^2$ | $R^3$ | $R^4$ | $R^5$ - $R^9$ | Smp.(°C): |
|---|---|---|---|---|---|---|
| 221. | $CCl_3$ | H | H | H | 3,5-di-$CH_3$ | |
| 222. | $CCl_3$ | H | H | H | 4-$CF_3$ | |
| 223. | $CCl_3$ | H | H | H | 3-$CF_3$ | |
| 224. | $CCl_3$ | H | H | H | 2-$CF_3$ | |
| 225. | $CCl_3$ | H | H | H | 4-$OCF_3$ | |
| 226. | $CCl_3$ | H | H | H | 4-O-Ph | |
| 227. | $CCl_3$ | H | H | H | 4-O-(2,4-di-Cl-Ph) | |
| 228. | $CCl_3$ | H | H | H | 4-O-$CH_2$Ph | |
| 229. | $CCl_3$ | H | H | H | 4-O-4-F-Ph | |
| 230. | $CCl_3$ | H | H | H | 4-$COCH_3$ | |
| 231. | $CCl_3$ | H | H | H | 4-CN | |
| 232. | $CCl_3$ | H | H | H | 4-$COOC_2H_5$ | |
| 233. | $CCl_3$ | H | H | H | 4-$CONH_2$ | |
| 234. | $CCl_3$ | H | H | H | 2-$COOC_2H_5$ | |
| 235. | $CCl_3$ | H | H | H | 3,5-di-$CF_3$ | |
| 236. | $CCl_3$ | $C_2H_5$ | H | H | 3,5-di-$CH_3$ | |
| 237. | $CCl_3$ | H | H | H | 2,4-di-$CH_3$ | |
| 238. | $CCl_3$ | H | H | H | 3-O-i-$C_3H_7$ | |
| 239. | $CCl_3$ | H | H | H | (3,4)-O-$CH_2$O- | |
| 240. | $CCl_3$ | H | H | H | (1,2)-$CH(CH_3)$-$C(CH_3)_2$- | |
| 241. | $CCl_3$ | H | H | H | 4-$N(CH_3)_2$ | |
| 242. | $CCl_3$ | H | H | H | 3-$NO_2$ | |
| 243. | $CCl_3$ | Cl | H | H | H | |
| 244. | $CCl_3$ | Cl | H | H | 4-Cl | |
| 245. | $CCl_3$ | Cl | H | H | 4-F | |
| 246. | $CCl_3$ | Cl | H | H | 4-$CH_3$ | |
| 247. | $CCl_3$ | Cl | H | H | 2,4-di-Cl | |
| 248. | $CCl_3$ | Cl | H | H | 3,5-di-Cl | |
| 249. | $CCl_3$ | Cl | H | H | 2,4-di-F | |
| 250. | $CCl_3$ | Cl | H | H | 2,6-di-F | |

| Nr. | $R^1$ | $R^2$ | $R^3$ | $R^4$ | $R^5$ - $R^9$ | Smp.(°C): |
|---|---|---|---|---|---|---|
| 251. | $CCl_3$ | Cl | H | H | 4-O-$CH_3$ | |
| 252. | $CCl_3$ | Cl | H | H | 3,5-di-$OCH_3$ | |
| 253. | $CCl_3$ | Cl | H | H | 4-O-$C_2H_5$ | |
| 254. | $CCl_3$ | Cl | H | H | 2-$CH_3$ | |
| 255. | $CCl_3$ | Cl | H | H | 2,6-di-$C_2H_5$ | |
| 256. | $CCl_3$ | Cl | H | H | 3,5-di-$CH_3$ | |
| 257. | $CCl_3$ | Cl | H | H | 4-$CF_3$ | |
| 258. | $CCl_3$ | Cl | H | H | 3-$CF_3$ | |
| 259. | $CCl_3$ | Cl | H | H | 2-$CF_3$ | |
| 260. | $CCl_3$ | Cl | H | H | 4-$OCF_3$ | |
| 261. | $CCl_3$ | Cl | H | H | 4-t.-$C_4H_9$ | |
| 262. | $CCl_3$ | Cl | H | H | 4-O-(2,4-di-Cl-Ph) | |
| 263. | $CCl_3$ | Cl | H | H | 4-O-$CH_2$Ph | |
| 264. | $CCl_3$ | Cl | H | H | 4-O-4-F-Ph | |
| 265. | $CCl_3$ | Cl | H | H | 4-$COCH_3$ | |
| 266. | $CCl_3$ | Cl | H | H | 4-$COOCH_3$ | |
| 267. | $CCl_3$ | Cl | H | H | 4-$COOC_2H_5$ | |
| 268. | $CCl_3$ | Cl | H | H | 4-$CONH_2$ | |
| 269. | $CCl_3$ | Cl | H | H | 2-$COOC_2H_5$ | |
| 270. | $CCl_3$ | Cl | H | H | 3,5-di-$CF_3$ | |
| 271. | $CCl_3$ | Cl | H | H | 3-Cl-5-$CH_3$ | |
| 272. | $CCl_3$ | Cl | H | H | 2,4-di-$CH_3$ | |
| 273. | $CCl_3$ | Cl | H | H | 3-O-i-$C_3H_7$ | |
| 274. | $CCl_3$ | Cl | H | H | (3,4)-O-$CH_2$O- | |
| 275. | $CCl_3$ | Cl | H | H | (1,2)-$CH(CH_3)$-$C(CH_3)_2$- | |
| 276. | $CCl_3$ | Cl | H | H | 4-$N(CH_3)_2$ | |
| 277. | $CCl_3$ | Cl | H | H | 3-$NO_2$ | |
| 278. | $CCl_3$ | $NH_2$ | H | H | H | |
| 279. | $CCl_3$ | $NH_2$ | H | H | 4-Cl | |
| 280. | $CCl_3$ | $NH_2$ | H | H | 4-F | |

| Nr. | $R^1$ | $R^2$ | $R^3$ | $R^4$ | $R^5$ - $R^9$ | Smp.(°C): |
|---|---|---|---|---|---|---|
| 281. | $CCl_3$ | $NH_2$ | H | H | 4-$CH_3$ | |
| 282. | $CCl_3$ | $NH_2$ | H | H | 2,4-di-Cl | |
| 283. | $CCl_3$ | $NH_2$ | H | H | 3,5-di-Cl | |
| 284. | $CCl_3$ | $NH_2$ | H | H | 2-$CH_3$-4-F | |
| 285. | $CCl_3$ | $NH_2$ | H | H | 2,6-di-F | |
| 286. | $CCl_3$ | $NH_2$ | H | H | 4-O-$CH_3$ | |
| 287. | $CCl_3$ | $NH_2$ | H | H | 3,5-di-$OCH_3$ | |
| 288. | $CCl_3$ | $NH_2$ | H | H | 4-O-$C_2H_5$ | |
| 289. | $CCl_3$ | $NH_2$ | H | H | 2-$CH_3$ | |
| 290. | $CCl_3$ | $NH_2$ | H | H | 2,6-di-$C_2H_5$ | |
| 291. | $CCl_3$ | $NH_2$ | H | H | 3,5-di-$CH_3$ | |
| 292. | $CCl_3$ | $NH_2$ | H | H | 4-$CF_3$ | |
| 293. | $CCl_3$ | $NH_2$ | H | H | 3-CCl3 | |
| 294. | $CCl_3$ | $NH_2$ | H | H | 2-$CF_3$ | |
| 295. | $CCl_3$ | $NH_2$ | H | H | 4-$OCF_3$ | |
| 296. | $CCl_3$ | $NH_2$ | H | H | 4-O-Ph | |
| 297. | $CCl_3$ | $NH_2$ | H | H | 4-O-(2,4-di-Cl-Ph) | |
| 298. | $CCl_3$ | $NH_2$ | H | H | 4-O-$CH_2$Ph | |
| 299. | $CCl_3$ | $NH_2$ | H | H | 4-O-4-F-Ph | |
| 300. | $CCl_3$ | $NH_2$ | H | H | 4-$COCH_3$ | |
| 301. | $CCl_3$ | $NH_2$ | H | H | 4-$COOCH_3$ | |
| 302. | $CCl_3$ | $NH_2$ | H | H | 4-$COOC_2H_5$ | |
| 303. | $CCl_3$ | $NH_2$ | H | H | 4-COO-i-$C_3H_7$ | |
| 304. | $CCl_3$ | $NH_2$ | H | H | 4-$CONH_2$ | |
| 305. | $CCl_3$ | $NH_2$ | H | H | 2-$COOC_2H_5$ | |
| 306. | $CCl_3$ | $NH_2$ | H | H | 3,5-di-$CF_3$ | |
| 307. | $CCl_3$ | $NH_2$ | H | $CH_3$ | 3,5-di-$CH_3$ | |
| 308. | $CCl_3$ | $NH_2$ | H | H | 2,4-di-$CH_3$ | |
| 309. | $CCl_3$ | $NH_2$ | H | H | 3-O-i-$C_3H_7$ | |
| 310. | $CCl_3$ | $NH_2$ | H | H | (3,4)-O-$CH_2$O- | |

| Nr. | $R^1$ | $R^2$ | $R^3$ | $R^4$ | $R^5$ - $R^9$ | Smp.(°C): |
|---|---|---|---|---|---|---|
| 311. | $CCl_3$ | $NH_2$ | H | H | (1,2)-$CH(CH_3)$-$C(CH_3)_2$- | |
| 312. | $CCl_3$ | $NH_2$ | H | H | 4-$N(CH_3)_2$ | |
| 313. | $CCl_3$ | $NH_2$ | H | H | 3-$NO_2$ | |
| 314. | $CCl_3$ | $NH_2$ | H | H | 2-$SCH_3$ | |
| 315. | $CCl_3$ | H | H | $CH_3$ | H | |
| 316. | $CCl_3$ | H | $CH_3$ | H | 4-Cl | |
| 317. | $CCl_3$ | H | $C_2H_5$ | H | 4-F | |
| 318. | $CCl_3$ | H | $C_6H_5$ | H | 4-$CH_3$ | |
| 319. | $CCl_3$ | H | H | CHO | 2,4-di-Cl | |
| 320. | $CCl_3$ | H | H | H | 3,5-di-Cl | |
| 321. | $CCl_3$ | t-$C_4H_9$ | H | H | 2,4-di-F | |
| 322. | $CCl_3$ | $SCH_3$ | H | H | 4-O-$CH_3$ | |
| 323. | $CCl_3$ | $SO_2CH_3$ | H | H | 4-O-$C_2H_5$ | |
| 324. | $CCl_3$ | H | H | H | 3-$CH_3$ | |
| 325. | $CCl_3$ | $OCH_3$ | H | H | 2,6-di-$C_2H_5$ | |
| 326. | $CCl_3$ | H | n-$C_4H_9$ | H | 3,5-di-$CH_3$ | |
| 327. | $CCl_3$ | H | H | $CH_2OCH_3$ | 4-$CF_3$ | |
| 328. | $CCl_3$ | $CH_2Cl$ | H | H | 3-$CF_3$ | |
| 329. | $CCl_3$ | $CH_2OCH_3$ | H | H | 2-$CF_3$ | |
| 330. | $CCl_3$ | H | $CF_3$ | H | 4-$OCF_3$ | |
| 331. | $CCl_3$ | $CH_2NHCH_3$ | H | H | 4-O-Ph | |
| 332. | $CCl_3$ | H | H | $CH_3$ | 4-O-2,4-di-Cl-Ph | |
| 333. | $CCl_3$ | H | $C_2H_5$ | H | 4-O-$CH_2$Ph | |
| 334. | $CCl_3$ | $OCH_2CH=CH_2$ | H | H | 4-O-4-F-Ph | |
| 335. | $CCl_3$ | $OCH_2C\equiv CH$ | H | H | 4-$COCH_3$ | |
| 336. | $CCl_3$ | H | H | $CH_2C\equiv CH$ | 4-$COOCH_3$ | |
| 337. | $CCl_3$ | $OCH_2C\equiv CH$ | H | H | 4-$COOC_2H_5$ | |
| 338. | $CCl_3$ | H | H | $CH_3$ | 4-$CONH_2$ | |
| 339. | $CCl_3$ | O-n-$C_3H_7$ | H | H | 2-$COOC_2H_5$ | |
| 340. | $CFCl_{32}$ | H | $CH_3$ | H | 3,5-di-$CF_3$ | |

| Nr. | $R^1$ | $R^2$ | $R^3$ | $R^4$ | $R^5$ - $R^9$ | Smp.(°C): |
|---|---|---|---|---|---|---|
| 341. | $CCl_3$ | H | 2-Thienyl | H | 3,5-di-$CH_3$ | |
| 342. | $CCl_3$ | H | $SCH_3$ | H | 2,4-di-$CH_3$ | |
| 343. | $CCl_3$ | H | $OCH_3$ | H | 3-O-i-$C_3H_7$ | |
| 344. | $CCl_3$ | H | $N(CH_3)_2$ | H | (3,4)-O-$CH_2$O- | |
| 345. | $CCl_3$ | $SCH_3$ | H | H | (1,2)-CH($CH_3$)-C($CH_3$)$_2$- | |
| 346. | $CCl_3$ | $CF_3$ | H | H | 4-$N(CH_3)_2$ | |
| 347. | $CCl_3$ | $CCl_3$ | H | H | 3-$NO_2$ | |
| 348. | $CCl_3$ | Cl | 2-Thienyl | H | H | |
| 349. | $CCl_3$ | Cl | 2-Furyl | H | 4-Cl | |
| 350. | $CCl_3$ | Cl | H | $C_6H_5$ | 4-F | |
| 351. | $CCl_3$ | Cl | 4-F-$C_6H_4$ | H | 4-$CH_3$ | |
| 352. | $CCl_3$ | Cl | H | $CH_3$ | 2,4-di-Cl | |
| 353. | $CCl_3$ | Cl | 2-Thienyl | H | 3,5-di-Cl | |
| 354. | $CCl_3$ | Cl | H | $COCH_3$ | 2,4-di-F | |
| 355. | $CCl_3$ | Cl | $T^2$ | H | 2,6-di-F | |
| 356. | $CCl_3$ | Cl | $SCH_2C{\equiv}CH$, | H | 4-O-$CH_3$ | |
| 357. | $CCl_3$ | Cl | $SCH_2C_6H_5$ | H | 3,5-di-$OCH_3$ | |
| 358. | $CCl_3$ | Cl | $CH_2COOCH_3$, | H | 4-O-$C_2H_5$ | |
| 359. | $CCl_3$ | Cl | $SO_2CH_3$ | H | 2-$CH_3$ | |
| 360. | $CCl_3$ | Cl | $OCH_2C{\equiv}CH$, | H | 2,6-di-$C_2H_5$ | |
| 361. | $CCl_3$ | Cl | H | $CH_3$ | 3,5-di-$CH_3$ | |
| 362. | $CCl_3$ | Cl | H | CHO | 4-$CF_3$ | |
| 363. | $CCl_3$ | Cl | $N(CH_3)_2$ | H | 3-$CF_3$ | |
| 364. | $CCl_3$ | Cl | O-i-$C_3H_7$ | H | 2-$CF_3$ | |
| 365. | $CCl_3$ | Cl | H | $CH_2OCH_3$ | 4-$OCF_3$ | |
| 366. | $CCl_3$ | Cl | S-$COCH_3$ | H | 4-O-Ph | |
| 367. | $CCl_3$ | Br | H | H | 4-O-(2,4-di-Cl-Ph) | |
| 368. | $CCl_3$ | I | H | H | 4-O-$CH_2$Ph | |
| 369. | $CCl_3$ | F | H | H | 4-O-4-F-Ph | |
| 370. | $CCl_3$ | Cl | H | S-$CCl_3$ | 4-$COCH_3$ | |

| Nr. | $R^1$ | $R^2$ | $R^3$ | $R^4$ | $R^5$ - $R^9$ | Smp.(°C): |
|---|---|---|---|---|---|---|
| 371. | $CCl_3$ | Cl | S-$CCl_3$ | H | 4-$COOC_2H_5$ | |
| 372. | $CCl_3$ | Cl | $T^3$ | H | 4-$COOC_2H_5$ | |
| 373. | $CCl_3$ | Cl | 3-t.-Butyl-phenyl | H | 4-$CONH_2$ | |
| 374. | $CCl_3$ | Cl | H | $CH_2C{\equiv}CH$ | 2-$COOC_2H_5$ | |
| 375. | $CCl_3$ | Cl | H | $CH_2CH{=}CH_2$ | 3,5-di-$CF_3$ | |
| 376. | $CCl_3$ | Cl | H | $CH_2COOCH_3$ | 3,5-di-$CH_3$ | |
| 377. | $CCl_3$ | Cl | $C_2H_5$ | H | 2,4-di-$CH_3$ | |
| 378. | $CCl_3$ | Cl | n-$C_4H_9$ | H | 3-O-i-$C_3H_7$ | |
| 379. | $CCl_3$ | Cl | $CCl_3$ | H | (3,4)-O-$CH_2$O- | |
| 380. | $CCl_3$ | Cl | $CF_3$ | H | (1,2)-$CH(CH_3)$-$C(CH_3)_2$- | |
| 381. | $CCl_3$ | Cl | $SCH_3$ | H | 4-$N(CH_3)_2$ | |
| 382. | $CCl_3$ | Cl | H | $CH_3$ | 3-$NO_2$ | |
| 383. | $CCl_3$ | $NH_2$ | $CH_3$ | H | H | |
| 384. | $CCl_3$ | $NH_2$ | $C_2H_5$ | H | 4-Cl | |
| 385. | $CCl_3$ | $NHCH_3$ | H | H | 4-F | |
| 386. | $CCl_3$ | $N(CH_3)_2$ | H | H | 4-$CH_3$ | |
| 387. | $CCl_3$ | $NHC_2H_5$ | H | H | 2,4-di-Cl | |
| 388. | $CCl_3$ | $NHCH_2C{\equiv}CH$, | H | H | 3,5-di-Cl | |
| 389. | $CCl_3$ | 4-Morpholino | H | H | 2-$CH_3$-4-Cl | |
| 390. | $CCl_3$ | $T^4$ | H | H | 2,6-di-F | |
| 391. | $CCl_3$ | $NHNH_2$ | H | H | 4-O-$CH_3$ | |
| 392. | $CCl_3$ | $NHOCH_3$ | H | H | 3,5-di-$OCH_3$ | |
| 393. | $CCl_3$ | NHCHO | H | H | 4-O-$C_2H_5$ | |
| 394. | $CCl_3$ | $NHCOCH_3$ | H | H | 2-$CH_3$ | |
| 395. | $CCl_3$ | $NHCH_2COOCH_3$, | H | H | 2,6-di-$C_2H_5$ | |
| 396. | $CCl_3$ | $T^5$ | H | H | 3,5-di-$CH_3$ | |
| 397. | $CCl_3$ | $NH_2$ | H | $CH_3$ | 4-$CF_3$ | |
| 398. | $CCl_3$ | $NH_2$ | H | $COCH_3$ | 3-$CF_3$ | |
| 399. | $CCl_3$ | $NH_2$ | H | CHO | 2-$CF_3$ | |

| Nr. | $R^1$ | $R^2$ | $R^3$ | $R^4$ | $R^5$ - $R^9$ | Smp.(°C): |
|---|---|---|---|---|---|---|
| 400. | $CCl_3$ | $NH_2$ | H | $CH_2OCH_3$ | 4-$OCF_3$ | |
| 401. | $CCl_3$ | $NH_2$ | 4-t.-Butyl-phenyl | H | 4-O-Ph | |
| 402. | $CCl_3$ | $NH_2$ | 2-Furyl | H | 4-O-(2,4-di-Cl-Ph) | |
| 403. | $CCl_3$ | $NH_2$ | n-$C_4H_9$ | H | 4-O-$CH_2$Ph | |
| 404. | $CCl_3$ | $NH_2$ | $C_6H_5$ | H | 4-O-4-F-Ph | |
| 405. | $CCl_3$ | $NH_2$ | $CF_3$ | H | 4-$COCH_3$ | |
| 406. | $CCl_3$ | $NH_2$ | $SCH_3$ | H | 4-$COOCH_3$ | |
| 407. | $CCl_3$ | $NH_2$ | $SO_2CH_3$ | H | 4-$COOC_2H_5$ | |
| 408. | $CCl_3$ | $NH_2$ | $OCH_3$ | H | 4-COO-i-$C_3H_7$ | |
| 409. | $CCl_3$ | $NH_2$ | $OCH_2CH=CH_2$, | H | 4-$CONH_2$ | |
| 410. | $CCl_3$ | $NH_2$ | $CCl_3$ | H | 2-$COOC_2H_5$ | |
| 411. | $CCl_3$ | $NH_2$ | H | $CH_2N(CH_3)_2$ | 3,5-di-$CF_3$ | |
| 412. | $CCl_3$ | $NH_2$ | $CH_3$ | H | 3,5-di-$CH_3$ | |
| 413. | $CCl_3$ | $NHC_2H_5$ | H | H | 2,4-di-$CH_3$ | |
| 414. | $CCl_3$ | $NH_2$ | $CH_3$ | $CH_3$ | 3-O-i-$C_3H_7$ | |
| 415. | $CCl_3$ | $NH_2$ | H | $C_2H_5$ | (3,4)-O-$CH_2$O- | |
| 416. | $CCl_3$ | $NH_2$ | $N(CH_3)_2$ | H | (1,2)-$CH(CH_3)$-$C(CH_3)_2$- | |
| 417. | $CCl_3$ | $NH_2$ | $T^5$ | H | 4-$N(CH_3)_2$ | |
| 418. | $CCl_3$ | $NH_2$ | $T^6$ | H | 3-$NO_2$ | |
| 419. | $CCl_3$ | $NH_2$ | $T^4$ | H | 2-$SCH_3$ | |
| 420. | $CF_3$ | H | H | H | H | |
| 421. | $CF_3$ | H | H | H | 4-Cl | |
| 422. | $CF_3$ | H | H | H | 4-F | |
| 423. | $CF_3$ | H | H | H | 4-$CH_3$ | |
| 424. | $CF_3$ | H | H | H | 2,4-di-Cl | |
| 425. | $CF_3$ | H | H | H | 2,4-di-F | |
| 426. | $CF_3$ | H | H | H | 4-O-$CH_3$ | |
| 427. | $CF_3$ | H | H | H | 4-O-$C_2H_5$ | |
| 428. | $CF_3$ | H | H | H | 2-$CH_3$ | |

| Nr. | $R^1$ | $R^2$ | $R^3$ | $R^4$ | $R^5$ - $R^9$ | Smp.(°C): |
|---|---|---|---|---|---|---|
| 429. | $CF_3$ | H | H | H | 2,6-di-$C_2H_5$ | |
| 430. | $CF_3$ | H | H | H | 3,5-di-$CH_3$ | |
| 431. | $CF_3$ | H | H | H | 4-$CF_3$ | |
| 432. | $CF_3$ | H | H | H | 3-$CF_3$ | |
| 433. | $CF_3$ | H | H | H | 2-$CF_3$ | |
| 434. | $CF_3$ | H | H | H | 4-$OCF_3$ | |
| 435. | $CF_3$ | H | H | H | 4-O-Ph | |
| 436. | $CF_3$ | H | H | H | 4-O-(2,4-di-Cl-Ph) | |
| 437. | $CF_3$ | H | H | H | 4-O-$CH_2$Ph | |
| 438. | $CF_3$ | H | H | H | 4-O-4-F-Ph | |
| 439. | $CF_3$ | H | H | H | 4-$COCH_3$ | |
| 440. | $CF_3$ | H | H | H | 4-CN | |
| 441. | $CF_3$ | H | H | H | 4-$COOC_2H_5$ | |
| 442. | $CF_3$ | H | H | H | 4-$CONH_2$ | |
| 443. | $CF_3$ | H | H | H | 2-$COOC_2H_5$ | |
| 444. | $CF_3$ | H | H | H | 3,5-di-$CF_3$ | |
| 445. | $CF_3$ | H | H | H | 3,5-di-$CH_3$-4-Cl | |
| 446. | $CF_3$ | H | H | H | 2,4-di-$CH_3$ | |
| 447. | $CF_3$ | H | H | H | 3-O-i-$C_3H_7$ | |
| 448. | $CF_3$ | H | H | H | (3,4)-O-$CH_2$O- | |
| 449. | $CF_3$ | H | H | H | (1,2)-$CH(CH_3)$-$C(CH_3)_2$- | |
| 450. | $CF_3$ | H | H | H | 4-$N(CH_3)_2$ | |
| 451. | $CF_3$ | H | H | H | 3-$NO_2$ | |
| 452. | $CF_3$ | Cl | H | H | H | 183-184 |
| 453. | $CF_3$ | Cl | H | H | 4-Cl | 148-152 |
| 454. | $CF_3$ | Cl | H | H | 4-F | 151-152 |
| 455. | $CF_3$ | Cl | H | H | 4-$CH_3$ | |
| 456. | $CF_3$ | Cl | H | H | 2,4-di-Cl | 198-200 |
| 457. | $CF_3$ | Cl | H | H | 3,5-di-Cl | 197-199 |
| 458. | $CF_3$ | Cl | H | H | 2,4-di-F | 147-148 |

| Nr. | $R^1$ | $R^2$ | $R^3$ | $R^4$ | $R^5$ - $R^9$ | Smp.(°C): |
|---|---|---|---|---|---|---|
| 459. | $CF_3$ | Cl | H | H | 2,6-di-F | 187-189 |
| 460. | $CF_3$ | Cl | H | H | 4-O-$CH_3$ | |
| 461. | $CF_3$ | Cl | H | H | 3,5-di-$OCH_3$ | |
| 462. | $CF_3$ | Cl | H | H | 4-O-$C_2H_5$ | |
| 463. | $CF_3$ | Cl | H | H | 2-$CH_3$ | |
| 464. | $CF_3$ | Cl | H | H | 2,6-di-$C_2H_5$ | 222-225 |
| 465. | $CF_3$ | Cl | H | H | 3,5-di-$CH_3$ | 180-182 |
| 466. | $CF_3$ | Cl | H | H | 4-$CF_3$ | 120-122 |
| 467. | $CF_3$ | Cl | H | H | 3-$CF_3$ | 135-137 |
| 468. | $CF_3$ | Cl | H | H | 2-$CF_3$ | |
| 469. | $CF_3$ | Cl | H | H | 4-$OCF_3$ | 150-152 |
| 470. | $CF_3$ | Cl | H | H | 4-t.-$C_4H_9$ | |
| 471. | $CF_3$ | Cl | H | H | 4-O-(2,4-di-Cl-Ph) | 168-170 |
| 472. | $CF_3$ | Cl | H | H | 4-O-$CH_2$Ph | |
| 473. | $CF_3$ | Cl | H | H | 4-O-4-F-Ph | |
| 474. | $CF_3$ | Cl | H | H | 4-$COCH_3$ | |
| 475. | $CF_3$ | Cl | H | H | 4-$COOCH_3$ | |
| 476. | $CF_3$ | Cl | H | H | 4-$COOC_2H_5$ | |
| 477. | $CF_3$ | Cl | H | H | 4-$CONH_2$ | |
| 478. | $CF_3$ | Cl | H | H | 2-$COOC_2H_5$ | |
| 479. | $CF_3$ | Cl | H | H | 3,5-di-$CF_3$ | 156-158 |
| 480. | $CF_3$ | Cl | $N(CH_3)_2$ | H | 3,5-di-$CH_3$ | |
| 481. | $CF_3$ | Cl | H | H | 2,4-di-$CH_3$ | |
| 482. | $CF_3$ | Cl | H | H | 3-O-i-$C_3H_7$ | 127-129 |
| 483. | $CF_3$ | Cl | H | H | (3,4)-O-$CH_2$O- | 183-184 |
| 484. | $CF_3$ | Cl | H | H | (1,2)-CH($CH_3$)-$C(CH_3)_2$- | 224-225 |

| Nr. | $R^1$ | $R^2$ | $R^3$ | $R^4$ | $R^5$ - $R^9$ | Smp.(°C): |
|---|---|---|---|---|---|---|
| 485. | $CF_3$ | Cl | H | H | 4-$N(CH_3)_2$ | |
| 486. | $CF_3$ | Cl | H | H | 3-$NO_2$ | |
| 487. | $CF_3$ | $NH_2$ | H | H | H | |
| 488. | $CF_3$ | $NH_2$ | H | H | 4-Cl | |
| 489. | $CF_3$ | $NH_2$ | H | H | 4-F | 150-153 |
| 490. | $CF_3$ | $NH_2$ | H | H | 4-$CH_3$ | |
| 491. | $CF_3$ | $NH_2$ | H | H | 2,4-di-Cl | |
| 492. | $CF_3$ | $NH_2$ | H | H | 3,5-di-Cl | 297 |
| 493. | $CF_3$ | $NH_2$ | H | H | 2-$CH_3$-4-F | 199-201 |
| 494. | $CF_3$ | $NH_2$ | H | H | 2,6-di-F | |
| 495. | $CF_3$ | $NH_2$ | H | H | 4-O-$CH_3$ | |
| 496. | $CF_3$ | $NH_2$ | H | H | 3,5-di-$OCH_3$ | |
| 497. | $CF_3$ | $NH_2$ | H | H | 4-O-$C_2H_5$ | |
| 498. | $CF_3$ | $NH_2$ | H | H | 2-$CH_3$ | |
| 499. | $CF_3$ | $NH_2$ | H | H | 2,6-di-$C_2H_5$ | |
| 500. | $CF_3$ | $NH_2$ | H | H | 3,5-di-$CH_3$ | |
| 501. | $CF_3$ | $NH_2$ | H | H | 4-$CF_3$ | |
| 502. | $CF_3$ | $NH_2$ | H | H | 3-$CF_3$ | |
| 503. | $CF_3$ | $NH_2$ | H | H | 2-$CF_3$ | |
| 504. | $CF_3$ | $NH_2$ | H | H | 4-$OCF_3$ | 148-151 |
| 505. | $CF_3$ | $NH_2$ | H | H | 4-O-Ph | |
| 506. | $CF_3$ | $NH_2$ | H | H | 4-O-(2,4-di-Cl-Ph) | |
| 507. | $CF_3$ | $NH_2$ | H | H | 4-O-$CH_2$Ph | |
| 508. | $CF_3$ | $NH_2$ | H | H | 4-O-4-F-Ph | |
| 509. | $CF_3$ | $NH_2$ | H | H | 4-$COCH_3$ | |
| 510. | $CF_3$ | $NH_2$ | H | H | 4-$COOCH_3$ | |
| 511. | $CF_3$ | $NH_2$ | H | H | 4-$COOC_2H_5$ | |
| 512. | $CF_3$ | $NH_2$ | H | H | 4-COO-i-$C_3H_7$ | |
| 513. | $CF_3$ | $NH_2$ | H | H | 4-$CONH_2$ | |
| 514. | $CF_3$ | $NH_2$ | H | H | 2-$COOC_2H_5$ | |

| Nr. | $R^1$ | $R^2$ | $R^3$ | $R^4$ | $R^5$ - $R^9$ | Smp.(°C): |
|---|---|---|---|---|---|---|
| 515. | $CF_3$ | $NH_2$ | H | H | 3,5-di-$CF_3$ | |
| 516. | $CF_3$ | $NH_2$ | H | H | 3,5-di-$CH_3$-4-$OCH_3$ | |
| 517. | $CF_3$ | $NH_2$ | H | H | 2,4-di-$CH_3$ | |
| 518. | $CF_3$ | $NH_2$ | H | H | 3-O-i-$C_3H_7$ | |
| 519. | $CF_3$ | $NH_2$ | H | H | (3,4)-O-$CH_2$O- | |
| 520. | $CF_3$ | $NH_2$ | H | H | (1,2)-CH($CH_3$)-C($CH_3$)$_2$- | |
| 521. | $CF_3$ | $NH_2$ | H | H | 4-N($CH_3$)$_2$ | |
| 522. | $CF_3$ | $NH_2$ | H | H | 3-$NO_2$ | |
| 523. | $CF_3$ | $NH_2$ | H | H | 2-$SCH_3$ | 182-184 |
| 524. | $CF_3$ | H | H | $CH_3$ | H | |
| 525. | $CF_3$ | H | $CH_3$ | H | 4-Cl | |
| 526. | $CF_3$ | H | $C_2H_5$ | H | 4-F | |
| 527. | $CF_3$ | H | $C_6H_5$ | H | 4-$CH_3$ | |
| 528. | $CF_3$ | H | H | CHO | 2,4-di-Cl | |
| 529. | $CF_3$ | H | H | H | 3,5-di-Cl | 186-188 |
| 530. | $CF_3$ | H | H | H | 3-F | |
| 531. | $CF_3$ | $SCH_3$ | H | H | 4-O-$CH_3$ | |
| 532. | $CF_3$ | $SO_2CH_3$ | H | H | 4-O-$C_2H_5$ | |
| 533. | $CF_3$ | H | H | H | 2-$CH_3$-3-Cl | |
| 534. | $CF_3$ | $OCH_3$ | H | H | 2,6-di-$C_2H_5$ | |
| 535. | $CF_3$ | H | n-$C_4H_9$ | H | 3,5-di-$CH_3$ | |
| 536. | $CF_3$ | H | H | $CH_2OCH_3$ | 4-$CF_3$ | |
| 537. | $CF_3$ | $CH_2Cl$ | H | H | 3-$CF_3$ | |
| 538. | $CF_3$ | $CH_2OCH_3$ | H | H | 2-$CF_3$ | |
| 539. | $CF_3$ | H | $CF_3$ | H | 4-$OCF_3$ | |
| 540. | $CF_3$ | $CH_2NHCH_3$ | H | H | 4-O-Ph | |
| 541. | $CF_3$ | H | H | $CH_3$ | 4-O-(2,4-di-Cl-Ph) | |
| 542. | $CF_3$ | H | $C_2H_5$ | H | 4-O-$CH_2$Ph | |
| 543. | $CF_3$ | $OCH_2CH=CH_2$, | H | H | 4-O-4-F-Ph | |
| 544. | $CF_3$ | $OCH_2C\equiv CH$, | H | H | 4-$COCH_3$ | |

| Nr. | $R^1$ | $R^2$ | $R^3$ | $R^4$ | $R^5$ - $R^9$ | Smp.(°C): |
|---|---|---|---|---|---|---|
| 545. | $CF_3$ | H | H | $CH_2C{\equiv}CH$ | 4-$COOCH_3$ | |
| 546. | $CF_3$ | $OCH_2C{\equiv}CH$, | H | H | 4-$COOC_2H_5$ | |
| 547. | $CF_3$ | H | H | $CH_3$ | 4-$CONH_2$ | |
| 548. | $CF_3$ | O-n-$C_3H_7$ | H | H | 2-$COOC_2H_5$ | |
| 549. | $CF_2Cl$ | H | $CH_3$ | H | 3,5-di-$CF_3$ | |
| 550. | $CF_3$ | H | 2-Thienyl | H | 3,5-di-$CH_3$ | |
| 551. | $CF_3$ | H | $SCH_3$ | H | 2,4-di-$CH_3$ | |
| 552. | $CF_3$ | H | $OCH_3$ | H | 3-O-i-$C_3H_7$ | |
| 553. | $CF_3$ | H | $N(CH_3)_2$ | H | (3,4)-O-$CH_2$O- | |
| 554. | $CF_3$ | $SCH_3$ | H | H | (1,2)-$CH(CH_3)$-$C(CH_3)_2$- | |
| 555. | $CF_3$ | $CF_3$ | H | H | 4-$N(CH_3)_2$ | |
| 556. | $CF_3$ | $CCl_3$ | H | H | 3-$NO_2$ | |
| 557. | $CF_3$ | Cl | 2-Thienyl | H | 3,5-di-Cl | |
| 558. | $CF_3$ | Cl | 2-Furyl | H | 4-Cl | |
| 559. | $CF_3$ | Cl | H | $C_6H_5$ | 4-F | |
| 560. | $CF_3$ | Cl | 4-F-$C_6H_4$ | H | 4-$CH_3$ | |
| 561. | $CF_3$ | Cl | H | $CH_3$ | 2,4-di-Cl | |
| 562. | $CF_3$ | Cl | 2-Thienyl | H | 3,5-di-Cl | |
| 563. | $CF_3$ | Cl | H | $COCH_3$ | 2,4-di-Cl | |
| 564. | $CF_3$ | Cl | $T^2$ | H | 2,6-di-F | |
| 565. | $CF_3$ | Cl | $SCH_2C{\equiv}CH$, | H | 4-O-$CH_3$ | |
| 566. | $CF_3$ | Cl | $SCH_2C_6H_5$ | H | 3,5-di-$OCH_3$ | |
| 567. | $CF_3$ | Cl | $CH_2COOCH_3$, | H | 4-O-$C_2H_5$ | |
| 568. | $CF_3$ | Cl | $SO_2CH_3$ | H | 2-$CH_3$ | |
| 569. | $CF_3$ | Cl | $OCH_2C{\equiv}CH$, | H | 2,6-di-$C_2H_5$ | |
| 570. | $CF_3$ | Cl | H | $CH_3$ | 3,5-di-$CH_3$ | |
| 571. | $CF_3$ | Cl | H | CHO | 4-$CF_3$ | |
| 572. | $CF_3$ | Cl | $N(CH_3)_2$ | H | 3-$CF_3$ | |
| 573. | $CF_3$ | Cl | O-i-$C_3H_7$ | H | 2-$CF_3$ | |
| 574. | $CF_3$ | Cl | H | $CH_2OCH_3$ | 4-$OCF_3$ | |

| Nr. | $R^1$ | $R^2$ | $R^3$ | $R^4$ | $R^5$ - $R^9$ | Smp.(°C): |
|---|---|---|---|---|---|---|
| 575. | $CF_3$ | Cl | S-$COCH_3$ | H | 4-O-Ph | |
| 576. | $CF_3$ | Br | H | H | 4-O-2,4-di-Cl-Ph | |
| 577. | $CF_3$ | I | H | H | 4-O-$CH_2$Ph | |
| 578. | $CF_3$ | F | H | H | 4-O-4-F-Ph | |
| 579. | $CF_3$ | Cl | H | S-$CCl_3$ | 4-$COCH_3$ | |
| 580. | $CF_3$ | Cl | S-$CCl_3$ | H | 4-$COOCH_3$ | |
| 581. | $CF_3$ | Cl | $T^3$ | H | 4-$COOC_2H_5$ | |
| 582. | $CF_3$ | Cl | 4-t.-Butyl-phenyl | H | 4-$CONH_2$ | |
| 583. | $CF_3$ | Cl | H | $CH_2C\equiv CH$ , | 2-$COOC_2H_5$ | |
| 584. | $CF_3$ | Cl | H | $CH_2CH=CH_2$, | 3,5-di-$CF_3$ | |
| 585. | $CF_3$ | Cl | H | $CH_2COOCH_3$, | 3,5-di-$CH_3$ | |
| 586. | $CF_3$ | Cl | $C_2H_5$ | H | 2,4-di-$CH_3$ | |
| 587. | $CF_3$ | Cl | n-$C_4H_9$ | H | 3-O-i-$C_3H_7$ | |
| 588. | $CF_3$ | Cl | $CCl_3$ | H | (3,4)-O-$CH_2$O- | |
| 589. | $CF_3$ | Cl | $CF_3$ | H | (1,2)-$CH(CH_3)$-$C(CH_3)_2$- | |
| 590. | $CF_3$ | Cl | $SCH_3$ | H | 4-$N(CH_3)_2$ | |
| 591. | $CF_3$ | Cl | H | $CH_3$ | 3-$NO_2$ | |
| 592. | $CF_3$ | $NH_2$ | $CH_3$ | H | H | |
| 593. | $CF_3$ | $NH_2$ | $C_2H_5$ | H | 4-Cl | |
| 594. | $CF_3$ | $NHCH_3$ | H | H | 4-F | |
| 595. | $CF_3$ | $N(CH_3)_2$ | H | H | 4-$CH_3$ | |
| 596. | $CF_3$ | $NHC_2H_5$ | H | H | 2,4-di-Cl | |
| 597. | $CF_3$ | $NHCH_2CSCH_2$, | H | H | 3,5-di-Cl | |
| 598. | $CF_3$ | 4-Morpholino | H | H | 2-$CH_3$-4-F | |
| 599. | $CF_3$ | $T^4$ | H | H | 2,6-di-F | |
| 600. | $CF_3$ | $NHNH_2$ | H | H | 4-O-$CH_3$ | |
| 601. | $CF_3$ | $NHOCH_3$ | H | H | 3,5-di-$OCH_3$ | |
| 602. | $CF_3$ | NHCHO | H | H | 4-O-$C_2H_5$ | |
| 603. | $CF_3$ | $NHCOCH_3$ | H | H | 2-$CH_3$ | |

| Nr. | $R^1$ | $R^2$ | $R^3$ | $R^4$ | $R^5$ - $R^9$ | Smp.(°C): |
|---|---|---|---|---|---|---|
| 604. | $CF_3$ | $NHCH_2COOCH_3$, | H | H | 2,6-di-$C_2H_5$ | |
| 605. | $CF_3$ | $T^5$ | H | H | 3,5-di-$CH_3$ | |
| 606. | $CF_3$ | $NH_2$ | H | $CH_3$ | 4-$CF_3$ | |
| 607. | $CF_3$ | $NH_2$ | H | $COCH_3$ | 3-$CF_3$ | |
| 608. | $CF_3$ | $NH_2$ | H | CHO | 2-$CF_3$ | |
| 609. | $CF_3$ | $NH_2$ | H | $CH_2OCH_3$ | 4-$OCF_3$ | |
| 610. | $CF_3$ | $NH_2$ | 4-t.-Butyl-phenyl | H | 4-O-Ph | |
| 611. | $CF_3$ | $NH_2$ | 2-Thienyl | H | 4-O-2,4-di-Cl-Ph | |
| 612. | $CF_3$ | $NH_2$ | n-$C_4H_9$ | H | 4-O-$CH_2$Ph | |
| 613. | $CF_3$ | $NH_2$ | $C_6H_5$ | H | 4-O-4-F-Ph | |
| 614. | $CF_3$ | $NH_2$ | $CF_3$ | H | 4-$COCH_3$ | |
| 615. | $CF_3$ | $NH_2$ | $SCH_3$ | H | 4-$COOCH_3$ | |
| 616. | $CF_3$ | $NH_2$ | $SO_2CH_3$ | H | 4-$COOC_2H_5$ | |
| 617. | $CF_3$ | $NH_2$ | $OCH_3$ | H | 4-COO-i-$C_3H_7$ | |
| 618. | $CF_3$ | $NH_2$ | $OCH_2CH=CH_2$, | H | 4-$CONH_2$ | |
| 619. | $CF_3$ | $NH_2$ | $CCl_3$ | H | 2-$COOC_2H_5$ | |
| 620. | $CF_3$ | $NH_2$ | H | $CH_2N(CH_3)_2$ | 3,5-di-$CF_3$ | |
| 621. | $CF_3$ | $NH_2$ | $OCH_3$ | H | 3,5-di-$CH_3$ | |
| 622. | $CF_3$ | $NHC_2H_5$ | H | H | 2,4-di-$CH_3$ | |
| 623. | $CF_3$ | $NH_2$ | $CH_3$ | $CH_3$ | 3-O-i-$C_3H_7$ | |
| 624. | $CF_3$ | $NH_2$ | H | $C_2H_5$ | (3,4)-O-$CH_2$O- | |
| 625. | $CF_3$ | $NH_2$ | $N(CH_3)_2$ | H | (1,2)-$CH(CH_3)$-$C(CH_3)_2$- | |
| 626. | $CF_3$ | $NH_2$ | $T^5$ | H | 4-$N(CH_3)_2$ | |
| 627. | $CF_3$ | $NH_2$ | $T^6$ | H | 3-$NO_2$ | |
| 628. | $CF_3$ | $NH_2$ | $T^4$ | H | 2-$SCH_3$ | |
| 629. | $CF_3$ | $CF_3$ | H | H | 2,4-di-Cl | |
| 630. | $CH_3$ | OH | H | H | 2,4-di-F | 220 |
| 631. | $CH_3$ | H | H | H | 2,4-di-Cl | |

| Nr. | $R^1$ | $R^2$ | $R^3$ | $R^4$ | $R^5$ - $R^9$ | Smp.(°C): |
|---|---|---|---|---|---|---|
| 632. | $CH_3$ | $O-CH_2C\equiv CCH_3$ | H | H | 4-Cl | |
| 633. | $CH_3$ | $O-CH_2C\equiv CH$ | H | H | 3,4-dil-Cl | |
| 634. | $CH_3$ | $O-CH_2-CH_3$ | H | H | 3,4-di-Cl | |
| 635. | $CH_3$ | $S-CH(CH_3)_2$ | H | H | 2,4-di-F | |
| 636. | $CH_3$ | $SO_2-CH_2CH_3$ | H | H | 2,4-di-F | |
| 637. | $CH_3$ | $CH_2CH_3$ | H | H | 3,5-di-Cl | |
| 638. | $CClF_2$ | $S-CH_3$ | H | H | 3,5-di-Cl | |
| 639. | $CH_3$ | $O-CH_2-C\equiv C-CH_3$ | H | H | 2,4-di-F | |
| 640. | $CH_3$ | O-4-t.-butyl-Ph | H | H | 2,4-di-F | 159-162 |
| 641. | $CH_3$ | $SO_2CH_3$ | H | H | 2,4-di-Cl | 185-188 |
| 642. | $CH_3$ | $SO_2CH_3$ | H | H | 4-Cl | |
| 643. | $CH_3$ | NH-Ph | H | H | 3,5-di-Cl | 225-226 |
| 644. | $CH_3$ | OH | H | H | 2,4-di-Cl | |
| 645. | $CH_3$ | O-4-F-Ph | H | H | 3,5-di-Cl | |
| 646. | $CH_3$ | $CH_3$ | H | H | 3,5-di-Cl | |
| 647. | $CClF_2$ | $OCH_3$ | H | H | 3,5-di-Cl | |
| 648. | $CF_2CF_3$ | T1 | H | H | 3,5-di-Cl | |
| 649. | $CH_3$ | $N(CH_2-CH_3)_2$ | H | H | 3,5-di-Cl | |
| 650. | $CCl_3$ | Cl | H | H | 3,4-di-Cl | |
| 651. | $CH_3$ | $CCl_3$ | H | H | 3,5-di-Cl | |
| 652. | $CF_3$ | $CH(CH_3)_2$ | H | H | 3,5-di-Cl | |
| 653. | $C(CH_3)_4$ | $NH_2$ | H | H | 3,5-di-Cl | |
| 654. | $CF_3$ | $N(CH_2CH_3)_2$ | H | H | 3,5-di-Cl | |

| Nr. | $R^1$ | $R^2$ | $R^3$ | $R^4$ | $R^5$ - $R^9$ | Smp. (°C): |
|---|---|---|---|---|---|---|
| 655. | $CF_3$ | $C(CH_3)_4$ | H | H | 3-O-$CH(CH_3)_2$ | |
| 656. | $CH_3$ | H | H | H | 2-Cl-5-$CF_3$ | |
| 657. | $CH_3$ | $CF_3$ | H | H | 4-$OCF_3$ | |
| 658. | $CH_3$ | $N(CH_3)_2$ | H | H | 4-$OCF_3$ | 163 |
| 659. | $CH_3$ | H | H | H | 4-$OCF_3$ | 171-173 |
| 660. | $CH_3$ | NH-$CH_2$-$CH_3$ | H | H | 4-$OCF_3$ | 150-155 |
| 661. | $CH_3$ | $OCH_3$ | H | H | 4-$OCF_3$ | 125-130 |
| 662. | $CH_3$ | $SCH_3$ | H | H | 4-$OCF_3$ | 148-149 |
| 663. | $CH_3$ | $SO_2CH_3$ | H | H | $OCF_3$ | 163-165 |
| 664. | $CF_3$ | $CH_3$ | H | H | 3,5-di-Cl | |
| 665. | $CF_3$ | Cl | Cl | H | 4-$CH(CH_3)_2$ | |
| 666. | $CF_3$ | SH | OH | H | 3,5-di-Cl | 240 |
| 667. | $CF_3$ | Cl | H | H | 4-O-Ph-4-Cl | |
| 668. | $CF_3$ | NH-Ph-4-O-Ph-4-Cl | H | H | 4-O-Ph-4-Cl | 224-226 |
| 669. | $CF_3$ | Cl | H | H | 3,5-di-Cl-4-$OCF_2CF_2H$ | 154-155 |
| 670. | $CF_3$ | Cl | H | H | 2,4,5-tri-Cl | 202-204 |
| 671. | $CF_3$ | Cl | H | H | 4-Cl-3,5-$CH_3$ | |
| 672. | $CF_3$ | NH-3,5-di-$CH_3$-Ph | H | H | 3,5-di-$CH_3$ | 230-234 |
| 673. | $CF_3$ | t.Butyl | H | H | 3,5-di-Cl | |
| 674. | $CF_3$ | Cl | H | H | (2,3)-O-$CH_2CH_2$ | |
| 675. | $CF_3$ | Cl | H | H | 3-$OCH_2CH_3$ | |
| 676. | $CClF_2$ | Cl | H | H | 4-Cl | |
| 677. | $CF_3$ | NH-4-Cl-Ph | H | H | 4-Cl | 210-211 |
| 678. | $C(CH_3)_2CH_2CH_3Cl$ | Cl | H | H | 3,5-di-Cl | |
| 679. | $CF_3$ | NH-Ph | H | H | H | 194-195 |

| Nr. | $R^1$ | $R^2$ | $R^3$ | $R^4$ | $R^5$ - $R^9$ | Smp.(°C): |
|---|---|---|---|---|---|---|
| 680. | $CF_3$ | Cl | H | H | 3-Cl | 146-147 |
| 681. | $CF_3$ | NH-3-Cl-Ph | H | H | 3-Cl | 177-178 |
| 682 | $CF_3$ | Cl | H | H | 2,6-di-$CH_3$ | |
| 683. | $CF_3$ | Cl | H | H | 3,5-di-Cl-4-$OCH_3$ | 177-178 |
| 684. | $CF_3$ | Cl | H | H | 2,4-di-Cl-6-$CH_3$ | |
| 685. | $CF_3$ | Cl | H | H | 2,5-di-Cl | 218-219 |
| 686. | $CF_3$ | Cl | H | H | 2-$CF_3$-4-Cl | 175-177 |
| 687. | $CF_3$ | Cl | H | H | 3,4-di-Cl | 155-156 |
| 688. | $CF_3$ | Cl | H | H | 3-Cl-4-F | 151-153 |
| 689. | $CF_3$ | NH-3-Cl-4-F-Ph | H | H | 3-Cl-4-F | 161-162 |
| 690. | $CF_3$ | Cl | H | H | 3,4-di-$OCH_3$ | |
| 691. | $CF_3$ | NH-3,4-di-$OCH_3$-Ph | H | H | 3,4-di-$OCH_3$ | 218 |
| 692. | $CF_3$ | Cl | H | H | 3-$CF_3$-5-Cl | |
| 693. | $CF_3$ | Cl | H | H | 3,4,5-tri-Cl | 190 |
| 694. | $CF_3$ | Cl | H | H | 2,3-di-$CH_3$ | 195-196 |
| 695. | $CF_3$ | NH-2,3-di-$CH_3$-Ph | H | H | 2,3-di-$CH_3$ | 228-230 |
| 696. | $CF_3$ | Cl | H | H | 3-Cl-4-CON$(CH_3)_2$ | 258 |
| 697. | $CF_3$ | H | OH | H | 3,5-di-Cl | 285 |
| 698. | $CF_3$ | H | Cl | H | 3,5-di-Cl | |
| 699. | $CF_3$ | Cl | H | H | 2-$OCH_3$-(3,4)-$OCH_2O$- | |
| 700. | $CF_3$ | Cl | H | H | 3-F-5-$CF_3$ | 161-162 |
| 701. | $CF_3$ | Cl | H | H | 3,5-di-F | 154-155 |
| 702. | $CF_3$ | Cl | H | H | 3-$CF_3$-4-F | 154-155 |

| Nr. | $R^1$ | $R^2$ | $R^3$ | $R^4$ | $R^5$ - $R^9$ | Smp.(°C): |
|---|---|---|---|---|---|---|
| 703. | $CF_3$ | OH | H | H | 3-$CF_3$-4-F | 190 |
| 704. | $CF_3$ | Cl | H | H | 3,5-di-$OCH_3$ | |
| 705. | $CF_3$ | OH | H | H | 3,5-di-$OCH_3$ | 268-271 |
| 706. | $CClF_2$ | Cl | H | H | 3,5-di-Cl | |
| 707. | $CF_3$ | H | H | H | (2,3)-$(CH_2)_3$- | |
| 708. | $CF_3$ | $CF_3$ | H | H | 3,4-di-Cl | |
| 709. | $CF_3$ | O-$CH_2C\equiv$ CH | H | H | 3,5-di-Cl | 178 |
| 710. | $CF_3$ | $OCH_3$ | H | H | 3,5-di-Cl | 176-177 |
| 711. | $CH_3$ | OH | H | H | 3,4-di-Cl | |
| 712. | $CF_3$ | Cl | H | H | 3-F-4-Cl | |
| 713. | $CF_3$ | NH-4-F-Ph | H | H | 4-F | 211-213 |
| 714. | $CF_3$ | O-$CH_2$CO $OCH_3$ | H | H | 3,5-di-Cl | 167-169 |
| 715. | $CF_3$ | NH-3,5-di-Cl-Ph | H | H | 3,5-di-Cl | 228-230 |
| 716. | $CF_3$ | $OCH_2CH_3$ | H | H | 3,5-di-Cl | |
| 717. | $CF_3$ | $T^1$ | H | H | 3,5-di -Cl | 137 |
| 718. | $CF_3$ | $T^4$ | H | H | 3,5-di-Cl | 196 |
| 719. | $CClF_2$ | Cl | H | H | 2-Cl-5-$CF_3$ | |
| 720. | $CF_3$ | Cl | H | $CH(CH_3)_2$ | 3,4-di-Cl | 126-128 |
| 721. | $CF_3$ | $NH_2$ | H | H | 3,5-di-Cl-4-$OCF_2CF_2H$ | |
| 722. | $CF_3$ | $NH_2$ | H | H | 3,4-di-Cl | 288-290 |
| 723. | $CF_3$ | Cl | H | H | 2-Cl-4-$CF_3$ | |
| 724. | $CF_3$ | OH | H | H | 3,5-di-Cl | 218-220 |
| 725. | $CF_3$ | Cl | H | H | 3,5-di-Cl-2,4-di-F | 175-176 |
| 726. | $CF_3$ | $NH_2$ | H | H | 2,4-di-F | |
| 727. | 2-$CF_3$ | Cl | H | H | 2-Cl-5-$CF_3$ | 195-197 |

| Nr. | $R^1$ | $R^2$ | $R^3$ | $R^4$ | $R^5$ - $R^9$ | Smp.(°C): |
|---|---|---|---|---|---|---|
| 728. | $CF_3$ | $CF_3$ | H | H | 3,5-di-Cl | |
| 729. | $CF_3$ | Cl | H | H | 2-Cl-4-$CH_3$ | 104-105 |
| 730. | t.Butyl | Cl | H | H | 3,5-di-Cl | 156-158 |
| 731. | t.Butyl | Cl | H | H | 2,4-di-Cl | 130-131 |
| 732. | t.Butyl | Cl | H | $CH(CH_3)_2$ | 3,4-di-Cl | 138-139 |
| 733. | t.Butyl | Cl | H | H | 3-$CF_3$-4-Cl | 178-180 |
| 734. | $CF_3$ | Cl | H | H | 3-$CF_3$-4-Cl | 125-127 |

In der Tabelle verwendete Abkurzungen

$T^1$ 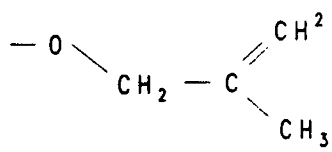

$T^2$ 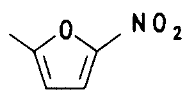

$T^3$ 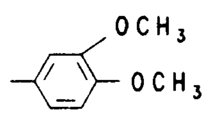

$T^4$ 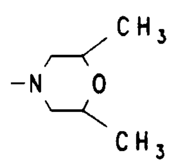

$T^5$ 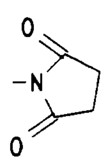

$T^6$ 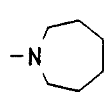

t. Butyl $-C(CH_3)_3$

$-C_6H_5$

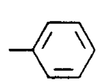

C. Biologische Beispiele

a.) Plasmopara viticola;

Weinsämlinge der Sorten "Riesling/Ehrenfelder" werden ca. 6 Wochen nach der Aussaat mit wäßrigen Suspensionen der beanspruchten Verbindungen tropfnaß behandelt. Nach dem Antrocknen des Spritzbelages werden die Pflanzen mit einer Zoosporangiensuspension von Plasmopara viticola inokuliert und tropfnaß in eine Klimakammer mit 23 °C und 80 - 90 % rel. Luftfeuchte gestellt. Nach einer Inkubationszeit von 7 Tagen werden die Pflanzen nochmals über Nacht in die Klimakammer gestellt, um die Sporulation des Pilzes anzuregen. Anschließend erfolgt die Befallsauswertung. Der Befallsgrad wird in % befallener Blattfläche bezogen auf unbehandelte, zu 100 % infizierte Kontrollpflanzen ausgedrückt.

Verbindungen aus der obigen Tabelle zeigen eine gute Befallsunterdrückung. Bei 500 mg Wirkstoff/l Spritzbrühe zeigen die Verbindungen 453, 456, 457, 459, 465, 467, 479, 489, 493, 504, 669, 670, 680, 683, 687, 688, 693, 694, 700 und 702 eine vollständige Befallsunterdrückung.

b. Botrytis cinerea

Ca. 14 Tage alte Ackerbohnen der Sorte "Harz Freya" oder "Frank's Ackerperle" wurden mit wäßrigen Suspensionen der beanspruchten Verbindungen tropfnaß behandelt. Nach Antrocknen des Spritzbelages wurden die Pflanzen mit einer Sporensuspension (1,5 Mio Sporen/ml) von Botrytis cinerea (BCM-resistenter Stamm) inokuliert. Die Pflanzen wurden in einer Klimakammer bei 20-22° C und ca. 99 % rel. Luftfeuchte weiterkultiviert. Die Infektion der Pflanzen äußert sich in der Bildung schwarzer Flecke auf Blättern und Stengeln. Die Auswertung der Versuche erfolgte ca. 1 Woche nach Inokulation. Der Befallsgrad der Pflanzen wurde prozentual zur unbehandelten, infizierten Kontrolle bonitiert.

Verbindungen aus der obigen Tabelle zeigen eine gute Befallsunterdrückung. Bei 500 mg Wirkstoff/l Spritzbrühe zeigen die Verbindungen 452, 456, 459, 479, 680 und 687 eine vollständige Befallsunterdrükkung.

**Patentansprüche**

**1.** Mittel, enthaltend mindestens eine Verbindung der Formel I oder deren Tautomer

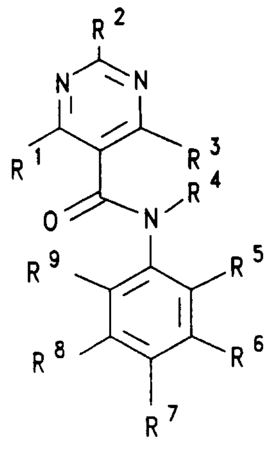

(I)

in welcher

$R^1$ = $(C_1\text{-}C_4)$-Alkyl, Hydroxy-$(C_1\text{-}C_4)$-alkyl, Halo-$(C_1\text{-}C_4)$-alkyl, Perhalo-$(C_1\text{-}C_4)$-alkyl, $(C_1\text{-}C_4)$-Alkoxy-$(C_1\text{-}C_4)$-alkyl, Halo-$(C_1\text{-}C_4)$-alkoxy-$(C_1\text{-}C_4)$-alkyl, $(C_1\text{-}C_4)$-Alkoxy-$(C_1\text{-}C_4)$-haloalkyl, Perhalo$(C_1\text{-}C_4)$-alkoxy-$(C_1\text{-}C_4)$-alkyl, Halo-$(C_1\text{-}C_4)$-alkylthio-$(C_1\text{-}C_4)$-alkyl, Perhalo-$(C_1\text{-}C_4)$-alkylthio-$(C_1\text{-}C_4)$-alkyl, $(C_1\text{-}C_4)$-Alkylthio-$(C_1\text{-}C_4)$-alkyl, $(C_1\text{-}C_4)$-Alkylamino-$(C_1\text{-}C_4)$-alkyl, $(C_1\text{-}C_4)$-Dialkylamino-$(C_1\text{-}C_4)$-alkyl, $(C_3\text{-}C_9)$-Cycloalkylamino-$(C_1\text{-}C_4)$-alkyl, $(C_3\text{-}C_9)$-Cycloalkyl, $(C_3\text{-}C_9)$-Cycloalkyl-$(C_1\text{-}C_4)$-alkyl oder Cyano-$(C_1\text{-}C_4)$-alkyl bedeutet und unter Halo die Halogenatome Cl, Br und insbesondere F zu verstehen sind;

$R^2$ = Wasserstoff, Hydroxy, Amino, Mercapto, Halogen, ($C_1$-$C_8$)-Alkyl, Hydroxy-($C_1$-$C_4$)-alkyl, Dihydroxy-($C_1$-$C_4$)-alkyl, Cyano-($C_1$-$C_4$)-alkyl, Halo-($C_1$-$C_4$)-alkyl, Perhalo-($C_1$-$C_4$)-alkyl, ($C_1$-$C_6$)-Alkoxy, ($C_2$-$C_6$)-Alkenyloxy, ($C_2$-$C_6$)-Alkinyloxy, ($C_1$-$C_4$)-Alkoxy-($C_1$-$C_4$)-alkyl, Halo($C_1$-$C_4$)-alkoxy-($C_1$-$C_4$)-alkyl, Perhalo-($C_1$-$C_4$)-alkoxy-($C_1$-$C_4$)-alkyl, ($C_1$-$C_4$)-Alkoxy-($C_1$-$C_4$)-haloalkyl, ($C_1$-$C_4$)-Alkylthio, Halo-($C_1$-$C_4$)-alkylthio, Perhalo-($C_1$-$C_4$)-alkylthio, Halo-($C_1$-$C_4$)-alkylthio-($C_1$-$C_4$)-alkyl, Perhalo-($C_1$-$C_4$)-alkylthio-($C_1$-$C_4$)-alkyl, ($C_1$-$C_4$)-Alkylthio-($C_1$-$C_4$)-alkyl, ($C_1$-$C_4$)-Alkylsulfonyl, ($C_1$-$C_4$)-Alkylsulfonyl-($C_1$-$C_4$)-alkyl, ($C_1$-$C_4$)-Alkoxycarbonyl-($C_1$-$C_4$)alkyl, ($C_1$-$C_4$)-Alkylamino-($C_1$-$C_4$)-alkyl, ($C_1$-$C_4$)-Dialkylamino-($C_1$-$C_4$)-alkyl, ($C_3$-$C_9$)-Cycloalkylamino-($C_1$-$C_4$)-alkyl, ($C_3$-$C_9$)-Cycloalkyl, ($C_3$-$C_9$)-Cycloalkyl-($C_1$-$C_4$)-alkyl oder ($C_3$-$C_9$)-Heterocycloalkyl-($C_1$-$C_4$)-alkyl bedeutet, wobei die cyclischen Reste bis zu dreifach durch ($C_1$-$C_4$)-Alkyl substituiert sein können und unter Halo die Halogenatome F, Br und/oder Cl zu verstehen sind;

$R^3$ = Wasserstoff, Hydroxy, Amino, Mercapto, Halogen, ($C_1$-$C_8$)-Alkyl, Hydroxy-($C_1$-$C_4$)-alkyl, Halo-($C_1$-$C_4$)-alkyl, Perhalo-($C_1$-$C_4$)-alkyl, ($C_1$-$C_6$)-Alkoxy, ($C_2$-$C_6$)-Alkenyloxy, ($C_2$-$C_6$)-Alkinyloxy, ($C_1$-$C_4$)-Alkoxy-($C_1$-$C_4$)-alkyl, Halo-($C_1$-$C_4$)-alkoxy-($C_1$-$C_4$)-alkyl, ($C_1$-$C_4$)-Alkoxy-($C_1$-$C_4$)-haloalkyl, Perhalo($C_1$-$C_4$)-alkoxy-($C_1$-$C_4$)-alkyl, ($C_1$-$C_4$)-Alkylthio, Halo-($C_1$-$C_4$)-alkylthio, Perhalo-($C_1$-$C_4$)-alkylthio, Halo-($C_1$-$C_4$)-alkylthio-($C_1$-$C_4$)-alkyl, Perhalo($C_1$-$C_4$)-alkylthio-($C_1$-$C_4$)-alkyl, ($C_1$-$C_4$)-Alkylthio-($C_1$-$C_4$)-alkyl, ($C_1$-$C_4$)-Alkylsulfonyl, ($C_1$-$C_4$)-Alkylsulfonyl-($C_1$-$C_4$)-alkyl, ($C_1$-$C_4$)-Alkoxycarbonyl-($C_1$-$C_4$)-alkyl, ($C_1$-$C_4$)-Alkylamino-($C_1$-$C_4$)-alkyl, ($C_1$-$C_4$)-Dialkylamino-($C_1$-$C_4$)-alkyl, ($C_3$-$C_9$)-Cycloalkylamino-($C_1$-$C_4$)-alkyl, ($C_3$-$C_9$)-Cycloalkyl, ($C_3$-$C_9$)-Cycloalkyl-($C_1$-$C_4$)-alkyl, ($C_3$-$C_9$)-Heterocycloalkyl-($C_1$-$C_4$)-alkyl, gegebenenfalls substituiertes Phenyl, gegebenenfalls substituiertes ($C_1$-$C_{10}$)-Heteroaryl, gegebenenfalls substituiertes Phenoxy-($C_1$-$C_4$)-alkyl, gegebenenfalls substituiertes Phenyl-($C_1$-$C_4$)-alkyl, gegebenenfalls substituiertes Phenylmercapto-($C_1$-$C_4$)-alkyl, gegebenenfalls substituiertes Phenylamino-($C_1$-$C_4$)-alkyl oder gegebenenfalls substituiertes Phenoxyphenyl-($C_1$-$C_4$)-alkyl bedeutet, wobei Halo die Halogenatome F,Br und/oder Cl zu verstehen sind;

$R^4$ = Wasserstoff, Formyl, ($C_1$-$C_4$)-Alkyl, ($C_1$-$C_4$)-Alkylcarbonyl, Hydroxy-($C_1$-$C_4$)-alkyl, Halo-($C_1$-$C_4$)-alkyl, Perhalo-($C_1$-$C_4$)-alkyl, ($C_1$-$C_4$)-Alkoxy-($C_1$-$C_4$)-alkyl, Halo($C_1$-$C_4$)-alkoxy-($C_1$-$C_4$)-alkyl, Perhalo($C_1$-$C_4$)-alkoxy-($C_1$-$C_4$)-alkyl, Perhalo-($C_1$-$C_4$)-alkylthio, ($C_1$-$C_4$)-Alkylthio-($C_1$-$C_4$)-alkyl, ($C_2$-$C_6$)-Alkenyl, ($C_2$-$C_6$)-Alkinyl, ($C_1$-$C_4$)-Alkylamino-($C_1$-$C_4$)-alkyl, ($C_1$-$C_4$)-Dialkylamino-($C_1$-$C_4$)-alkyl, ($C_3$-$C_9$)-Cycloalkylamino-($C_1$-$C_4$)-alkyl, ($C_3$-$C_9$)-Cycloalkyl, ($C_3$-$C_9$)-Cycloalkyl-($C_1$-$C_4$)-alkyl, ($C_3$-$C_9$)-Heterocycloalkyl-($C_1$-$C_4$)-alkyl, ($C_1$-$C_4$)-Alkoxycarbonyl-($C_1$-$C_4$)-alkyl oder gegebenenfalls substituiertes Phenyl-($C_1$-$C_4$)-alkyl bedeutet, wobei cyclische Reste bis zu dreifach durch ($C_1$-$C_6$)-Alkyl substituiert sein können und unter Halo die Halogenatome F, Cl, Br zu verstehen sind;

$R^5$, $R^6$, $R^7$, $R^8$ und $R^9$ gleich oder verschieden sind und unabhängig voneinander Wasserstoff, Halogen, Hydroxy, Amino, Nitro, Cyano, Thiocyano, ($C_1$-$C_4$)-Alkyl, Cyano-($C_1$-$C_4$)-alkyl, ($C_1$-$C_4$)-Alkoxy, ($C_1$-$C_4$)-Alkylamino, ($C_1$-$C_4$)-Dialkylamino, ($C_1$-$C_4$)-Alkylcarbonylamino, Halo-($C_1$-$C_4$)-alkyl, Perhalo-($C_1$-$C_4$)-alkyl, Hydroxy-($C_1$-$C_4$)-alkyl, Dihydroxy-($C_1$-$C_4$)-alkyl, ($C_1$-$C_4$)-Alkoxy-($C_1$-$C_4$)-alkyl, Halo($C_1$-$C_4$)-alkoxy-($C_1$-$C_4$)-alkyl, ($C_1$-$C_4$)-Alkoxy-($C_1$-$C_4$)-haloalkyl, Perhalo($C_1$-$C_4$)-alkoxy-($C_1$-$C_4$)-alkyl, ($C_1$-$C_4$)-Alkylthio, Halo-($C_1$-$C_4$)-alkylthio, Perhalo-($C_1$-$C_4$)-alkylthio, Halo-($C_1$-$C_4$)-alkylthio-($C_1$-$C_4$)-alkyl, Perhalo-($C_1$-$C_4$)-alkylthio($C_1$-$C_4$)-alkyl, ($C_1$-$C_4$)-Alkylthio-($C_1$-$C_4$)-alkyl, ($C_2$-$C_6$)-Alkenyl, ($C_2$-$C_6$)-Alkinyl, ($C_1$-$C_4$)-Alkylamino-($C_1$-$C_4$)-alkyl, ($C_1$-$C_4$)-Dialkylamino-($C_1$-$C_4$)-alkyl, ($C_3$-$C_9$)-Cycloalkylamino-($C_1$-$C_4$)-alkyl, ($C_3$-$C_9$)-Cycloalkyl, ($C_3$-$C_9$)-Cycloalkyl-($C_1$-$C_4$)-alkyl, ($C_3$-$C_9$)-Heterocycloalkyl-($C_1$-$C_4$)-alkyl, wobei die cyclischen Reste bis zu dreifach durch ($C_1$-$C_4$)-Alkyl substituiert sein können, gegebenenfalls substituiertes Phenyl, gegebenfalls substituiertes ($C_1$-$C_{10}$)-Heteroaryl, gegebenenfalls substituiertes Phe-

noxy, gegebenenfalls substituiertes Anilino, gegebenenfalls substituiertes Phenylmercapto, gegebenenfalls substituiertes Benzoyl-($C_1$-$C_4$)-alkyl, ($C_1$-$C_4$)-Alkoxycarbonyl-($C_1$-$C_4$)-alkyl, gegebenenfalls substituiertes Phenyloxycarbonyl-($C_1$-$C_4$)-alkyl, ($C_1$-$C_4$)-Alkylaminocarbonyl-($C_1$-$C_4$)-alkyl, ($C_1$-$C_4$)-Dialkylaminocarbonyl-($C_1$-$C_4$)-alkyl, ($C_1$-$C_4$)-Alkylcarbonyl, ($C_1$-$C_4$)-Haloalkylcarbonyl, ($C_1$-$C_4$)-Perhaloalkylcarbonyl, ($C_1$-$C_4$)-Alkyloxycarbonyl, ($C_1$-$C_4$)-Haloalkyloxycarbonyl, ($C_1$-$C_4$)-Perhaloalkyloxycarbonyl, ($C_1$-$C_4$)-Alkylthiocarbonyl, ($C_1$-$C_4$)-Haloalkylthiocarbonyl, ($C_1$-$C_4$)-Perhaloalkylthiocarbonyl, Aminocarbonyl, ($C_1$-$C_4$)-Alkylaminocarbonyl, ($C_1$-$C_4$)-Haloalkylaminocarbonyl, ($C_1$-$C_4$)-Perhaloalkylaminocarbonyl, gegebenenfalls substituiertes Phenoxy-($C_1$-$C_4$)-alkyl, gegebenenfalls substituiertes Phenyl-($C_1$-$C_4$)-alkyl, gegebenenfalls substituiertes Phenylmercapto-($C_1$-$C_4$)-alkyl, gegebenenfalls substituiertes Phenylamino-($C_1$-$C_4$)-alkyl, gegebenenfalls substituiertes Phenyl-($C_1$-$C_4$)-alkylamino, gegebenenfalls substituiertes N-Phenyl-N-($C_1$-$C_4$)-alkylamino oder gegebenenfalls substituiertes Phenoxyphenyl-($C_1$-$C_4$)-alkyl bedeuten, wobei Halo die Halogenatome F, Cl, Br oder J zu verstehen sind; oder

zwei benachbarte Reste aus der Reihe,

$R^5$, $R^6$, $R^7$,$R^8$ und $R^9$ zusammen mit den dazu tragenden C-Atomen für einen teilweise ungesättigten oder aromatischen Isocyclus oder Heterocyclus mit den Heteroatomen O, N, S, Si und/oder P und 4 bis 10 Ringgliedern stehen und die übrigen Reste wie vorstehend definiert sind,

oder deren Säureadditionssalz und mindestens ein geeignetes Formulierungshilfsmittel.

**2.** Mittel gemäß Anspruch 1, enthaltend mindestens eine Verbindung der Formel I oder deren Tautomer, in welcher

$R^1$ = ($C_1$-$C_4$)-Alkyl, Hydroxy-($C_1$-$C_4$)-alkyl, Halo-($C_1$-$C_4$)-alkyl, Perhalo-($C_1$-$C_4$)-alkyl,($C_1$-$C_4$)-Alkoxy-($C_1$-$C_4$)-alkyl, Perhalo($C_1$-$C_4$)-alkoxy-($C_1$-$C_4$)-alkyl, Halo-($C_1$-$C_4$)-alkylthio-($C_1$-$C_4$)-alkyl, ($C_1$-$C_4$)-Alkylamino-($C_1$-$C_4$)-alkyl, ($C_1$-$C_4$)-Dialkylamino-($C_1$-$C_4$)-alkyl, ($C_3$-$C_9$)-Cycloalkylamino-($C_1$-$C_4$)-alkyl, -($C_3$-$C_9$)-Cycloalkyl oder ($C_3$-$C_9$)-Cycloalkyl-($C_1$-$C_4$)-alkyl bedeutet;

$R^2$ = Wasserstoff, Hydroxy, Amino, Mercapto, Halogen, ($C_1$-$C_8$)-Alkyl, Hydroxy-($C_1$-$C_4$)-alkyl, Halo-($C_1$-$C_4$)-alkyl, ($C_1$-$C_6$)-Alkoxy, ($C_2$-$C_6$)-Alkenyloxy, ($C_2$-$C_6$)-Alkinyloxy, ($C_1$-$C_4$)-Alkoxy-($C_1$-$C_4$)-alkyl, Halo-($C_1$-$C_4$)-alkoxy-($C_1$-$C_4$)-alkyl, ($C_1$-$C_4$)-Alkylthio, Halo-($C_1$-$C_4$)-alkylthio, Halo-($C_1$-$C_4$)-alkylthio-($C_1$-$C_4$)-alkyl, ($C_1$-$C_4$)-Alkylsulfonyl, ($C_1$-$C_4$)-Alkylsulfonyl-($C_1$-$C_4$)-alkyl, ($C_1$-$C_4$)-Alkoxycarbonyl-($C_1$-$C_4$)-alkyl, ($C_1$-$C_4$)-Alkylamino-($C_1$-$C_4$)-alkyl, ($C_1$-$C_4$)-Dialkylamino-($C_1$-$C_4$)-alkyl, ($C_3$-$C_9$)-Cycloalkylamino-($C_1$-$C_4$)-alkyl oder ($C_3$-$C_9$)-Cycloalkyl bedeutet, wobei die cyclischen Reste bis zu dreifach durch ($C_1$-$C_4$)-Alkyl substituiert sein können:

$R^3$ = Wasserstoff, Hydroxy, Amino, Mercapto, ($C_1$-$C_8$)-Alkyl, Hydroxy-($C_1$-$C_4$)-alkyl, Halo-($C_1$-$C_4$)-alkyl, ($C_1$-$C_6$)-Alkoxy, ($C_2$-$C_6$)-Alkenyloxy, ($C_2$-$C_6$)-Alkinyloxy, ($C_1$-$C_4$)-Alkoxy-($C_1$-$C_4$)-alkyl, Halo-($C_1$-$C_4$)-alkoxy-($C_1$-$C_4$)-alkyl, ($C_1$-$C_4$)-Alkylthio, Halo-($C_1$-$C_4$)-alkylthio, Halo-($C_1$-$C_4$)-alkylthio-($C_1$-$C_4$)-alkyl, ($C_1$-$C_4$)-Alkylsulfonyl, ($C_1$-$C_4$)-Alkylsulfonyl-($C_1$-$C_4$)-alkyl, ($C_1$-$C_4$)-Alkoxycarbonyl-($C_1$-$C_4$)-alkyl, ($C_1$-$C_4$)-Alkylamino-($C_1$-$C_4$)-alkyl, ($C_1$-$C_4$)-Dialkylamino-($C_1$-$C_4$)-alkyl, ($C_3$-$C_9$)-Cycloalkylamino-($C_1$-$C_4$)-alkyl, ($C_3$-$C_9$)-Cycloalkyl, ($C_3$-$C_9$)-Cycloalkyl-($C_1$-$C_4$)-alkyl, gegebenenfalls substituiertes Phenyl, gegebenenfalls substituiertes ($C_1$-$C_{10}$)-Heteroaryl, gegebenenfalls substituiertes Phenoxy-($C_1$-$C_4$)-alkyl oder gegebenenfalls substituiertes Phenyl-($C_1$-$C_4$)-alkyl bedeutet;

$R^4$ = Wasserstoff, Formyl, ($C_1$-$C_4$)-Alkyl, ($C_1$-$C_4$)-Alkylcarbonyl, Hydroxy-($C_1$-$C_4$)-alkyl, Halo-($C_1$-$C_4$)-alkyl, ($C_1$-$C_4$)-Alkoxy-($C_1$-$C_4$)-alkyl, Halo($C_1$-$C_4$)-alkoxy-($C_1$-$C_4$)-alkyl, ($C_2$-$C_6$)-Alkenyl, ($C_2$-$C_6$)-Alkinyl, ($C_1$-$C_4$)-Alkylamino-($C_1$-$C_4$)-alkyl, ($C_1$-$C_4$)-Dialkylamino-($C_1$-$C_4$)-alkyl, ($C_3$-$C_9$)-Cycloalkylamino-($C_1$-$C_4$)-alkyl, wobei die cyclischen Reste bis zu dreifach durch ($C_1$-$C_4$)-Alkyl substituiert sein können, ($C_1$-$C_4$)-Alkoxycarbonyl-($C_1$-$C_4$)-alkyl oder gegebenenfalls substituiertes Phenyl-($C_1$-$C_4$)-alkyl bedeutet;

$R^5$, $R^6$, $R^7$, $R^8$ und $R^9$ gleich oder verschieden sind und unabhängig voneinander Wasserstoff, Halogen, Hydroxy, Amino, Nitro, Cyano, $(C_1-C_4)$-Alkyl, $(C_1-C_4)$-Alkoxy, $(C_1-C_4)$-Alkylamino, $(C_1-C_4)$-Dialkylamino, $(C_1-C_4)$-Alkylcarbonylamino, Halo-$(C_1-C_4)$-alkyl, Perhalo-$(C_1-C_4)$-alkyl, Hydroxy-$(C_1-C_4)$-alkyl, Dihydroxy-$(C_1-C_4)$-alkyl, $(C_1-C_4)$-Alkoxy-$(C_1-C_4)$-alkyl, Halo$(C_1-C_4)$-alkoxy-$(C_1-C_4)$-alkyl, $(C_1-C_4)$-Alkylthio, Halo-$(C_1-C_4)$-alkylthio, Halo-$(C_1-C_4)$-alkylthio-$(C_1-C_4)$-alkyl, $(C_1-C_4)$-Alkylthio-$(C_1-C_4)$-alkyl, $(C_2-C_6)$-Alkenyl, ( $C_2-C_6$)-Alkinyl, $(C_1-C_4)$-Alkylamino-$(C_1-C_4)$-alkyl, $(C_1-C_4)$-Dialkylamino-$(C_1-C_4)$-alkyl, $(C_3-C_9)$-Cycloalkylamino-$(C_1-C_4)$-alkyl, $(C_3-C_9)$-Cycloalkyl, $(C_3-C_9)$-Cycloalkyl-$(C_1-C_4)$-alkyl, $(C_3-C_9)$-Heterocycloalkyl-$(C_1-C_4)$-alkyl, wobei die cyclischen Reste bis zu dreifach durch $(C_1-C_4)$-Alkyl substituiert sein können, gegebenenfalls substituiertes Phenyl, gegebenenfalls substituiertes $(C_1-C_{10})$-Heteroaryl, gegebenenfalls substituiertes Phenoxy, gegebenenfalls substituiertes Benzoyl-$(C_1-C_4)$-alkyl, $(C_1-C_4)$-Alkoxycarbonyl-$(C_1-C_4)$-alkyl, gegebenenfalls substituiertes Phenyloxycarbonyl-$(C_1-C_4)$-alkyl, $(C_1-C_4)$-Alkylaminocarbonyl-$(C_1-C_4)$-alkyl, $(C_1-C_4)$-Dialkylaminocarbonyl-$(C_1-C_4)$-alkyl, $(C_1-C_4)$-Alkylcarbonyl, $(C_1-C_4)$-Haloalkylcarbonyl, $(C_1-C_4)$-Alkyloxycarbonyl, $(C_1-C_4)$-Haloalkyloxycarbonyl, $(C_1-C_4)$-Alkylthiocarbonyl, $(C_1-C_4)$-Haloalkylthiocarbonyl, $(C_1-C_4)$-Perhaloalkylthiocarbonyl, Aminocarbonyl, $(C_1-C_4)$-Alkylaminocarbonyl $(C_1-C_4)$-Haloalkylaminocarbonyl, $(C_1-C_4)$-Perhaloalkylaminocarbonyl, gegebenenfalls substituiertes Phenoxy-( $C_1-C_4$)-alkyl, gegebenenfalls substituiertes Phenyl-$(C_1-C_4)$-alkyl, gegebenenfalls substituiertes Phenylmercapto-$(C_1-C_4)$-alkyl oder gegebenenfalls substituiertes Phenylamino-$(C_1-C_4)$-alkyl bedeuten; oder

zwei der Reste

$R^5$, $R^6$, $R^7$,$R^8$ und $R^9$ zusammen mit den diese tragenden C-Atomen für einen teilweise ungesättigten oder aromatischen Isocyclus oder Heterocyclus mit den Heteroatomen O, N und/oder S und 4 bis 10 Ringgliedern stehen und die anderen Reste wie vorstehend definiert sind,

oder deren Säureadditionssalz.

**3.** Mittel gemäß einem der Ansprüche 1 oder 2, enthaltend mindestens eine Verbindung der Formel I, in welcher

$R^1$ = $(C_1-C_4)$-Alkyl, Halo-$(C_1-C_4)$-alkyl oder Perhalo-$(C_1-C_4)$-alkyl bedeutet, und unter Halo die Halogenatome Cl und insbesondere F zu verstehen sind;

$R^2$ = Wasserstoff, Hydroxy, Amino, Mercapto, Halogen; $(C_1-C_8)$-Alkyl, $(C_1-C_6)$-Alkoxy, $(C_2-C_6)$-Alkenyloxy, $(C_2-C_6)$-Alkinyloxy, $(C_1-C_4)$-Alkylthio, $(C_1-C_4)$-Alkylsulfonyl, $(C_1-C_4)$-Alkylamino-$(C_1-C_4)$-alkyl oder $(C_1-C_4)$-Dialkylamino-$(C_1-C_4)$-alkyl bedeutet;

$R^3$ = Wasserstoff, Hydroxy, Amino, Mercapto, $(C_1-C_8)$-Alkyl, $(C_1-C_4)$-Alkylthio, gegebenenfalls substituiertes $(C_1-C_8)$-Heteroaryl bedeutet;

$R^4$ = Wasserstoff, Formyl, $(C_1-C_4)$-Alkyl,

$R^5$, $R^6$, $R^7$, $R^8$ und $R^9$ gleich oder verschieden sind und unabhängig voneinander gleich Wasserstoff, Halogen, Hydroxy, Amino, Nitro, Cyano, $(C_1-C_4)$-Alkyl, $(C_1-C_4)$-Alkoxy, $(C_1-C_4)$-Alkylamino, $(C_1-C_4)$-Dialkylamino, gegebenenfalls substituiertes Phenyl oder gegebenenfalls substituiertes Phenoxy bedeuten und unter Halo die Halogenatome F, Cl, Br zu verstehen sind,

oder zwei der Reste

$R^5$, $R^6$, $R^7$,$R^8$ und $R^9$ zusammen mit den diese tragenden C-Atome für einen teilweise ungesättigten oder aromatischen Isocyclus oder Heterocyclus mit den Heteroatomen O und/oder N und 5 bis 10 Ringgliedern stehen und die anderen Reste wie vorstehend definiert sind,

oder deren Säureadditionssalz.

**4.** Verbindung der Formel I, deren Tautomer oder deren Säureadditionssalz, welche wie in einem der Ansprüche 1 bis 3 definiert ist, wobei jene Verbindungen der Formel I, worin $R^1$ = $CF_3$, $R^2$ = Cl, $R^3$ = H, $R^4$ = H und $R^5$ bis $R^9$ = 3,5-Dichlor, 2,6-Difluor, 3-Trifluormethyl oder 4-Trifluormethoxy bedeuten oder worin $R^1$ = $CF_3$, $R^2$ = $NH_2$, $R^3$ = H, $R^4$ = H und $R^5$ bis $R^9$ = 4-Fluor, 4-Trifluormethoxy, 2-Methyl-4-fluor oder 2-Methylthio bedeuten, ausgenommen sind.

**5.** Verfahren zur Herstellung einer Verbindung der Formel I gemäß Anspruch 4, dadurch gekennzeichnet, daß man

a) eine Verbindung der Formel III,

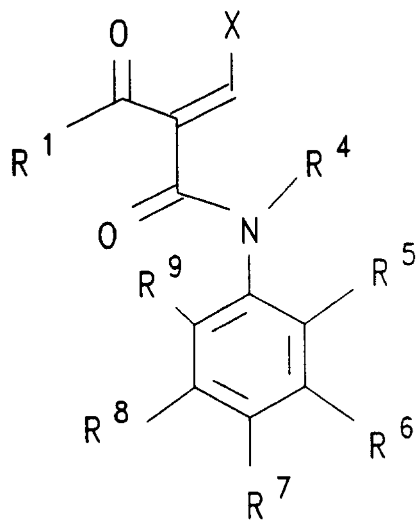

( I I I )

in welcher $R^4$ bis $R^9$ wie in Formel I definiert sind und X eine Abgangsgruppe bedeutet, umsetzt mit dem entsprechenden (Thio)-Harnstoff; (Thio)-Harnstoff-Derivat, Guanidin oder Guanidin-Derivat,

b) eine Verbindung der Formel V,

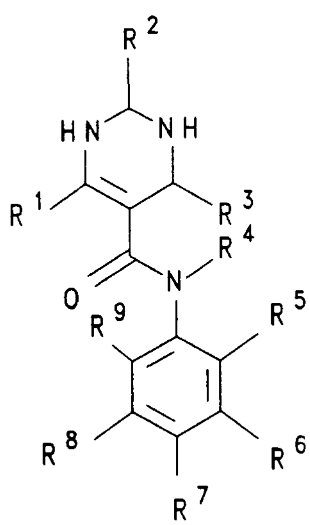

( V )

in welcher $R^1$ bis $R^9$ wie in Formel I definiert sind, oxydiert,

c) eine Verbindung der Formel VII,

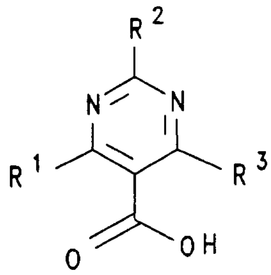

(VII)

in welcher $R^1$, $R^2$ und $R^3$ wie in Formel I definiert sind, gegebenenfalls über das entsprechende Säurechlorid, umsetzt mit einer Verbindung der Formel VIII,

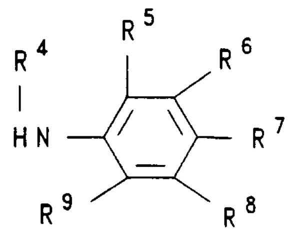

(VIII)

in welcher $R^4$ bis $R^9$ wie in Formel I definiert sind,
in den so erhaltenen Verbindungen der Formel I gegebenenfalls Gruppen oxydiert, Hydroxy gegen Halogen austauscht und/oder Abgangsgruppen nucleophil austauscht und so erhaltene Verbindungen der Formel I gegebenenfalls in ihr Säureadditionssalz überführt.

**6.** Mittel, enthaltend mindestens eine Verbindung gemäß Anspruch 4 und mindestens ein Formulierungsmittel.

**7.** Fungizides Mittel gemäß einem der Ansprüche 1 bis 3 oder 6, enthaltend eine fungizid wirksame Menge mindestens einer wie in den Ansprüche 1 bis 4 definierte Verbindung zusammen mit den für deren Anwendung üblichen Zusatz- oder Hilfsstoffen.

**8.** Pflanzenschutzmittel, enthaltend eine fungizid wirksame Menge mindestens einer wie in den Ansprüchen 1 bis 4 definierten Verbindung und mindestens einem weiteren Wirkstoff, vorzugsweise aus der Reihe der Fungizide, Insektizide, Lockstoffe, Sterilantien, Akariziden, Nematiziden und Herbizide zusammen mit den für diese Anwendung üblichen Hilfs- und Zusatzstoffen.

**9.** Mittel zur Anwendung im Holzschutz oder als Konservierungsmittel in Anstrichfarben, in Kühlschmiermitteln für die Metallbearbeitung oder in Bohr- und Schneidölen, enthaltend eine wirksame Menge mindestens einer wie in den Ansprüchen 1 bis 4 definierten Verbindung zusammen mit den für diese Anwendungen üblichen Hilfs- und Zusatzstoffen.

**10.** Verfahren zur Herstellung eines Mittels gemäß einem der Ansprüche 1 bis 3 und 6 bis 9, dadurch gekennzeichnet, daß man den Wirkstoff und die weiteren Zusätze zusammen gibt und in eine geeignete Anwendungsform bringt.

11. Verwendung einer Verbindung gemäß Anspruch 4 oder eines Mittels gemäß einem der Ansprüche 1 bis 3 und 6 bis 8 als Fungizid.

12. Verwendung einer Verbindung der Formel I gemäß Anspruch 4 oder eines Mittels gemäß einem der Ansprüche 1 bis 3 und 6 bis 9 als Holzschutzmittel oder als Konservierungsmittel in Anstrichfarben, in Kühlschmiermitteln für die Metallbearbeitung oder in Bohr- und Schneidölen.

13. Verfahren zur Bekämpfung von phytopathogenen Pilzen, dadurch gekennzeichnet, daß man auf diese oder die von ihnen befallenen Pflanzen, Pflanzenteile, Flächen, Substrate oder das von ihnen befallene Saatgut eine fungizid wirksame Menge einer Verbindung der Formel I gemäß Anspruch 4 oder eines Mittels gemäß einem der Ansprüche 1 bis 3 und 6 bis 8 appliziert.

Europäisches Patentamt

# EUROPÄISCHER RECHERCHENBERICHT

Nummer der Anmeldung

EP 93 10 7583
PAGE1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | PHARMAZIE<br>Bd. 42, Nr. 6, 1987, BERLIN DD<br>Seiten 381 - 383<br>A. DLUGOSZ.<br>* Seite 381; Beispiel 6c * | 4 | C07D239/28<br>C07D239/30<br>C07D239/34<br>C07D239/38<br>C07D239/42<br>C07D239/48<br>C07D239/46<br>C07D401/04<br>C07D407/04<br>C07D409/04<br>C07D413/04<br>A01N43/54 |
| X,D | POL. J. PHARMACOL. PHARM.<br>Bd. 25, Nr. 6, 1973,<br>Seiten 579 - 585<br>Z. MACHON ET AL.<br>* Seite 582; Beispiele VII-XIII * | 4 | |
| X | ARCHIV DER PHARMAZIE<br>Bd. 322, Nr. 10, 1989, WEINHEIM DE<br>Seiten 599 - 602<br>A. DLUGOSZ.<br>* Seite 599; Beispiele 1a-1d * | 4 | |
| X | ARCHIV DER PHARMAZIE<br>Bd. 323, Nr. 1, 1990, WEINHEIM DE<br>Seiten 59 - 60<br>A. DLUGOSZ.<br>* Seite 59; Beispiele 2,3 * | 4 | RECHERCHIERTE SACHGEBIETE (Int. Cl.5)<br>C07D<br>A01N |
| X | CHEM. ABS. SERV. 'CAS Registry Handbook, 1989 supplement'<br>1989 , AM. CHEM. SOC.<br>* RN : 124173-00-8 * | 4 | |
| X | CHEMICAL ABSTRACTS, vol. 110, no. 9, 27. Februar 1989, Columbus, Ohio, US;<br>abstract no. 75558m,<br>& JP-A-63 198 670 (DAICEL CHEMICAL INDUSTRIES LTD.)<br>* RN : 118648-60-5; 118648-59-2; 118648-58-1; 118648-57-0 * | 4,6,8,10 | |

-/--

Der vorliegende Recherchenbericht wurde für alle Patentansprüche erstellt

| Recherchenort | Abschlußdatum der Recherche | Prüfer |
|---|---|---|
| BERLIN | 08 SEPTEMBER 1993 | FRELON D. L. M. G. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 93 10 7583
PAGE2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | CHEMICAL ABSTRACTS, vol. 112, no. 7, 12. Februar 1990, Columbus, Ohio, US; abstract no. 50609g, & JP-A-01 180 805 (DAICEL CHEMICAL INDUSTRIES, LTD.) * RN : 124188-81-4; 124188-80-3; 124188-79-0; 124188-73-4; 124188-72-3; 124188-71-2; 124188-68-7; 124173-09-7; 124173-08-6; 124173-07-5; 124173-06-4; 124173-05-3; 124173-04-2; 124173-02-0, etc. * | 1-3,6-13 | |
| X<br>D | CHEMICAL ABSTRACTS, vol. 112, no. 1, 1. Januar 1990, Columbus, Ohio, US; abstract no. 2621h, & JP-A-01 180 804 (DAICEL CHEMICAL INDUSTRIES, LTD.) * RN : 124206-61-7; 124189-05-5; 124189-03-3; 124189-02-2; 124189-00-0; 124188-98-3; 124188-97-2; 124188-95-0; 124188-94-9; 124188-91-6; 124188-95-0; 124188-94-9; 124188-91-6; 124188-90-5, etc. * | 1-3,6-13 | RECHERCHIERTE SACHGEBIETE (Int. Cl.5) |
| A | EP-A-0 142 040 (BAYER A.G.) * Bsp. 3, s. 47; Bsp. 35,36, s. 51 * | 1-13 | |
| A | CH-A-606 028 (CIBA-GEIGY A.G.) * Bsp. 17,19; Sp. 11-12 * | 1-13 | |
| A | DE-A-3 205 638 (HOECHST A.G.) * Tab. 1, s. 28-29 * | 1-13 | |
| A | DE-A-3 206 900 (HOECHST A.G.) * Beispiele 24,25 * | 1-13 | |

-----

Der vorliegende Recherchenbericht wurde für alle Patentansprüche erstellt

| Recherchenort | Abschlußdatum der Recherche | Prüfer |
|---|---|---|
| BERLIN | 08 SEPTEMBER 1993 | FRELON D. L. M. G. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)